# EUROPEAN PATENT APPLICATION

(11) **EP 3 828 198 A1**
(43) Date of publication of application: **02.06.2021**
(21) Application number: 20202446.9
(22) Date of filing: 07.01.2016
(51) Int. Cl.: C07K 14/71, A61K 38/17, A61P 19/08, A61P 5/00, A61P 35/00, C07K 14/47

(54) **SOLUBLE FGFR3 DECOYS FOR TREATING SKELETAL GROWTH DISORDERS**

(30) Priority: 07.01.2015 EP 15290003
(62) Divisional of application: 16716673.5
(71) Applicant: Pfizer Inc., New York, NY 10017 (US); INSERM (Institut National de la Santé et de la Recherche Médicale), 75013 Paris (FR); Université Côte d'Azur, 06100 Nice Cedex 2 (FR)
(72) Inventor: GOUZE, Elvire, 06410 Biot (FR); GARCIA, Stéphanie, 06200 Nice (FR)
(74) Representative: Zwicker, Jörk

(57) **Abstract**

The invention features soluble FGF decoy polypeptides and fusion polypeptides comprising an FGF decoy polypeptide linked to a heterologous polypeptide, such as an aggrecan binding protein. Both soluble FGF decoy polypeptides and fusion polypeptides can be used to prevent or treat skeletal disorders, such as achondroplasia.

SEQUENCE LISTING

<110>  PFIZER INC. et al.

<120>  SOLUBLE FGFR3 DECOYS FOR TREATING SKELETAL GROWTH DISORDERS

<130>  901-3 PCTEPT1

<150>  EP 15290003.1
<151>  2015-01-07

<150>  PCT/IB2016/000403
<151>  2016-01-07

<150>  EP 16 716 673.5
<151>  2016-01-07

<160>  34

<170>  PatentIn version 3.5

<210>  1
<211>  694
<212>  PRT
<213>  Artificial Sequence

<220>
<223>  sFGFR3

<400>  1

Met Gly Ala Pro Ala Cys Ala Leu Ala Leu Cys Val Ala Val Ala Ile
1               5                   10                  15

Val Ala Gly Ala Ser Ser Glu Ser Leu Gly Thr Glu Gln Arg Val Val
            20                  25                  30

Gly Arg Ala Ala Glu Val Pro Gly Pro Glu Pro Gly Gln Gln Glu Gln
        35                  40                  45

Leu Val Phe Gly Ser Gly Asp Ala Val Glu Leu Ser Cys Pro Pro Pro
    50                  55                  60

Gly Gly Gly Pro Met Gly Pro Thr Val Trp Val Lys Asp Gly Thr Gly
65                  70                  75                  80

Leu Val Pro Ser Glu Arg Val Leu Val Gly Pro Gln Arg Leu Gln Val
                85                  90                  95

Leu Asn Ala Ser His Glu Asp Ser Gly Ala Tyr Ser Cys Arg Gln Arg
            100                 105                 110

Leu Thr Gln Arg Val Leu Cys His Phe Ser Val Arg Val Thr Asp Ala
        115                 120                 125

Pro Ser Ser Gly Asp Asp Glu Asp Gly Glu Asp Glu Ala Glu Asp Thr
    130                 135                 140

Gly Val Asp Thr Gly Ala Pro Tyr Trp Thr Arg Pro Glu Arg Met Asp
145                 150                 155                 160

Lys Lys Leu Leu Ala Val Pro Ala Ala Asn Thr Val Arg Phe Arg Cys
                165                 170                 175

Pro Ala Ala Gly Asn Pro Thr Pro Ser Ile Ser Trp Leu Lys Asn Gly
            180                 185                 190

Arg Glu Phe Arg Gly Glu His Arg Ile Gly Gly Ile Lys Leu Arg His
        195                 200                 205

Gln Gln Trp Ser Leu Val Met Glu Ser Val Val Pro Ser Asp Arg Gly
    210                 215                 220

Asn Tyr Thr Cys Val Val Glu Asn Lys Phe Gly Ser Ile Arg Gln Thr
225                 230                 235                 240

Tyr Thr Leu Asp Val Leu Glu Arg Ser Pro His Arg Pro Ile Leu Gln
                245                 250                 255

Ala Gly Leu Pro Ala Asn Gln Thr Ala Val Leu Gly Ser Asp Val Glu
            260                 265                 270

Phe His Cys Lys Val Tyr Ser Asp Ala Gln Pro His Ile Gln Trp Leu
        275                 280                 285

Lys His Val Glu Val Asn Gly Ser Lys Val Gly Pro Asp Gly Thr Pro
    290                 295                 300

Tyr Val Thr Val Leu Lys Val Ser Leu Glu Ser Asn Ala Ser Met Ser
305                 310                 315                 320

Ser Asn Thr Pro Leu Val Arg Ile Ala Arg Leu Ser Ser Gly Glu Gly
                325                 330                 335

Pro Thr Leu Ala Asn Val Ser Glu Leu Glu Leu Pro Ala Asp Pro Lys
            340                 345                 350

Trp Glu Leu Ser Arg Ala Arg Leu Thr Leu Gly Lys Pro Leu Gly Glu
        355                 360                 365

Gly Cys Phe Gly Gln Val Val Met Ala Glu Ala Ile Gly Ile Asp Lys
    370                 375                 380

Asp Arg Ala Ala Lys Pro Val Thr Val Ala Val Lys Met Leu Lys Asp
385                 390                 395                 400

Asp Ala Thr Asp Lys Asp Leu Ser Asp Leu Val Ser Glu Met Glu Met
                405                 410                 415

Met Lys Met Ile Gly Lys His Lys Asn Ile Ile Asn Leu Leu Gly Ala
            420                 425                 430

Cys Thr Gln Gly Gly Pro Leu Tyr Val Leu Val Glu Tyr Ala Ala Lys
        435                 440                 445

Gly Asn Leu Arg Glu Phe Leu Arg Ala Arg Arg Pro Pro Gly Leu Asp
    450                 455                 460

Tyr Ser Phe Asp Thr Cys Lys Pro Pro Glu Glu Gln Leu Thr Phe Lys
465                 470                 475                 480

Asp Leu Val Ser Cys Ala Tyr Gln Val Ala Arg Gly Met Glu Tyr Leu
                485                 490                 495

Ala Ser Gln Lys Cys Ile His Arg Asp Leu Ala Ala Arg Asn Val Leu
            500                 505                 510

Val Thr Glu Asp Asn Val Met Lys Ile Ala Asp Phe Gly Leu Ala Arg
        515                 520                 525

Asp Val His Asn Leu Asp Tyr Tyr Lys Lys Thr Thr Asn Gly Arg Leu
    530                 535                 540

Pro Val Lys Trp Met Ala Pro Glu Ala Leu Phe Asp Arg Val Tyr Thr
545                 550                 555                 560

His Gln Ser Asp Val Trp Ser Phe Gly Val Leu Leu Trp Glu Ile Phe
                565                 570                 575

Thr Leu Gly Gly Ser Pro Tyr Pro Gly Ile Pro Val Glu Glu Leu Phe
            580                 585                 590

Lys Leu Leu Lys Glu Gly His Arg Met Asp Lys Pro Ala Asn Cys Thr
        595                 600                 605

His Asp Leu Tyr Met Ile Met Arg Glu Cys Trp His Ala Ala Pro Ser
    610                 615                 620

Gln Arg Pro Thr Phe Lys Gln Leu Val Glu Asp Leu Asp Arg Val Leu
625                 630                 635                 640

Thr Val Thr Ser Thr Asp Glu Tyr Leu Asp Leu Ser Ala Pro Phe Glu
                645                 650                 655

Gln Tyr Ser Pro Gly Gly Gln Asp Thr Pro Ser Ser Ser Ser Ser Gly
            660                 665                 670

Asp Asp Ser Val Phe Ala His Asp Leu Leu Pro Pro Ala Pro Pro Ser
        675                 680                 685

Ser Gly Gly Ser Arg Thr
    690

<210>  2
<211>  741
<212>  PRT
<213>  Artificial Sequence

<220>
<223>  sFGFR3 with C-terminal part of Ig-like domain III

<400>  2
   Met Gly Ala Pro Ala Cys Ala Leu Ala Leu Cys Val Ala Val Ala Ile
   1               5                   10                  15
   Val Ala Gly Ala Ser Ser Glu Ser Leu Gly Thr Glu Gln Arg Val Val
               20                  25                  30
   Gly Arg Ala Ala Glu Val Pro Gly Pro Glu Pro Gly Gln Gln Glu Gln
           35                  40                  45
   Leu Val Phe Gly Ser Gly Asp Ala Val Glu Leu Ser Cys Pro Pro Pro
       50                  55                  60
   Gly Gly Gly Pro Met Gly Pro Thr Val Trp Val Lys Asp Gly Thr Gly
   65                  70                  75                  80
   Leu Val Pro Ser Glu Arg Val Leu Val Gly Pro Gln Arg Leu Gln Val
                   85                  90                  95
   Leu Asn Ala Ser His Glu Asp Ser Gly Ala Tyr Ser Cys Arg Gln Arg
               100                 105                 110
   Leu Thr Gln Arg Val Leu Cys His Phe Ser Val Arg Val Thr Asp Ala
           115                 120                 125
   Pro Ser Ser Gly Asp Asp Glu Asp Gly Glu Asp Glu Ala Glu Asp Thr
       130                 135                 140
   Gly Val Asp Thr Gly Ala Pro Tyr Trp Thr Arg Pro Glu Arg Met Asp
   145                 150                 155                 160
   Lys Lys Leu Leu Ala Val Pro Ala Ala Asn Thr Val Arg Phe Arg Cys
                   165                 170                 175
   Pro Ala Ala Gly Asn Pro Thr Pro Ser Ile Ser Trp Leu Lys Asn Gly
               180                 185                 190
   Arg Glu Phe Arg Gly Glu His Arg Ile Gly Gly Ile Lys Leu Arg His
           195                 200                 205
   Gln Gln Trp Ser Leu Val Met Glu Ser Val Val Pro Ser Asp Arg Gly
       210                 215                 220
   Asn Tyr Thr Cys Val Val Glu Asn Lys Phe Gly Ser Ile Arg Gln Thr
   225                 230                 235                 240
   Tyr Thr Leu Asp Val Leu Glu Arg Ser Pro His Arg Pro Ile Leu Gln
                   245                 250                 255
   Ala Gly Leu Pro Ala Asn Gln Thr Ala Val Leu Gly Ser Asp Val Glu
               260                 265                 270
   Phe His Cys Lys Val Tyr Ser Asp Ala Gln Pro His Ile Gln Trp Leu
           275                 280                 285
   Lys His Val Glu Val Asn Gly Ser Lys Val Gly Pro Asp Gly Thr Pro
       290                 295                 300
   Tyr Val Thr Val Leu Lys Thr Ala Gly Ala Asn Thr Thr Asp Lys Glu
   305                 310                 315                 320
   Leu Glu Val Leu Ser Leu His Asn Val Thr Phe Glu Asp Ala Gly Glu
                   325                 330                 335
   Tyr Thr Cys Leu Ala Gly Asn Ser Ile Gly Phe Ser His His Ser Ala
               340                 345                 350
   Trp Leu Val Val Leu Val Ser Leu Glu Ser Asn Ala Ser Met Ser Ser
           355                 360                 365
   Asn Thr Pro Leu Val Arg Ile Ala Arg Leu Ser Ser Gly Glu Gly Pro
       370                 375                 380
   Thr Leu Ala Asn Val Ser Glu Leu Glu Leu Pro Ala Asp Pro Lys Trp
   385                 390                 395                 400
   Glu Leu Ser Arg Ala Arg Leu Thr Leu Gly Lys Pro Leu Gly Glu Gly
                   405                 410                 415
   Cys Phe Gly Gln Val Val Met Ala Glu Ala Ile Gly Ile Asp Lys Asp
               420                 425                 430
   Arg Ala Ala Lys Pro Val Thr Val Ala Val Lys Met Leu Lys Asp Asp
           435                 440                 445
   Ala Thr Asp Lys Asp Leu Ser Asp Leu Val Ser Glu Met Glu Met Met
       450                 455                 460
   Lys Met Ile Gly Lys His Lys Asn Ile Ile Asn Leu Leu Gly Ala Cys
   465                 470                 475                 480
   Thr Gln Gly Gly Pro Leu Tyr Val Leu Val Glu Tyr Ala Ala Lys Gly
                   485                 490                 495
   Asn Leu Arg Glu Phe Leu Arg Ala Arg Arg Pro Pro Gly Leu Asp Tyr
               500                 505                 510
   Ser Phe Asp Thr Cys Lys Pro Pro Glu Glu Gln Leu Thr Phe Lys Asp
           515                 520                 525
   Leu Val Ser Cys Ala Tyr Gln Val Ala Arg Gly Met Glu Tyr Leu Ala
       530                 535                 540
   Ser Gln Lys Cys Ile His Arg Asp Leu Ala Ala Arg Asn Val Leu Val
   545                 550                 555                 560
   Thr Glu Asp Asn Val Met Lys Ile Ala Asp Phe Gly Leu Ala Arg Asp
                   565                 570                 575
   Val His Asn Leu Asp Tyr Tyr Lys Lys Thr Thr Asn Gly Arg Leu Pro
               580                 585                 590
   Val Lys Trp Met Ala Pro Glu Ala Leu Phe Asp Arg Val Tyr Thr His
           595                 600                 605
   Gln Ser Asp Val Trp Ser Phe Gly Val Leu Leu Trp Glu Ile Phe Thr
       610                 615                 620
   Leu Gly Gly Ser Pro Tyr Pro Gly Ile Pro Val Glu Glu Leu Phe Lys
   625                 630                 635                 640
   Leu Leu Lys Glu Gly His Arg Met Asp Lys Pro Ala Asn Cys Thr His
   645                                    650                                    655
   Asp Leu Tyr Met Ile Met Arg Glu Cys Trp His Ala Ala Pro Ser Gln
               660                 665                 670
   Arg Pro Thr Phe Lys Gln Leu Val Glu Asp Leu Asp Arg Val Leu Thr
           675                 680                 685
   Val Thr Ser Thr Asp Glu Tyr Leu Asp Leu Ser Ala Pro Phe Glu Gln
       690                 695                 700
   Tyr Ser Pro Gly Gly Gln Asp Thr Pro Ser Ser Ser Ser Ser Gly Asp
   705                 710                 715                 720
   Asp Ser Val Phe Ala His Asp Leu Leu Pro Pro Ala Pro Pro Ser Ser
                   725                 730                 735
   Gly Gly Ser Arg Thr
               740

<210>  3
<211>  353
<212>  PRT
<213>  Homo sapiens

<400>  3

Met Lys Ser Leu Leu Leu Leu Val Leu Ile Ser Ile Cys Trp Ala Asp
1               5                   10                  15

His Leu Ser Asp Asn Tyr Thr Leu Asp His Arg Ala Ile His Ile Gln
            20                  25                  30

Ala Glu Asn Gly Pro His Leu Leu Val Glu Ala Glu Gln Ala Lys Val
        35                  40                  45

Phe Ser His Arg Gly Gly Asn Val Thr Leu Pro Cys Lys Phe Tyr Arg
    50                  55                  60

Asp Pro Thr Ala Phe Gly Ser Gly Ile His Lys Ile Arg Ile Lys Trp
65                  70                  75                  80

Thr Lys Leu Thr Ser Asp Tyr Leu Lys Glu Val Asp Val Phe Val Ser
                85                  90                  95

Met Gly Tyr His Lys Lys Thr Tyr Gly Gly Tyr Gln Gly Arg Val Phe
            100                 105                 110

Leu Lys Gly Gly Ser Asp Ser Asp Ala Ser Leu Val Ile Thr Asp Leu

115                 120                 125

Thr Leu Glu Asp Tyr Gly Arg Tyr Lys Cys Glu Val Ile Glu Gly Leu
    130                 135                 140

Glu Asp Asp Thr Val Val Val Ala Leu Asp Leu Gln Gly Val Val Phe
145                 150                 155                 160

Pro Tyr Phe Pro Arg Leu Gly Arg Tyr Asn Leu Asn Phe His Glu Ala
                165                 170                 175

Gln Gln Ala Cys Leu Asp Gln Asp Ala Val Ile Ala Ser Phe Asp Gln
            180                 185                 190

Leu Tyr Asp Ala Trp Arg Gly Gly Leu Asp Trp Cys Asn Ala Gly Trp
        195                 200                 205

Leu Ser Asp Gly Ser Val Gln Tyr Pro Ile Thr Lys Pro Arg Glu Pro
    210                 215                 220

Cys Gly Gly Gln Asn Thr Val Pro Gly Val Arg Asn Tyr Gly Phe Trp
225                 230                 235                 240

Asp Lys Asp Lys Ser Arg Tyr Asp Val Phe Cys Phe Thr Ser Asn Phe
                245                 250                 255

Asn Gly Arg Phe Tyr Tyr Leu Ile His Pro Thr Lys Leu Thr Tyr Asp
            260                 265                 270

Glu Ala Val Gln Ala Cys Leu Asn Asp Gly Ala Gln Ile Ala Lys Val
        275                 280                 285

Gly Gln Ile Phe Ala Ala Trp Lys Ile Leu Gly Tyr Asp Arg Cys Asp
    290                 295                 300

Ala Gly Trp Leu Ala Asp Gly Ser Val Arg Tyr Pro Ile Ser Arg Pro
305                 310                 315                 320

Arg Arg Arg Cys Ser Pro Thr Glu Ala Ala Val Arg Phe Val Gly Phe
                325                 330                 335

Pro Asp Lys Lys His Lys Leu Tyr Gly Val Tyr Cys Phe Arg Ala Tyr
            340                 345                 350

Asn

<210>  4
<211>  549
<212>  PRT
<213>  Artificial Sequence

<220>
<223>  FLAG-sFGFR3-Del4-LK1-LK2

<400>  4

Asp Tyr Lys Asp Asp Asp Asp Lys Leu Val Asp Gln Ile Pro Ala Met
1               5                   10                  15

Gly Ala Pro Ala Cys Ala Leu Ala Leu Cys Val Ala Val Ala Ile Val
            20                  25                  30

Ala Gly Ala Ser Ser Glu Ser Leu Gly Thr Glu Gln Arg Val Val Gly
        35                  40                  45

Arg Ala Ala Glu Val Pro Gly Pro Glu Pro Gly Gln Gln Glu Gln Leu
    50                  55                  60

Val Phe Gly Ser Gly Asp Ala Val Glu Leu Ser Cys Pro Pro Pro Gly
65                  70                  75                  80

Gly Gly Pro Met Gly Pro Thr Val Trp Val Lys Asp Gly Thr Gly Leu
                85                  90                  95

Val Pro Ser Glu Arg Val Leu Val Gly Pro Gln Arg Leu Gln Val Leu
            100                 105                 110

Asn Ala Ser His Glu Asp Ser Gly Ala Tyr Ser Cys Arg Gln Arg Leu
        115                 120                 125

Thr Gln Arg Val Leu Cys His Phe Ser Val Arg Val Thr Asp Ala Pro
    130                 135                 140

Ser Ser Gly Asp Asp Glu Asp Gly Glu Asp Glu Ala Glu Asp Thr Gly
145                 150                 155                 160

Val Asp Thr Gly Ala Pro Tyr Trp Thr Arg Pro Glu Arg Met Asp Lys
                165                 170                 175

Lys Leu Leu Ala Val Pro Ala Ala Asn Thr Val Arg Phe Arg Cys Pro
            180                 185                 190

Ala Ala Gly Asn Pro Thr Pro Ser Ile Ser Trp Leu Lys Asn Gly Arg
        195                 200                 205

Glu Phe Arg Gly Glu His Arg Ile Gly Gly Ile Lys Leu Arg His Gln

210                 215                 220

Gln Trp Ser Leu Val Met Glu Ser Val Val Pro Ser Asp Arg Gly Asn
225                 230                 235                 240

Tyr Thr Cys Val Val Glu Asn Lys Phe Gly Ser Ile Arg Gln Thr Tyr
                245                 250                 255

Thr Leu Asp Val Leu Glu Arg Ser Pro His Arg Pro Ile Leu Gln Ala
            260                 265                 270

Gly Leu Pro Ala Asn Gln Thr Ala Val Leu Gly Ser Asp Val Glu Phe
        275                 280                 285

His Cys Lys Val Tyr Ser Asp Ala Gln Pro His Ile Gln Trp Leu Lys
    290                 295                 300

His Val Glu Val Asn Gly Ser Lys Val Gly Pro Asp Gly Thr Pro Tyr
305                 310                 315                 320

Val Thr Val Leu Lys Val Ser Leu Glu Ser Asn Ala Ser Met Ser Ser
                325                 330                 335

Asn Thr Ser Gly Ser Gly Ser Gly Ser Gly Ser Gly Ser Gly Ser Gly
            340                 345                 350

Ser Val Val Phe Pro Tyr Phe Pro Arg Leu Gly Arg Tyr Asn Leu Asn
        355                 360                 365

Phe His Glu Ala Gln Gln Ala Cys Leu Asp Gln Asp Ala Val Ile Ala
    370                 375                 380

Ser Phe Asp Gln Leu Tyr Asp Ala Trp Arg Gly Gly Leu Asp Trp Cys
385                 390                 395                 400

Asn Ala Gly Trp Leu Ser Asp Gly Ser Val Gln Tyr Pro Ile Thr Lys
                405                 410                 415

Pro Arg Glu Pro Cys Gly Gly Gln Asn Thr Val Pro Gly Val Arg Asn
            420                 425                 430

Tyr Gly Phe Trp Asp Lys Asp Lys Ser Arg Tyr Asp Val Phe Cys Phe
        435                 440                 445

Thr Ser Asn Phe Asn Gly Arg Phe Tyr Tyr Leu Ile His Pro Thr Lys
    450                 455                 460

Leu Thr Tyr Asp Glu Ala Val Gln Ala Cys Leu Asn Asp Gly Ala Gln
465                 470                 475                 480

Ile Ala Lys Val Gly Gln Ile Phe Ala Ala Trp Lys Ile Leu Gly Tyr
                485                 490                 495

Asp Arg Cys Asp Ala Gly Trp Leu Ala Asp Gly Ser Val Arg Tyr Pro
            500                 505                 510

Ile Ser Arg Pro Arg Arg Arg Cys Ser Pro Thr Glu Ala Ala Val Arg
        515                 520                 525

Phe Val Gly Phe Pro Asp Lys Lys His Lys Leu Tyr Gly Val Tyr Cys
    530                 535                 540

Phe Arg Ala Tyr Asn
545

<210>  5
<211>  1638
<212>  DNA
<213>  Artificial Sequence

<220>
<223>  FLAG-sFGFR3-Del4-LK1-LK2

<400>  5
aagcttgtcg accagattcc cgccatgggc gctccagcct gtgccctggc tctgtgtgtg      60

gccgtggcca ttgtggctgg agccagcagc gagagcctgg gcacagagca gcgggtggtg     120

ggaagggccg ctgaagtgcc tggccctgag cctggccagc aggaacagct ggtctttggc     180

agcggcgacg ccgtggaact gagctgtcct ccacccggcg gaggccctat gggccctacc     240

gtgtgggtga aagacggcac cggcctggtg cccagcgaga gggtgctggt gggaccccag     300

cggctgcagg tcctgaacgc cagccacgag gacagcggcg cctacagctg cagacagaga     360

ctgacccagc gggtgctgtg ccacttcagc gtgcgcgtga ccgacgcccc tagctctggc     420

gacgacgagg acggcgagga cgaggccgag gataccggcg tggacacagg cgccccttac     480

tggaccagac ccgagcggat ggacaagaaa ctgctggccg tgcctgccgc caacaccgtg     540

cggttcagat gtcccgccgc tggcaacccc acccccagca tcagctggct gaagaacggc     600

agagagttcc ggggcgagca ccggatcggc ggcatcaagc tgcggcacca gcagtggtcc     660

ctggtcatgg aaagcgtggt gccctccgac cggggcaact acacctgtgt ggtggaaaac     720

aagttcggca gcatccggca gacctacacc ctggacgtgc tggaaagaag cccccaccgg     780

cccatcctgc aggctggact gcccgccaat cagacagccg tgctgggcag cgacgtggaa     840

tttcactgca aggtgtacag cgacgcccag ccccacatcc agtggctcaa gcacgtggaa     900

gtgaacggca gcaaagtggg ccccgacggc accccttacg tgaccgtgct gaaagtgtcc 960

ctggaaagca acgccagcat gagcagcaac acctgaggta ccagcggcag cggctctggc 1020

tctggaagcg gaagcggatc tgggagcgtg gtgttcccct acttcccccg gctgggccgg 1080

tacaacctga acttccacga ggcccagcag gcttgcctcg accaggatgc cgtgatcgcc 1140

agcttcgacc agctgtacga tgcctggcgg ggaggcctgg actggtgcaa tgccggctgg 1200

ctgtccgacg gcagcgtcca gtaccccatc accaagccca gagagccctg cggcggacag 1260

aacacagtgc ccggcgtgcg gaactacggc ttctgggaca aggacaagag cagatacgac 1320

gtgttctgct tcaccagcaa cttcaacggc cggttctact acctgatcca ccccaccaag 1380

ctgacctacg acgaagccgt ccaggcctgt ctgaacgacg gcgcccagat cgccaaagtg 1440

ggacagatct tcgccgcctg gaagatcctg ggctacgaca gatgcgacgc cggatggctg 1500

gccgatggct ccgtgcgcta ccctatcagc agaccccgca gaagatgcag ccctaccgag 1560

gccgccgtgc gcttcgtggg cttccccgac aagaagcaca agctgtacgg cgtctactgc 1620

ttccgggcct acaactga 1638

<210> 6
<211> 806
<212> PRT
<213> Homo sapiens

<400> 6

Met Gly Ala Pro Ala Cys Ala Leu Ala Leu Cys Val Ala Val Ala Ile
1               5                   10                  15

Val Ala Gly Ala Ser Ser Glu Ser Leu Gly Thr Glu Gln Arg Val Val
            20                  25                  30

Gly Arg Ala Ala Glu Val Pro Gly Pro Glu Pro Gly Gln Gln Glu Gln
        35                  40                  45

Leu Val Phe Gly Ser Gly Asp Ala Val Glu Leu Ser Cys Pro Pro Pro
    50                  55                  60

Gly Gly Gly Pro Met Gly Pro Thr Val Trp Val Lys Asp Gly Thr Gly
65                  70                  75                  80

Leu Val Pro Ser Glu Arg Val Leu Val Gly Pro Gln Arg Leu Gln Val
                85                  90                  95

Leu Asn Ala Ser His Glu Asp Ser Gly Ala Tyr Ser Cys Arg Gln Arg
            100                 105                 110

Leu Thr Gln Arg Val Leu Cys His Phe Ser Val Arg Val Thr Asp Ala

115                 120                 125

Pro Ser Ser Gly Asp Asp Glu Asp Gly Glu Asp Glu Ala Glu Asp Thr
    130                 135                 140

Gly Val Asp Thr Gly Ala Pro Tyr Trp Thr Arg Pro Glu Arg Met Asp
145                 150                 155                 160

Lys Lys Leu Leu Ala Val Pro Ala Ala Asn Thr Val Arg Phe Arg Cys
                165                 170                 175

Pro Ala Ala Gly Asn Pro Thr Pro Ser Ile Ser Trp Leu Lys Asn Gly
            180                 185                 190

Arg Glu Phe Arg Gly Glu His Arg Ile Gly Gly Ile Lys Leu Arg His
        195                 200                 205

Gln Gln Trp Ser Leu Val Met Glu Ser Val Val Pro Ser Asp Arg Gly
    210                 215                 220

Asn Tyr Thr Cys Val Val Glu Asn Lys Phe Gly Ser Ile Arg Gln Thr
225                 230                 235                 240

Tyr Thr Leu Asp Val Leu Glu Arg Ser Pro His Arg Pro Ile Leu Gln
                245                 250                 255

Ala Gly Leu Pro Ala Asn Gln Thr Ala Val Leu Gly Ser Asp Val Glu
            260                 265                 270

Phe His Cys Lys Val Tyr Ser Asp Ala Gln Pro His Ile Gln Trp Leu
        275                 280                 285

Lys His Val Glu Val Asn Gly Ser Lys Val Gly Pro Asp Gly Thr Pro
    290                 295                 300

Tyr Val Thr Val Leu Lys Thr Ala Gly Ala Asn Thr Thr Asp Lys Glu
305                 310                 315                 320

Leu Glu Val Leu Ser Leu His Asn Val Thr Phe Glu Asp Ala Gly Glu
                325                 330                 335

Tyr Thr Cys Leu Ala Gly Asn Ser Ile Gly Phe Ser His His Ser Ala
            340                 345                 350

Trp Leu Val Val Leu Pro Ala Glu Glu Glu Leu Val Glu Ala Asp Glu
        355                 360                 365

Ala Gly Ser Val Tyr Ala Gly Ile Leu Ser Tyr Gly Val Gly Phe Phe
    370                 375                 380

Leu Phe Ile Leu Val Val Ala Ala Val Thr Leu Cys Arg Leu Arg Ser
385                 390                 395                 400

Pro Pro Lys Lys Gly Leu Gly Ser Pro Thr Val His Lys Ile Ser Arg
                405                 410                 415

Phe Pro Leu Lys Arg Gln Val Ser Leu Glu Ser Asn Ala Ser Met Ser
            420                 425                 430

Ser Asn Thr Pro Leu Val Arg Ile Ala Arg Leu Ser Ser Gly Glu Gly
        435                 440                 445

Pro Thr Leu Ala Asn Val Ser Glu Leu Glu Leu Pro Ala Asp Pro Lys
    450                 455                 460

Trp Glu Leu Ser Arg Ala Arg Leu Thr Leu Gly Lys Pro Leu Gly Glu
465                 470                 475                 480

Gly Cys Phe Gly Gln Val Val Met Ala Glu Ala Ile Gly Ile Asp Lys
                485                 490                 495

Asp Arg Ala Ala Lys Pro Val Thr Val Ala Val Lys Met Leu Lys Asp
            500                 505                 510

Asp Ala Thr Asp Lys Asp Leu Ser Asp Leu Val Ser Glu Met Glu Met
        515                 520                 525

Met Lys Met Ile Gly Lys His Lys Asn Ile Ile Asn Leu Leu Gly Ala
    530                 535                 540

Cys Thr Gln Gly Gly Pro Leu Tyr Val Leu Val Glu Tyr Ala Ala Lys
545                 550                 555                 560

Gly Asn Leu Arg Glu Phe Leu Arg Ala Arg Arg Pro Pro Gly Leu Asp
                565                 570                 575

Tyr Ser Phe Asp Thr Cys Lys Pro Pro Glu Glu Gln Leu Thr Phe Lys
            580                 585                 590

Asp Leu Val Ser Cys Ala Tyr Gln Val Ala Arg Gly Met Glu Tyr Leu
        595                 600                 605

Ala Ser Gln Lys Cys Ile His Arg Asp Leu Ala Ala Arg Asn Val Leu
    610                 615                 620

Val Thr Glu Asp Asn Val Met Lys Ile Ala Asp Phe Gly Leu Ala Arg
625                 630                 635                 640

Asp Val His Asn Leu Asp Tyr Tyr Lys Lys Thr Thr Asn Gly Arg Leu
                645                 650                 655

Pro Val Lys Trp Met Ala Pro Glu Ala Leu Phe Asp Arg Val Tyr Thr
            660                 665                 670

His Gln Ser Asp Val Trp Ser Phe Gly Val Leu Leu Trp Glu Ile Phe
        675                 680                 685

Thr Leu Gly Gly Ser Pro Tyr Pro Gly Ile Pro Val Glu Glu Leu Phe
    690                 695                 700

Lys Leu Leu Lys Glu Gly His Arg Met Asp Lys Pro Ala Asn Cys Thr
705                 710                 715                 720

His Asp Leu Tyr Met Ile Met Arg Glu Cys Trp His Ala Ala Pro Ser
                725                 730                 735

Gln Arg Pro Thr Phe Lys Gln Leu Val Glu Asp Leu Asp Arg Val Leu
            740                 745                 750

Thr Val Thr Ser Thr Asp Glu Tyr Leu Asp Leu Ser Ala Pro Phe Glu
        755                 760                 765

Gln Tyr Ser Pro Gly Gly Gln Asp Thr Pro Ser Ser Ser Ser Ser Gly
    770                 775                 780

Asp Asp Ser Val Phe Ala His Asp Leu Leu Pro Pro Ala Pro Pro Ser
785                 790                 795                 800

Ser Gly Gly Ser Arg Thr
                805

<210>  7
<211>  155
<212>  PRT
<213>  Homo sapiens

<400>  7

Met Ala Glu Gly Glu Ile Thr Thr Phe Thr Ala Leu Thr Glu Lys Phe
1               5                   10                  15

Asn Leu Pro Pro Gly Asn Tyr Lys Lys Pro Lys Leu Leu Tyr Cys Ser
            20                  25                  30

Asn Gly Gly His Phe Leu Arg Ile Leu Pro Asp Gly Thr Val Asp Gly
        35                  40                  45

Thr Arg Asp Arg Ser Asp Gln His Ile Gln Leu Gln Leu Ser Ala Glu
    50                  55                  60

Ser Val Gly Glu Val Tyr Ile Lys Ser Thr Glu Thr Gly Gln Tyr Leu
65                  70                  75                  80

Ala Met Asp Thr Asp Gly Leu Leu Tyr Gly Ser Gln Thr Pro Asn Glu
                85                  90                  95

Glu Cys Leu Phe Leu Glu Arg Leu Glu Glu Asn His Tyr Asn Thr Tyr
            100                 105                 110

Ile Ser Lys Lys His Ala Glu Lys Asn Trp Phe Val Gly Leu Lys Lys
        115                 120                 125

Asn Gly Ser Cys Lys Arg Gly Pro Arg Thr His Tyr Gly Gln Lys Ala
    130                 135                 140

Ile Leu Phe Leu Pro Leu Pro Val Ser Ser Asp
145                 150                 155

<210>  8
<211>  288
<212>  PRT
<213>  Homo sapiens

<400>  8

Met Val Gly Val Gly Gly Gly Asp Val Glu Asp Val Thr Pro Arg Pro
1               5                   10                  15

Gly Gly Cys Gln Ile Ser Gly Arg Gly Ala Arg Gly Cys Asn Gly Ile
            20                  25                  30

Pro Gly Ala Ala Ala Trp Glu Ala Ala Leu Pro Arg Arg Arg Pro Arg
        35                  40                  45

Arg His Pro Ser Val Asn Pro Arg Ser Arg Ala Ala Gly Ser Pro Arg
    50                  55                  60

Thr Arg Gly Arg Arg Thr Glu Glu Arg Pro Ser Gly Ser Arg Leu Gly
65                  70                  75                  80

Asp Arg Gly Arg Gly Arg Ala Leu Pro Gly Gly Arg Leu Gly Gly Arg
                85                  90                  95

Gly Arg Gly Arg Ala Pro Glu Arg Val Gly Gly Arg Gly Arg Gly Arg
            100                 105                 110

Gly Thr Ala Ala Pro Arg Ala Ala Pro Ala Ala Arg Gly Ser Arg Pro
        115                 120                 125

Gly Pro Ala Gly Thr Met Ala Ala Gly Ser Ile Thr Thr Leu Pro Ala
    130                 135                 140

Leu Pro Glu Asp Gly Gly Ser Gly Ala Phe Pro Pro Gly His Phe Lys
145                 150                 155                 160

Asp Pro Lys Arg Leu Tyr Cys Lys Asn Gly Gly Phe Phe Leu Arg Ile
                165                 170                 175

His Pro Asp Gly Arg Val Asp Gly Val Arg Glu Lys Ser Asp Pro His
            180                 185                 190

Ile Lys Leu Gln Leu Gln Ala Glu Glu Arg Gly Val Val Ser Ile Lys
        195                 200                 205

Gly Val Cys Ala Asn Arg Tyr Leu Ala Met Lys Glu Asp Gly Arg Leu
    210                 215                 220

Leu Ala Ser Lys Cys Val Thr Asp Glu Cys Phe Phe Phe Glu Arg Leu
225                 230                 235                 240

Glu Ser Asn Asn Tyr Asn Thr Tyr Arg Ser Arg Lys Tyr Thr Ser Trp
                245                 250                 255

Tyr Val Ala Leu Lys Arg Thr Gly Gln Tyr Lys Leu Gly Ser Lys Thr
            260                 265                 270

Gly Pro Gly Gln Lys Ala Ile Leu Phe Leu Pro Met Ser Ala Lys Ser
        275                 280                 285

<210>  9
<211>  208
<212>  PRT
<213>  Homo sapiens

<400>  9

<210>  10
<211>  207
<212>  PRT
<213>  Homo sapiens

<400>  10

Met Tyr Ser Ala Pro Ser Ala Cys Thr Cys Leu Cys Leu His Phe Leu
1               5                   10                  15

Leu Leu Cys Phe Gln Val Gln Val Leu Val Ala Glu Glu Asn Val Asp
            20                  25                  30

Phe Arg Ile His Val Glu Asn Gln Thr Arg Ala Arg Asp Asp Val Ser
        35                  40                  45

Arg Lys Gln Leu Arg Leu Tyr Gln Leu Tyr Ser Arg Thr Ser Gly Lys
    50                  55                  60

His Ile Gln Val Leu Gly Arg Arg Ile Ser Ala Arg Gly Glu Asp Gly
65                  70                  75                  80

Asp Lys Tyr Ala Gln Leu Leu Val Glu Thr Asp Thr Phe Gly Ser Gln
                85                  90                  95

Val Arg Ile Lys Gly Lys Glu Thr Glu Phe Tyr Leu Cys Met Asn Arg
            100                 105                 110

Lys Gly Lys Leu Val Gly Lys Pro Asp Gly Thr Ser Lys Glu Cys Val
        115                 120                 125

Phe Ile Glu Lys Val Leu Glu Asn Asn Tyr Thr Ala Leu Met Ser Ala
    130                 135                 140

Lys Tyr Ser Gly Trp Tyr Val Gly Phe Thr Lys Lys Gly Arg Pro Arg
145                 150                 155                 160

Lys Gly Pro Lys Thr Arg Glu Asn Gln Gln Asp Val His Phe Met Lys
                165                 170                 175

Arg Tyr Pro Lys Gly Gln Pro Glu Leu Gln Lys Pro Phe Lys Tyr Thr
            180                 185                 190

Thr Val Thr Lys Arg Ser Arg Arg Ile Arg Pro Thr His Pro Ala
        195                 200                 205


<210>  11
<211>  15
<212>  PRT
<213>  Artificial Sequence

<220>
<223>  label

<400>  11

Gly Leu Asn Asp Ile Phe Glu Ala Gln Lys Ile Glu Trp His Glu
1               5                   10                  15


<210>  12
<211>  26
<212>  PRT
<213>  Artificial Sequence

<220>
<223>  label

<400>  12

Lys Arg Arg Trp Lys Lys Asn Phe Ile Ala Val Ser Ala Ala Asn Arg
1               5                   10                  15

Phe Lys Lys Ile Ser Ser Ser Gly Ala Leu
            20                  25

<210>  13
<211>  6
<212>  PRT
<213>  Artificial Sequence

<220>
<223>  label

<400>  13

Glu Glu Glu Glu Glu Glu
1               5

<210>  14
<211>  13
<212>  PRT
<213>  Artificial Sequence

<220>
<223>  label

<400>  14

Gly Ala Pro Val Pro Tyr Pro Asp Pro Leu Glu Pro Arg
1               5                   10

<210>  15
<211>  8
<212>  PRT
<213>  Artificial Sequence

<220>
<223>  label

<400>  15

Asp Tyr Lys Asp Asp Asp Asp Lys
1               5

<210>  16
<211>  9
<212>  PRT
<213>  Artificial Sequence

<220>
<223>  label

<400>  16

Tyr Pro Tyr Asp Val Pro Asp Tyr Ala

1               5


<210>  17
<211>  6
<212>  PRT
<213>  Artificial Sequence

<220>
<223>  label

<400>  17

His His His His His His
1               5


<210>  18
<211>  10
<212>  PRT
<213>  Artificial Sequence

<220>
<223>  label

<400>  18

Glu Gln Lys Leu Ile Ser Glu Glu Asp Leu
1               5                   10


<210>  19
<211>  15
<212>  PRT
<213>  Artificial Sequence

<220>
<223>  label

<400>  19

Lys Glu Thr Ala Ala Ala Lys Phe Glu Arg Gln His Met Asp Ser
1               5                   10                  15


<210>  20
<211>  38
<212>  PRT
<213>  Artificial Sequence

<220>
<223>  label

<400>  20

Met Asp Glu Lys Thr Thr Gly Trp Arg Gly Gly His Val Val Glu Gly
1               5                   10                  15


Leu Ala Gly Glu Leu Glu Gln Leu Arg Ala Arg Leu Glu His His Pro
            20                  25                  30

Gln Gly Gln Arg Glu Pro
        35

<210>  21
<211>  13
<212>  PRT
<213>  Artificial Sequence

<220>
<223>  label

<400>  21

Ser Leu Ala Glu Leu Leu Asn Ala Gly Leu Gly Gly Ser
1               5                   10

<210>  22
<211>  8
<212>  PRT
<213>  Artificial Sequence

<220>
<223>  label

<400>  22

Thr Gln Asp Pro Ser Arg Val Gly
1               5

<210>  23
<211>  8
<212>  PRT
<213>  Artificial Sequence

<220>
<223>  label

<400>  23

Trp Ser His Pro Gln Phe Glu Lys
1               5

<210>  24
<211>  6
<212>  PRT
<213>  Artificial Sequence

<220>
<223>  label

<400>  24

Cys Cys Pro Gly Cys Cys
1               5

<210>  25
<211>  14
<212>  PRT

<213>  Artificial Sequence

<220>
<223>  label

<400>  25

Gly Lys Pro Ile Pro Asn Pro Leu Leu Gly Leu Asp Ser Thr
1               5                   10


<210>  26
<211>  11
<212>  PRT
<213>  Artificial Sequence

<220>
<223>  label

<400>  26

Tyr Thr Asp Ile Glu Met Asn Arg Leu Gly Lys
1               5                   10


<210>  27
<211>  8
<212>  PRT
<213>  Artificial Sequence

<220>
<223>  label

<400>  27

Asp Leu Tyr Asp Asp Asp Asp Lys
1               5


<210>  28
<211>  16
<212>  PRT
<213>  Artificial Sequence

<220>
<223>  label

<400>  28

Thr Asp Lys Asp Met Thr Ile Thr Phe Thr Asn Lys Lys Asp Ala Glu
1               5                   10                  15


<210>  29
<211>  13
<212>  PRT
<213>  Artificial Sequence

<220>
<223>  label

<400>  29

Ala His Ile Val Met Val Asp Ala Tyr Lys Pro Thr Lys
1               5                   10

<210>  30
<211>  7
<212>  PRT
<213>  Artificial Sequence

<220>
<223>  cleavable sequence

<400>  30

Leu Val Asp Gln Ile Pro Ala
1               5

<210>  31
<211>  24
<212>  DNA
<213>  Artificial Sequence

<220>
<223>  primer

<400>  31
aggtggcctt tgacacctac cagg                                              24

<210>  32
<211>  24
<212>  DNA
<213>  Artificial Sequence

<220>
<223>  primer

<400>  32
tctgttgtgt ttcctccctg ttgg                                              24

<210>  33
<211>  541
<212>  PRT
<213>  Artificial Sequence

<220>
<223>  Synthetic Construct

<400>  33

Leu Val Asp Gln Ile Pro Ala Met Gly Ala Pro Ala Cys Ala Leu Ala
1               5                   10                  15

Leu Cys Val Ala Val Ala Ile Val Ala Gly Ala Ser Ser Glu Ser Leu
            20                  25                  30

Gly Thr Glu Gln Arg Val Val Gly Arg Ala Ala Glu Val Pro Gly Pro
        35                  40                  45

Glu Pro Gly Gln Gln Glu Gln Leu Val Phe Gly Ser Gly Asp Ala Val
    50                  55                  60

Glu Leu Ser Cys Pro Pro Pro Gly Gly Gly Pro Met Gly Pro Thr Val
65                  70                  75                  80

Trp Val Lys Asp Gly Thr Gly Leu Val Pro Ser Glu Arg Val Leu Val
                85                  90                  95

Gly Pro Gln Arg Leu Gln Val Leu Asn Ala Ser His Glu Asp Ser Gly
            100                 105                 110

Ala Tyr Ser Cys Arg Gln Arg Leu Thr Gln Arg Val Leu Cys His Phe
        115                 120                 125

Ser Val Arg Val Thr Asp Ala Pro Ser Ser Gly Asp Asp Glu Asp Gly
    130                 135                 140

Glu Asp Glu Ala Glu Asp Thr Gly Val Asp Thr Gly Ala Pro Tyr Trp
145                 150                 155                 160

Thr Arg Pro Glu Arg Met Asp Lys Lys Leu Leu Ala Val Pro Ala Ala
                165                 170                 175

Asn Thr Val Arg Phe Arg Cys Pro Ala Ala Gly Asn Pro Thr Pro Ser
            180                 185                 190

Ile Ser Trp Leu Lys Asn Gly Arg Glu Phe Arg Gly Glu His Arg Ile
        195                 200                 205

Gly Gly Ile Lys Leu Arg His Gln Gln Trp Ser Leu Val Met Glu Ser
    210                 215                 220

Val Val Pro Ser Asp Arg Gly Asn Tyr Thr Cys Val Val Glu Asn Lys
225                 230                 235                 240

Phe Gly Ser Ile Arg Gln Thr Tyr Thr Leu Asp Val Leu Glu Arg Ser
                245                 250                 255

Pro His Arg Pro Ile Leu Gln Ala Gly Leu Pro Ala Asn Gln Thr Ala
            260                 265                 270

Val Leu Gly Ser Asp Val Glu Phe His Cys Lys Val Tyr Ser Asp Ala
        275                 280                 285

Gln Pro His Ile Gln Trp Leu Lys His Val Glu Val Asn Gly Ser Lys
    290                 295                 300

Val Gly Pro Asp Gly Thr Pro Tyr Val Thr Val Leu Lys Val Ser Leu
305                 310                 315                 320

Glu Ser Asn Ala Ser Met Ser Ser Asn Thr Ser Gly Ser Gly Ser Gly
                325                 330                 335

Ser Gly Ser Gly Ser Gly Ser Gly Ser Val Val Phe Pro Tyr Phe Pro
            340                 345                 350

Arg Leu Gly Arg Tyr Asn Leu Asn Phe His Glu Ala Gln Gln Ala Cys
        355                 360                 365

Leu Asp Gln Asp Ala Val Ile Ala Ser Phe Asp Gln Leu Tyr Asp Ala
    370                 375                 380

Trp Arg Gly Gly Leu Asp Trp Cys Asn Ala Gly Trp Leu Ser Asp Gly
385                 390                 395                 400

Ser Val Gln Tyr Pro Ile Thr Lys Pro Arg Glu Pro Cys Gly Gly Gln
                405                 410                 415

Asn Thr Val Pro Gly Val Arg Asn Tyr Gly Phe Trp Asp Lys Asp Lys
            420                 425                 430

Ser Arg Tyr Asp Val Phe Cys Phe Thr Ser Asn Phe Asn Gly Arg Phe
        435                 440                 445

Tyr Tyr Leu Ile His Pro Thr Lys Leu Thr Tyr Asp Glu Ala Val Gln
    450                 455                 460

Ala Cys Leu Asn Asp Gly Ala Gln Ile Ala Lys Val Gly Gln Ile Phe
465                 470                 475                 480

Ala Ala Trp Lys Ile Leu Gly Tyr Asp Arg Cys Asp Ala Gly Trp Leu
                485                 490                 495

Ala Asp Gly Ser Val Arg Tyr Pro Ile Ser Arg Pro Arg Arg Arg Cys
            500                 505                 510

Ser Pro Thr Glu Ala Ala Val Arg Phe Val Gly Phe Pro Asp Lys Lys
        515                 520                 525

His Lys Leu Tyr Gly Val Tyr Cys Phe Arg Ala Tyr Asn
    530                 535                 540

<210>  34

<211>  1614
<212>  DNA
<213>  Artificial Sequence

<220>
<223>  Synthetic Construct

<400>  34
atgggcgctc cagcctgtgc cctggctctg tgtgtggccg tggccattgt ggctggagcc      60

agcagcgaga gcctgggcac agagcagcgg gtggtgggaa gggccgctga agtgcctggc     120

cctgagcctg gccagcagga acagctggtc tttggcagcg gcgacgccgt ggaactgagc     180

tgtcctccac ccggcggagg ccctatgggc cctaccgtgt gggtgaaaga cggcaccggc     240

ctggtgccca gcgagagggt gctggtggga ccccagcggc tgcaggtcct gaacgccagc     300

cacgaggaca gcggcgccta cagctgcaga cagagactga cccagcgggt gctgtgccac     360

ttcagcgtgc gcgtgaccga cgcccctagc tctggcgacg acgaggacgg cgaggacgag     420

gccgaggata ccggcgtgga cacaggcgcc ccttactgga ccagacccga gcggatggac     480

aagaaactgc tggccgtgcc tgccgccaac accgtgcggt tcagatgtcc cgccgctggc     540

aaccccaccc ccagcatcag ctggctgaag aacggcagag agttccgggg cgagcaccgg     600

atcggcggca tcaagctgcg gcaccagcag tggtccctgg tcatggaaag cgtggtgccc     660

tccgaccggg gcaactacac ctgtgtggtg gaaaacaagt tcggcagcat ccggcagacc     720

tacaccctgg acgtgctgga aagaagcccc caccggccca tcctgcaggc tggactgccc     780

gccaatcaga cagccgtgct gggcagcgac gtggaatttc actgcaaggt gtacagcgac     840

gcccagcccc acatccagtg gctcaagcac gtggaagtga acggcagcaa agtgggcccc     900

gacggcaccc cttacgtgac cgtgctgaaa gtgtccctgg aaagcaacgc cagcatgagc     960

agcaacacct gaggtaccag cggcagcggc tctggctctg gaagcggaag cggatctggg    1020

agcgtggtgt tcccctactt cccccggctg ggccggtaca acctgaactt ccacgaggcc    1080

cagcaggctt gcctcgacca ggatgccgtg atcgccagct tcgaccagct gtacgatgcc    1140

tggcggggag gcctggactg gtgcaatgcc ggctggctgt ccgacggcag cgtccagtac    1200

cccatcacca agcccagaga gccctgcggc ggacagaaca cagtgcccgg cgtgcggaac    1260

tacggcttct gggacaagga caagagcaga tacgacgtgt tctgcttcac cagcaacttc    1320

aacggccggt tctactacct gatccacccc accaagctga cctacgacga agccgtccag    1380

gcctgtctga acgacggcgc ccagatcgcc aaagtgggac agatcttcgc cgcctggaag    1440

atcctgggct acgacagatg cgacgccgga tggctggccg atggctccgt gcgctaccct    1500

atcagcagac cccgcagaag atgcagccct accgaggccg ccgtgcgctt cgtgggcttc    1560

cccgacaaga agcacaagct gtacggcgtc tactgcttcc gggcctacaa ctga          1614

## Description

### FIELD OF THE INVENTION

The invention features soluble fibroblast growth factor (FGF) decoy polypeptides and fusion polypeptides including an FGF decoy polypeptide and an aggrecan binding protein. The invention also features methods to prevent or treat skeletal growth retardation disorders, such as achondroplasia.

### BACKGROUND OF THE INVENTION

Fibroblast growth factor receptor 3 (FGFR3) is a member of the fibroblast growth factor (FGFR) family, in which there is high conservation of amino acid sequence between family members. Members of the FGFR family are differentiated by both ligand binding affinities and tissue distribution. A full-length FGFR polypeptide contains an extracellular domain, a single hydrophobic transmembrane domain, and a cytoplasmic tyrosine kinase domain. The extracellular domain (ECD) of FGFR polypeptides interacts with fibroblast growth factors (FGFs) to mediate downstream signaling. This signalling ultimately influences cellular differentiation. In particular, the FGFR3 protein plays a role in bone development by inhibiting chondrocyte proliferation at the growth plate and limiting bone elongation.

Gain-of-function point mutations in FGFR3 are known to cause several types of human skeletal growth retardation disorders, such as achondroplasia, severe achondroplasia with developmental delay and acanthosis nigricans (SADDAN), hypochondroplasia, thanatophoric dysplasia, and craniosynostosis. Achondroplasia is the most common form of short-limb dwarfism and is characterized by disproportionate shortness of limbs and relative macrocephaly. Approximately 97% of achondroplasia is caused by a single point mutation in the gene encoding FGFR3, in which a glycine residue is substituted with an arginine residue at position 380 of the *FGFR3* gene. Following ligand binding, the mutation decreases the elimination of the receptor/ligand complex resulting in prolonged intracellular signaling. This prolonged FGFR3 signaling inhibits the proliferation and the differentiation of the cartilage growth plate, consequently impairing endochondral bone growth.

There exists a need for improved therapeutics that target dysfunctional FGFR3 for treating growth disorders, such achondroplasia.

### SUMMARY OF THE INVENTION

The invention features improved sFGFR3 decoy polypeptides and methods of treatment using the same.

In a first aspect, the invention features a soluble Fibroblast Growth Factor (FGF) decoy polypeptide comprising a protein, said protein including an amino acid sequence including at least amino acid residues 1 to 310 or 1 to 323 of the amino acid sequence of SEQ ID NO: 1, or a variant of the amino acid sequence, wherein the variant has a sequence identity of at least 80% sequence identity (e.g., at least 85%, 86%, 87%, 88%, 89%, 90%, 91%, 92%, 93%, 94%, 95%, 96%, 97%, 98%, 99%, or more sequence identity) to the amino acid sequence of SEQ ID NO: 1 and specifically binds to an FGF (e.g., (FGF1), Fibroblast Growth Factor 2 (FGF2), Fibroblast Growth Factor 9 (FGF9), or Fibroblast Growth Factor (FGF 18)), in the presence or absence of a covalently linked heterologous polypeptide. When the heterologous polypeptide is present, it is generally present as a fusion protein that includes the aforementioned FGF binding moiety.

For example, the sFGF decoy polypeptide includes an amino acid sequence: (i) including at least amino acid residues 1 to 310 of the amino acid sequence of SEQ ID NO: 1, but not including at least amino acid residues 655 to 694 of the amino acid sequence of SEQ ID NO: 1, or a variant of the amino acid sequence, wherein the variant includes an amino acid sequence including at least 80% sequence identity (e.g., at least 85%, 86%, 87%, 88%, 89%, 90%, 91%, 92%, 93%, 94%, 95%, 96%, 97%, 98%, 99%, or more sequence identity) to the amino acid sequence of SEQ ID NO: 1; or (ii) including at least amino acid residues 1 to 357 of the amino acid sequence of SEQ ID NO: 2, but not including at least amino acid residues 703 to 741 of the amino acid sequence of SEQ ID NO: 2, or a variant of the amino acid sequence, wherein the variant includes an amino acid sequence including at least 80% sequence identity (e.g., at least 85%, 86%, 87%, 88%, 89%, 90%, 91%, 92%, 93%, 94%, 95%, 96%, 97%, 98%, 99%, or more sequence identity) to the amino acid sequence of SEQ ID NO: 2. Furthermore, the variant of (i) or (ii) specifically binds to an FGF (e.g., FGF1, FGF2, FGF9, or FGF 18). The fusion protein sFGF decoy polypeptide of the first aspect can also include an amino acid sequence including at least 80% sequence identity (e.g., at least 85%, 86%, 87%, 88%, 89%, 90%, 91%, 92%, 93%, 94%, 95%, 96%, 97%, 98%, 99%, or more sequence identity) to the amino acid sequence of SEQ ID NO: 33, which is a fusion protein including an aggrecan-binding domain.

With respect to the fusion protein sFGF decoy polypeptide of the first aspect of the invention, the heterologous polypeptide can be an aggrecan-binding protein or fragment thereof. For example, the aggrecan-binding protein or fragment thereof can be selected from the group consisting of:
(a) a HPLN1 fragment including an amino acid sequence including at least amino acid residues 158 to 252, amino acid residues 259 to 349, or amino acid residues 158 to 349 of the amino acid sequence of SEQ ID NO: 3, or a variant of the amino acid sequence, in which the variant includes a sequence identity including at least 80% sequence identity (e.g., at least 85%, 86%, 87%, 88%, 89%, 90%, 91%, 92%, 93%, 94%, 95%, 96%, 97%, 98%, 99%, or more sequence identity) to the amino acid sequence of SEQ ID NO: 3, and wherein the variant specifically binds to aggrecan;
(b) HPLN1 or a variant thereof, wherein the variant includes an amino acid sequence including at least 80% sequence identity (e.g., at least 85%, 86%, 87%, 88%, 89%, 90%, 91%, 92%, 93%, 94%, 95%, 96%, 97%, 98%, 99%, or more sequence identity) to the amino acid sequence of SEQ ID NO: 3, wherein the variant specifically binds to aggrecan;
(c) an antibody, an antibody derivative, or an antibody mimetic specific for aggrecan; or
(d) fibulin-1, borrelial aggrecan-binding protein (e.g., Borrelia glycosaminoglycan-binding protein (Bgp) and Borrelia burgdorferi high temperature requirement A (BbHtrA)), Cartilage oligomeric matrix protein/thrombospondin 5 (COMP/TSP5), or aggrecan-binding fragments thereof.

Alternatively, the heterologous polypeptide includes an Fc region.

In a second aspect, the invention features a soluble Fibroblast Growth Factor (sFGF) decoy polypeptide including an amino acid sequence: (i) including at least amino acid residues 1 to 310 or to 323 of the amino acid sequence of SEQ ID NO: 1, wherein the decoy polypeptide has a length of at least 311 amino acids, but not including at least amino acid residues 655 to 694 of the amino acid sequence of SEQ ID NO: 1, or a variant of the amino acid sequence, in which the variant includes an amino acid sequence including at least 80% sequence identity (e.g., at least 85%, 86%, 87%, 88%, 89%, 90%, 91%, 92%, 93%, 94%, 95%, 96%, 97%, 98%, 99%, or more sequence identity) to the amino acid sequence of SEQ ID NO: 1; or (ii) including at least amino acid residues 1 to 357 of the amino acid sequence of SEQ ID NO: 2, but not including at least amino acid residues 703 to 741 of the amino acid sequence of SEQ ID NO: 2, or a variant of the amino acid sequence, in which the variant includes an amino acid sequence including at least 80% sequence identity (e.g., at least 85%, 86%, 87%, 88%, 89%, 90%, 91%, 92%, 93%, 94%, 95%, 96%, 97%, 98%, 99%, or more sequence identity) to the amino acid sequence of SEQ ID NO: 2. In particular, the variant of (i) or (ii) specifically binds to an FGF (e.g., FGF1, FGF2, FGF9, or FGF 18).

In either the first or the second aspect of the invention, the fusion polypeptide or the sFGF decoy polypeptide can include:
(a) an amino acid sequence including amino acid residues 1 to 310 of the amino acid sequence of SEQ ID NO: 1, but not including amino acid residues 324 to 694 of the amino acid sequence of SEQ ID NO: 1, or a variant of the amino acid sequence, in which the variant includes an amino acid sequence including at least 80% (e.g., at least 85%, 86%, 87%, 88%, 89%, 90%, 91%, 92%, 93%, 94%, 95%, 96%, 97%, 98%, 99%, or more sequence identity) sequence identity to the amino acid sequence of SEQ ID NO: 1;
(b) an amino acid sequence including amino acid residues 1 to 357 of the amino acid sequence of SEQ ID NO: 2, but not including amino acid residues 371 to 741 of the amino acid sequence of SEQ ID NO:2, or a variant of the amino acid sequence, in which the variant includes an amino acid sequence including at least 80% sequence identity (e.g., at least 85%, 86%, 87%, 88%, 89%, 90%, 91%, 92%, 93%, 94%, 95%, 96%, 97%, 98%, 99%, or more sequence identity) to the amino acid sequence of SEQ ID NO: 2;
(c) an amino acid sequence including amino acid residues 1 to 310 of the amino acid sequence of SEQ ID NO: 1, but not including amino acid residues 441 to 694 of the amino acid sequence of SEQ ID NO: 1, or a variant of the amino acid sequence, in which the variant includes an amino acid sequence including at least 80% sequence identity (e.g., at least 85%, 86%, 87%, 88%, 89%, 90%, 91%, 92%, 93%, 94%, 95%, 96%, 97%, 98%, 99%, or more sequence identity) to the amino acid sequence of SEQ ID NO: 1;
(d) an amino acid sequence including amino acid residues 1 to 357 of the amino acid sequence of SEQ ID NO: 2, but not including amino acid residues 488 to 741 of the amino acid sequence of SEQ ID NO:2, or a variant of the amino acid sequence, in which the variant includes an amino acid sequence including at least 80% sequence identity (e.g., at least 85%, 86%, 87%, 88%, 89%, 90%, 91%, 92%, 93%, 94%, 95%, 96%, 97%, 98%, 99%, or more sequence identity) to the amino acid sequence of SEQ ID NO: 2;
(e) an amino acid sequence including amino acid residues 1 to 310 of the amino acid sequence of SEQ ID NO: 1, but not including amino acid residues 549 to 694 of the amino acid sequence of SEQ ID NO: 1, or a variant of the amino acid sequence, in which the variant includes an amino acid sequence including at least 80% sequence identity (e.g., at least 85%, 86%, 87%, 88%, 89%, 90%, 91%, 92%, 93%, 94%, 95%, 96%, 97%, 98%, 99%, or more sequence identity) to the amino acid sequence of SEQ ID NO: 1; or
(f) an amino acid sequence including amino acid residues 1 to 357 of the amino acid sequence of SEQ ID NO: 2, but not including amino acid residues 596 to 741 of the amino acid sequence of SEQ ID NO:2, or a variant of the amino acid sequence, in which the variant includes an amino acid sequence including at least 80% sequence identity to the amino acid sequence of SEQ ID NO: 2.

Furthermore, the variant of (a)-(f) specifically binds to an FGF (e.g., FGF1, FGF2, FGF9, or FGF 18).

Additionally, the sFGF decoy polypeptide can include (i) an amino acid sequence including amino acid residues 1 to 325 of the amino acid sequence of SEQ ID NO: 33, or (ii) an amino acid sequence including amino acid residues 1 to 338 of the amino acid sequence of SEQ ID NO: 33, or a variant of the amino acid sequence, in which the variant of (i) or (ii) includes an amino acid sequence including at least 80% sequence identity (e.g., at least 85%, 86%, 87%, 88%, 89%, 90%, 91%, 92%, 93%, 94%, 95%, 96%, 97%, 98%, 99%, or more sequence identity) to the amino acid sequence of SEQ ID NO: 33.

In another embodiment of the second aspect of the invention, the sFGF decoy polypeptide includes amino acids 1 to 310 of SEQ ID NO: 1 and also has a length of at least 311 amino acids.

In a third aspect, the invention features a nucleic acid encoding the fusion protein of the first aspect or the sFGF decoy polypeptide of the second aspect.

In a fourth aspect, the invention features a vector (e.g., a viral or other vector described herein) comprising the nucleic acid of the third aspect.

In a fifth aspect, the invention features a cell (such as a cell described herein) comprising the fusion protein of the first aspect, the sFGF decoy polypeptide of the second aspect, the nucleic acid of the third aspect, or the vector of the fourth aspect.

In a sixth aspect, the invention features the fusion protein of the first aspect, the sFGF decoy polypeptide of the second aspect, the nucleic acid of the third aspect, the vector of the fourth aspect or the cell of the fifth aspect for use as a medicament, in particular for use in the prevention or treatment of a skeletal growth retardation disorder, such as achondroplasia, in a subject in need thereof (e.g., a human). For instance, the skeletal growth retardation disorder is a FGFR3-related skeletal disease. The skeletal growth retardation disorder can be selected from the group consisting of achondroplasia, thanatophoric dysplasia type I (TDI), thanatophoric dysplasia type II (TDII), severe achondroplasia with developmental delay and acanthosis nigricans (SADDAN), hypochondroplasia, and a craniosynostosis syndrome. In particular, the skeletal growth retardation disorder is achondroplasia. Furthermore, the craniosynostosis syndrome is selected from the group consisting of Muenke syndrome, Crouzon syndrome, Apert syndrome, Jackson-Weiss syndrome, Pfeiffer syndrome, and Crouzonodermoskeletal syndrome.

The invention also features the fusion polypeptide of the first aspect or the sFGF decoy polypeptide of the second aspect formulated to provide about 0.0002 mg/kg/day to about 20 mg/kg/day of the fusion polypeptide or the sFGF decoy polypeptide to the subject in need thereof (e.g., a human). In particular, the fusion polypeptide or the sFGF decoy polypeptide are formulated to provide about 0.001 mg/kg/day to about 7 mg/kg/day of the fusion polypeptide or the sFGF decoy polypeptide to the subject in need thereof (e.g., a human).

In a seventh aspect, the invention features a method for monitoring the treatment of a skeletal growth retardation disorder including measuring body weight and/or skull size of a subject in need thereof (e.g., a human). In particular, body weight and/or skull size of the subject in need thereof (e.g., a human) are measured in response to administration of the fusion protein of the first aspect, the sFGF decoy polypeptide of the second aspect, the nucleic acid of the third aspect, the vector of the fourth aspect or the cell of the fifth aspect to the subject in need thereof (e.g., a human).

The invention also features a pharmaceutical composition including the fusion protein of the first aspect, the sFGF decoy polypeptide of the second aspect, the nucleic acid of the third aspect, the vector of the fourth aspect or the cell of the fifth aspect, and a pharmaceutically acceptable carrier for use in the prevention or treatment of a skeletal growth retardation disorder. The pharmaceutical composition can be formulated for subcutaneous, topical, oral, intranasal, intraocular, intravenous, or intramuscular administration. In particular, the pharmaceutical composition is formulated for subcutaneous administration.

### Definitions

Before the present invention is described in detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

Preferably, the terms used herein are defined as described in "A multilingual glossary of biotechnological terms: (IUPAC Recommendations)", Leuenberger, H.G.W, Nagel, B. and Kölbl, H. eds. (1995), Helvetica Chimica Acta, CH-4010 Basel, Switzerland).

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine the explicitly described embodiments with any number of the disclosed and/or preferred elements. Furthermore, any permutations and combinations of all described elements in this application should be considered disclosed by the description of the present application unless the context indicates otherwise.

Throughout this specification and the claims which follow, unless the context requires otherwise, the word "comprise", and variations such as "comprises" and "comprising", are to be understood to imply the inclusion of a stated integer or step or group of integers or steps but not the exclusion of any other integer or step or group of integer or step. As used in this specification and the appended claims, the singular forms "a", "an", and "the" include plural referents, unless the content clearly dictates otherwise.

The term "aggrecan" as used herein refers to a protein that in humans is encoded by the *ACAN* gene. It is also known as cartilage-specific proteoglycan core protein (CSPCP) and chondroitin sulfate proteoglycan 1. The gene is a member of the lectican (chondrotoitin sulfate proteoglycan) family. The encoded protein is an integral part of the extracellular matrix in cartilagenous tissue and it withstands compression in cartilage. The protein is 2316 amino acids long and can be expressed in multiple isoforms fue to alternative splicing. The amino acid sequence can be obtained from the NCBI database (RefSeq accession number NP_001126).

An "aggrecan-binding protein" is a binding protein or fragment thereof that specifically binds to aggrecan, e.g., any protein or fragment thereof that interacts with the globular domain (G1, G2, or G3) of aggrecan. Exemplary aggrecan-binding proteins can include antibodies, fibulin-1, borrelial aggrecan-binding proteins (e.g., Borrelia glycosaminoglycan-binding protein (Bgp) and Borrelia burgdorferi high temperature requirement A (BbHtrA)), and cartilage oligomeric matrix protein/thrombospondin 5 (COMP/TSP5). For example, an aggrecan-binding protein may include human hyaluronan and proteoglycan link protein 1 (HPLN1) or fragments thereof. In particular, the HPLN1 fragment can include a cartilage link domain 1 (LK1; amino acids 158 to 252 of SEQ ID NO: 3), a cartilage link domain 2 (LK2; amino acids 259 to 349 of SEQ ID NO: 3), or both LK1 and LK2 domains (158 to 349 amino acids of SEQ ID NO: 3). Preferably, the HPLN1 fragment has an amino acid sequence excluding at least residues 53 to 140 of SEQ ID NO: 3, and/or excluding amino acids 1 to 140 or 1 to 157 of SEQ ID NO: 3. For example, an aggrecan-binding protein, such as HPLN1 or fragments thereof (e.g., amino acids 158 to 349 (LK1-LK2 domains) of SEQ ID NO: 3), may be fused to a sFGFR3 polypeptide fragment, such as a sFGFR3 polypeptide fragment including, e.g., amino acids 1 to 323 of SEQ ID NO: 1, in a sFGFR3 fusion polypeptide.

The term "binding protein" as used herein refers to proteins that specifically bind to another molecule. It is understood that the term includes fragments of the polypeptide so long as binding function is retained. Such binding proteins can include single binding domain proteins as well as multi-binding domain proteins. Multi-binding domain proteins include those proteins which contain two or more discreet and noncontiguous regions along the primary structure of the polypeptide which together contribute to the overall biding activity. A specific example of multi-binding domain proteins includes heavy and light antibody chains since the binding activity of each is derived from three noncontiguous complementarity determining regions. Binding proteins can be monomeric or multimeric species. Heteromeric binding proteins are a specific example of multimeric binding proteins which are composed of at least two different subunits which together exhibit binding activity toward a particular ligand. It is understood that when referring to multimeric binding proteins that the term includes fragments of the subunits so long as assembly of the polypeptides and binding function of the assembled complex is retained. Heteromeric binding proteins include, for example, antibodies and fragments thereof such as Fab and (Fab')₂ portions.

The term "biological activity of the parent polypeptide" refers to any activity or the parent polypeptide inside or outside a cell. For example, with respect to an aggrecan-binding protein, it is the ability to specifically bind aggrecan. With respect to the FGF decoy polypeptide, the term refers to (i) the capacity to specifically bind to FGF (e.g., FGF2); (ii) the capacity to reduce FGF intracellular signaling (e.g. Erk phosphorylation following FGFR3 receptor activation by its binding with FGFs); and/or (iii) the capacity to restore bone growth *in vivo* (e.g. in *Fgfr3*^{*ach*/*+*} mice).

The skilled in the art can easily determine whether a fragment or variant of a parent polypeptide is biologically active. It is noted that functional activation of the FGFR3 receptor may be readily assessed by the one skilled in the art according to known methods, as well as those described herein. Indeed, since activated FGFR3 receptor is phosphorylated on tyrosine residues located towards the cytoplasmic domain, i.e., on Tyr648 and Tyr647, functional activation of the FGFR3 receptor may for example be assessed by measuring its phosphorylation. For instance, analysis of ligand-induced phosphorylation of the FGFR3 receptor can be performed as described in Le Corre et al. (Org. Biomol. Chem., 8: 2164-2173, 2010). Alternatively, receptor phosphorylation in cells can be readily detected by immunocytochemistry, immunohistochemistry and/or flow cytometry using antibodies which specifically recognize this modification. For instance phosphorylation of FGFR3 on the Tyr648 and Tyr647 residues can be detected by immunocytochemistry, immunohistochemistry and/or flow cytometry using monoclonal or polyclonal antibodies directed against phosphorylated Tyr648 and Tyr647-FGFR3. Further, FGFR3, when associated with its ligand, mediates signaling by activating the ERK and p38 MAP kinase pathways, and the STAT pathway. Therefore, activation of FGFR3 receptor can also be assessed by determining the activation of these specific pathways as described by Horton et al. (Lancet, 370: 162-172, 2007).

The term "cell culture" refers to the process by which cells are grown under controlled conditions outside of their natural environment in or on a cell culture medium. The term "cell culture medium" refers to a liquid or gel for supporting the survival or growth of cells, especially cells as defined above, for example cells derived from multi-cellular eukaryotes, in particular animal cells. Such a medium comprises all nutrients required to support the survival or growth of such cells. The cell culture medium composition can be a dry powder composition, a liquid composition or a solid (e.g. gel or agar) composition. Suitable cell cultures and culturing techniques are well known in the art, see for example Peterson et al., Comp Immunol Microbiol Infect Dis. 1988;11(2):93-8.

The term "codon-optimized" as used herein refers to the alteration of codons in the nucleic acid, in particular to reflect the typical codon usage of the host organism without altering the polypeptide encoded by the DNA. The host cell or host organism is preferably human. Codon-optimization can be performed by the skilled person without undue burden, e.g. by using online tools such as the JAVA Codon Adaption Tool (www.jcat.de) or Integrated DNA Technologies Tool (www.eu.idtdna.com/CodonOpt) by simply entering the nucleic acid sequence and the host organism for which the codons are to be optimized. The codon usage of different organisms is available in online databases, for example, on www.kazusa.or.jp/codon (to date 35,799 different organisms).

Optionally, the nucleic acid of the third aspect may be codon-optimized. With respect to the nucleic acid encoding for the fusion protein of the first aspect, only a part of the nucleic acid may be codon optimized, in particular one or more of the parts encoding for the detectable marker, the FGF decoy polypeptide, the peptide linker and/or the aggrecan-binding protein. With respect to the nucleic acid encoding for the FGF decoy polypeptide protein of the first aspect, only a part of the nucleic acid may be codon optimized, in particular one or more of the parts encoding for the detectable marker and/or the FGF decoy polypeptide. Preferably, the nucleic acid is codon-optimized to facilitate genetic manipulations by decreasing the GC content and/or for expression in a host cell.

The term "decoy" or "decoy receptor", also known as "sink" or "trap", refers to a receptor that binds a ligand, but is structurally incapable of signaling or presenting the agonist to signaling receptor complexes. A decoy acts as a molecular trap for the ligand, thereby preventing it from binding to its functional receptor. A decoy is preferably soluble. A decoy can be a sFGFR3 polypeptide or a fragment thereof as described herein.

The term "detectable label", "marker" or "tag" as used herein refers to any kind of substance which is able to indicate the presence of another substance or complex of substances, in particular of the fusion protein of the first aspect or of the soluble FGF decoy polypetide of the second aspect. The detectable label can be a substance that is linked to or introduced in the substance to be detected. Preferred is a detectable label suitable for allowing for purification, quantification and/or *in vivo* detection. Examples of suitable labels include, but are not limited to, a fluorophore, a chromophore, a radiolabel, a metal colloid, an enzyme, or a chemiluminescent or bioluminescent molecule. In the context of the present invention suitable tags are preferably protein tags whose peptide sequences are genetically grafted into or onto a recombinant protein. Protein tags may e.g. encompass affinity tags, solubilization tags, chromatography tags, epitope tags, or fluorescence tags. Affinity tags are appended to proteins so that they can be purified from their crude biological source using an affinity technique. These include chitin binding protein (CBP), maltose binding protein (MBP), and glutathione-S-transferase (GST). The poly(His) tag is a widely used protein tag which binds to metal matrices. Solubilization tags are used, especially for recombinant proteins expressed in chaperone-deficient species to assist in the proper folding in proteins and keep them from precipitating. These include thioredoxin (TRX) and poly(NANP). Some affinity tags have a dual role as a solubilization agent, such as MBP, and GST. Chromatography tags are used to alter chromatographic properties of the protein to afford different resolution across a particular separation technique. Often, these consist of polyanionic amino acids, such as FLAG-tag. Epitope tags are short peptide sequences which are chosen because high-affinity antibodies can be reliably produced in many different species. These are usually derived from viral genes, which explain their high immunoreactivity. Epitope tags include V5-tag, Myc-tag, and HA-tag. These tags are particularly useful for western blotting, immunofluorescence and immunoprecipitation experiments, although they also find use in antibody purification. Fluorescence tags are used to give visual readout on a protein. GFP and its variants (e.g. mutant GFPs having a different fluorescent spectrum) and RFP and its variants (e.g., mutant RFPs having a different fluorescent spectrum) are the most commonly used fluorescence tags. More advanced applications of GFP/RFP include using it as a folding reporter (fluorescent if folded, colorless if not). Further examples of fluorophores include fluorescein, rhodamine, and sulfoindocyanine dye Cy5.

Preferred examples of a detectable label include but are not limited to AviTag (a peptide allowing biotinylation by the enzyme BirA and isolation by streptavidin (GLNDIFEAQKIEWHE, SEQ ID NO: 11)), Calmodulin-tag (a peptide bound by the protein calmodulin (KRRWKKNFIAVSAANRFKKISSSGAL, SEQ ID NO: 12)), polyglutamate tag (a peptide binding efficiently to anion-exchange resin such as Mono-Q (EEEEEE, SEQ ID NO: 13)), E-tag (a peptide recognized by an antibody (GAPVPYPDPLEPR, SEQ ID NO: 14)), FLAG-tag (a peptide recognized by an antibody (DYKDDDDK, SEQ ID NO: 15)), HA-tag (a peptide recognized by an antibody (YPYDVPDYA, SEQ ID NO: 16)), His-tag (5-10 histidines bound by a nickel or cobalt chelate (HHHHHH, SEQ ID NO: 17)), Myc-tag (a short peptide recognized by an antibody (EQKLISEEDL, SEQ ID NO: 18)), S-tag (KETAAAKFERQHMDS, SEQ ID NO: 19), SBP-tag (a peptide which binds to streptavidin (MDEKTTGWRGGHVVEGLAGELEQLRA RLEHHPQGQREP, SEQ ID NO: 20)), Softag 1 (for mammalian expression (SLAELLNAGLGGS, SEQ ID NO: 21)), Softag 3 (for prokaryotic expression (TQDPSRVG, SEQ ID NO: 22)), Strep-tag (a peptide which binds to streptavidin or the modified streptavidin called streptactin (Strep-tag II: WSHPQFEK, SEQ ID NO: 23)), TC tag (a tetracysteine tag that is recognized by FlAsH and ReAsH biarsenical compounds (CCPGCC, SEQ ID NO: 24)), V5 tag (a peptide recognized by an antibody (GKPIPNPLLGLDST, SEQ ID NO: 25)), VSV-tag (a peptide recognized by an antibody (YTDIEMNRLGK, SEQ ID NO: 26)), Xpress tag (DLYDDDDK, SEQ ID NO: 27), Isopeptag (a peptide which binds covalently to pilin-C protein (TDKDMTITFTNKKDAE, SEQ ID NO: 28)), SpyTag (a peptide which binds covalently to SpyCatcher protein (AHIVMVDAYKPTK, SEQ ID NO: 29)), BCCP (Biotin Carboxyl Carrier Protein, a protein domain biotinylated by BirA enabling recognition by streptavidin), Glutathione-S-transferase-tag (a protein which binds to immobilized glutathione), Green fluorescent protein-tag (a protein which is spontaneously fluorescent and can be bound by nanobodies), Maltose binding protein-tag (a protein which binds to amylose agarose), Nus-tag, Thioredoxin-tag, Fc-tag (derived from immunoglobulin Fc domain), Ty tag, Designed Intrinsically Disordered tags containing disorder promoting amino acids (P,E,S,T,A,Q,G; see Minde et al., Designing disorder: Tales of the unexpected tails. Intrinsically Disord. Proteins 1, e26790 1-5 (2013)).

The detectable label can be linked to the fusion protein via a cleavable sequence. Preferably, the sequence is recognizable and cleavable by a protease. The protease is preferably an enterokinase. In particular, it is preferred that the first (i.e., the N-terminal) residue of the cleavable sequence is not proline. The length of the cleavable sequence is preferably 2-20, 3-15 or 4-10 amino acids. An exemplary cleavable sequence is LVDQIPA (SEQ ID NO: 30). Preferably, the detectable label is at the N-terminus or at the C-terminus of the fusion protein of the first aspect, more preferably it is at the N-terminus. For example, the detectable label, such as a FLAG-tag (e.g., SEQ ID NO: 15) may be at the N-terminus of the sFGFR3 fusion polypeptide (see, e.g., amino acid residues 1 to 8 of SEQ ID NO: 4). Alternatively, the detectable label, such as a FLAG-tag (e.g., SEQ ID NO: 15), may be at the C-terminus of the sFGFR3 fusion polypeptide. Additionally, a detectable label, such as a FLAG-tag (e.g., SEQ ID NO: 15), may be at the N-terminus or at the C-terminus of a sFGFR3 polypeptide, e.g., sFGFR3_Del2 (amino acids 1 to 548 of SEQ ID NO: 1), sFGFR3_Del3 (amino acids 1 to 440 of SEQ ID NO: 1), and sFGFR3_Del4 (amino acids 1 to 323 of SEQ ID NO: 1).

The term "eukaryotic cell" is in particular a yeast or an animal cell. A yeast cell can be, in the broadest sense, any cell of a yeast organism, for example a cell from *Kluyveromyces lactis, Kluyveromyces marxianus var.marxianus, Kluyveromyces thermotolerans, Candida utilis, Candida tropicalis, Candida albicans, Candida lipolytica* and *Candida versatilis,* of the genus *Pichia* like *Pichia stipidis, Piachia pastoris* and *Pichia sorbitophila, Cryptococcus, Debaromyces, Hansenula, Saccharomycecopsis, Saccharomycodes, Schizosaccharomyces, Wickerhamia, Debayomyces, Hanseniaspora, Kloeckera, Zygosaccharomyces, Ogataea, Kuraishia, Komagataella, Metschnikowia, Williopsis, Nakazawaea, Cryptococcus, Torulaspora, Bullera, Rhodotorula, Willopsis* or *Sporobolomyces.* Preferably, though, the yeast cell is a *Saccharomyces* cell, in particular a *Saccharomyces cerevisiae* cell.

An animal cell may be a cell of a primate, mouse, rat, rabbit, dog, cat, hamster, cow, insect (e.g. Sf9 or Sf21) etc., preferably a human. Also, it may be a suspension or an adherent cell. A suspension cell is a cell that may naturally live in suspension (i.e. without being attached to a surface), or a cell that has been modified to be able to survive in suspension cultures, for example to be grown to higher densities than adherent conditions would allow. An adherent cell is a cell that requires a surface, such as tissue culture plastic or a microcarrier, which may be coated with extracellular matrix components (such as collagen and laminin) to increase adhesion properties and provide other signals needed for growth and differentiation. In one embodiment, the adherent cell is a monolayer cell. For example, the cell is selected from the group consisting of a hybridoma cell, a primary epithelial cell, an endothelial cell, a keratinocyte, a monocyte/macrophage, a lymphocyte, a hematopoietic stem cell, a fibroblast, a chondrocyte and a hepatocyte. More specifically, it may be selected from the group consisting of a CHO-K1 SV cell, a CHO DG44 cell, a CHO DP-12 cell, a CHO DHFR- cell, a CHO-GS cell, a BHK-21 cell, a HEK-293 embryonic kidney cell, a HeLa cervical epithelial cell, a PER-C6 retinal cell, an MDCK cell, an HDMEC cell, a HepG2 cell, an HL-60 cell, an HMEC-1 cell, a HUVEC cell, an HT1080 cell, a Jurkat cell, an MRC5 cell,a K562 cell, a HeLa cell, an NS0 cell, an Sp20 cell, a COS cell, and a VERO cell.

The term "extracellular domain" or "ECD" refers to the portion of a polypeptide (e.g., a FGFR3 polypeptide) that extends beyond the transmembrane domain into the extracellular space. As is described above, the ECD includes the IgG1 domain (aa 57-110), the IgG2 domain (aa 161-245), and the IgG3 domain (aa 268-357). An ECD of a FGFR3 may include a complete ECD (aa 57-110 of FGFR3) or may include a fragment of the ECD, such as an FGFR3 ECD that is lacking one or more amino acids present in the full length FGFR3 ECD. The ECD mediates binding of FGFRs to one or more FGFs. For instance, a soluble FGFR (e.g., sFGFR3) will include at least a portion of the ECD of the FGFR polypeptide. Exemplary ECDs of FGFR3 polypeptides may include, e.g., at least amino acids 1 to 300, 1 to 310, 1 to 320, 1 to 330, 1 to 340, 1 to 350, 1 to 360, or 1 to 365 of SEQ ID NO: 1.

In the context of the invention, the term "FGFR3-related skeletal disease" is intended to mean a skeletal disease that is caused by an abnormal increased activation of FGFR3, in particular by expression of a gain-of-function mutant of the FGFR3 receptor. As used herein, the terms "gain-of-function FGFR3 receptor variant", "gain-of-function mutant of the FGFR3" or "mutant FGFR3 displaying a prolonged activity" are used interchangeably and refer to a mutant of said receptor exhibiting a biological activity (i.e., triggering downstream signaling) which is higher than the biological activity of the corresponding wild-type receptor in the presence of FGF ligand. The FGFR3-related skeletal diseases are preferably FGFR3-related skeletal dysplasias and FGFR3-related craniosynostosis. The FGFR3-related skeletal dysplasias according to the invention may correspond to an inherited or to a sporadic disease. FGFR3-related skeletal dysplasias can include, but are not limited to, thanatophoric dysplasia type I, thanatophoric dysplasia type II, hypochondroplasia, achondroplasia and SADDAN (severe achondroplasia with developmental delay and acanthosis nigricans), Crouzon syndrome with acnthosis nigricans, Muenke syndrome, and CATSHL (camptodactyly, tall stature, scoliosis, and hearing loss).

For example, the FGFR3-related skeletal dysplasia is an achondroplasia caused by expression of the G380R gain-of-function mutant of the FGFR3 receptor. Alternatively, the FGFR3-related skeletal dysplasia is an achondroplasia caused by expression of the G375C, G346E, or S279C of the FGFR3 receptor. It is further noted that achondroplasia caused by another mutant of the FGFR3 receptor which would be identified in the future is also encompassed. The FGFR3-related skeletal dysplasia can also be a hypochondroplasia caused by expression of the N540K, K650N, K650Q, S84L, R200C, N262H, G268C, Y278C, V381E, gain-of-function mutant of the FGFR3 receptor. The FGFR3-related skeletal dysplasia can further be a thanatophoric dysplasia type I caused by expression of a gain-of-function mutant of the FGFR3 receptor chosen from the group consisting of R248C, S248C, G370C, S371C; Y373C, X807R, X807C, X807G, X807S, X807W and K650M FGFR3 receptors or a thanatophoric dysplasia type II caused by expression of the K650E gain-of-function mutant of the FGFR3 receptor. Additionally, the FGFR3-related skeletal dysplasia can be a severe achondroplasia with developmental delay and acanthosis nigricans caused by expression of the K650M gain-of-function mutant of the FGFR3 receptor.

The term "Fibroblast Growth Factor" or "FGF" refers to a member of the FGF family, which in humans comprises structurally related signaling molecules. The term preferably refers to any of FGF1, FGF2, FGF3, FGF4, FGF5, FGF6, FGF7, FGF8, FGF9, FGF10 and FGF18, which all bind fibroblast growth factor receptors (FGFRs). More preferably, it refers to at least FGF1, FGF2, FGF9 and/or FGF 18, which have been shown to bind FGFR3. Thus, the FGF referred to in all aspects and embodiments described herein is preferably FGF1, FGF2, FGF9 and/or FGF 18. Nevertheless, it must be borne in mind that other FGFs may also bind FGFR3 and are therefore also within the scope of the term FGF according to the invention.

A preferred FGF1 is human FGF1 having the amino acid sequence according to SEQ ID NO: 7. A preferred FGF2 is human FGF2 having the amino acid sequence according to SEQ ID NO: 8. A preferred FGF9 is human FGF9 having the amino acid sequence according to SEQ ID NO: 9. A preferred FGF18 is human FGF18 having the amino acid sequence according to SEQ ID NO: 10.

The term "Fibroblast Growth Factor Receptor 3", "FGFR3" or "FGFR3 receptor", as used herein, refers to any naturally occurring and functionally signalling FGFR3 polypeptide. In particular, FGFR3 refers to a polypeptide that specifically binds one or more FGFs (e.g., FGF1, FGF2, FGF9 and/or FGF 18). The human *FGFR3* gene, which is located on the distal short arm of chromosome 4, encodes an 806 amino acid protein precursor (fibroblast growth factor receptor 3 isoform 1 precursor), which contains 19 exons. FGFRs (e.g., FGFR3) are tyrosine kinases spanning the cellular membrane, which are activated by binding of ligands of the FGF family of growth factors. FGFR3 and other FGFRs consist of three extracellular immunoglobulin-like domains of a membrane spanning domain and of two intracellular tyrosine kinase domains. FGFR3 can include all or fragment thereof and/or any mutation of a FGFR3 polypeptide, including soluble fragments of FGFR3, as well as polymorphic forms and splice variants. Mutations in the *FGFR3* gene lead to craniosynostosis and multiple types of skeletal dysplasia, such as substitutions of a glycine residue at position 380 with an arginine residue (i.e., G380R). Alternative splicing occurs and additional variants have been described, including those utilizing alternate exon 8 rather than 9, but their full-length nature has not been determined. The FGFR3 receptor comprises an extracellular domain (amino acid residues 1 to 366), a transmembrane domain (amino acid residues 367 to 399) and an intracellular domain (amino acid residues 400 to 806). The extracellular domain comprises the IgG1 domain (amino acid residues 57 to 110), the IgG2 domain (amino acid residues 161 to 245) and the IgG3 domain (amino acid residues 268 to 357). The intracellular domain comprises two tyrosine kinase domains (amino acid residues 459 to 792). The naturally occurring human FGFR3 gene has a nucleotide sequence as shown in Genbank Accession number NM_000142.4 and the naturally occurring human FGFR3 protein has an amino acid sequence as shown in Genbank Accession number NP_000133, herein represented by SEQ ID NO: 6.

The term "fragment" is meant a portion of a polypeptide or nucleic acid molecule that contains, preferably, at least 10%, 20%, 30%, 40%, 50%, 60%, 70%, 75%, 80%, 85%, 90%, 95%, 96%, 97%, 98%, 99%, or more of the entire length of the reference nucleic acid molecule or polypeptide. A fragment may contain, e.g., 10, 15, 20, 25, 30, 35, 40, 45, 50, 55, 60, 65, 70, 75, 80, 85, 90, 95, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 500, 600, 700, or more amino acid residues, up to the entire length of the reference polypeptide (e.g., FGFR3).

For example, a FGFR3 polypeptide fragment may include any polypeptide having at least 50% (e.g., 55%, 60%, 65%, 70%, 75%, 80%, 85%, 86%, 87%, 88%, 89%, 90%, 91%, 92%, 93%, 94%, 95%, 96%, 97%, 98%, 99%, or more) sequence identity to a fragment of SEQ ID NO: 1, e.g., at least amino acids 1 to 300, 1 to 310, 1 to 320, 1 to 330, 1 to 340, 1 to 350, 1 to 360, 1 to 370, 1 to 380, 1 to 390, 1 to 400, 1 to 410, 1 to 420, 1 to 430, 1 to 440, 1 to 440, 1 to 450, 1 to 460, 1 to 470, 1 to 480, 1 to 490, 1 to 500, 1 to 510, 1 to 520, 1 to 530, 1 to 540, 1 to 550, 1 to 560, 1 to 570, 1 to 580, 1 to 590, 1 to 600, 1 to 610, 1 to 620, 1 to 630, 1 to 640, 1 to 650, 1 to 660, 1 to 670, or 1 to 680 of SEQ ID NO: 1. For example, a FGFR3 polypeptide fragment may include any polypeptide having at least 50% sequence identity to a fragment of SEQ ID NO: 1, e.g., at least amino acids 1 to 300, 1 to 310, 1 to 320, 1 to 330, 1 to 340, 1 to 350, 1 to 360, 1 to 370, 1 to 380, 1 to 390, 1 to 400, 1 to 410, 1 to 420, 1 to 430, 1 to 440, 1 to 440, 1 to 450, 1 to 460, 1 to 470, 1 to 480, 1 to 490, 1 to 500, 1 to 510, 1 to 520, 1 to 530, 1 to 540, 1 to 550, 1 to 560, 1 to 570, 1 to 580, 1 to 590, 1 to 600, 1 to 610, 1 to 620, 1 to 630, 1 to 640, 1 to 650, 1 to 660, 1 to 670, or 1 to 680 of SEQ ID NO: 1. For example, a FGFR3 polypeptide fragment may include any polypeptide having at least 55% sequence identity to a fragment of SEQ ID NO: 1, e.g., at least amino acids 1 to 300, 1 to 310, 1 to 320, 1 to 330, 1 to 340, 1 to 350, 1 to 360, 1 to 370, 1 to 380, 1 to 390, 1 to 400, 1 to 410, 1 to 420, 1 to 430, 1 to 440, 1 to 440, 1 to 450, 1 to 460, 1 to 470, 1 to 480, 1 to 490, 1 to 500, 1 to 510, 1 to 520, 1 to 530, 1 to 540, 1 to 550, 1 to 560, 1 to 570, 1 to 580, 1 to 590, 1 to 600, 1 to 610, 1 to 620, 1 to 630, 1 to 640, 1 to 650, 1 to 660, 1 to 670, or 1 to 680 of SEQ ID NO: 1. For example, a FGFR3 polypeptide fragment may include any polypeptide having at least 60% sequence identity to a fragment of SEQ ID NO: 1, e.g., at least amino acids 1 to 300, 1 to 310, 1 to 320, 1 to 330, 1 to 340, 1 to 350, 1 to 360, 1 to 370, 1 to 380, 1 to 390, 1 to 400, 1 to 410, 1 to 420, 1 to 430, 1 to 440, 1 to 440, 1 to 450, 1 to 460, 1 to 470, 1 to 480, 1 to 490, 1 to 500, 1 to 510, 1 to 520, 1 to 530, 1 to 540, 1 to 550, 1 to 560, 1 to 570, 1 to 580, 1 to 590, 1 to 600, 1 to 610, 1 to 620, 1 to 630, 1 to 640, 1 to 650, 1 to 660, 1 to 670, or 1 to 680 of SEQ ID NO: 1. For example, a FGFR3 polypeptide fragment may include any polypeptide having at least 65% sequence identity to a fragment of SEQ ID NO: 1, e.g., at least amino acids 1 to 300, 1 to 310, 1 to 320, 1 to 330, 1 to 340, 1 to 350, 1 to 360, 1 to 370, 1 to 380, 1 to 390, 1 to 400, 1 to 410, 1 to 420, 1 to 430, 1 to 440, 1 to 440, 1 to 450, 1 to 460, 1 to 470, 1 to 480, 1 to 490, 1 to 500, 1 to 510, 1 to 520, 1 to 530, 1 to 540, 1 to 550, 1 to 560, 1 to 570, 1 to 580, 1 to 590, 1 to 600, 1 to 610, 1 to 620, 1 to 630, 1 to 640, 1 to 650, 1 to 660, 1 to 670, or 1 to 680 of SEQ ID NO: 1. For example, a FGFR3 polypeptide fragment may include any polypeptide having at least 70% sequence identity to a fragment of SEQ ID NO: 1, e.g., at least amino acids 1 to 300, 1 to 310, 1 to 320, 1 to 330, 1 to 340, 1 to 350, 1 to 360, 1 to 370, 1 to 380, 1 to 390, 1 to 400, 1 to 410, 1 to 420, 1 to 430, 1 to 440, 1 to 440, 1 to 450, 1 to 460, 1 to 470, 1 to 480, 1 to 490, 1 to 500, 1 to 510, 1 to 520, 1 to 530, 1 to 540, 1 to 550, 1 to 560, 1 to 570, 1 to 580, 1 to 590, 1 to 600, 1 to 610, 1 to 620, 1 to 630, 1 to 640, 1 to 650, 1 to 660, 1 to 670, or 1 to 680 of SEQ ID NO: 1. For example, a FGFR3 polypeptide fragment may include any polypeptide having at least 75% sequence identity to a fragment of SEQ ID NO: 1, e.g., at least amino acids 1 to 300, 1 to 310, 1 to 320, 1 to 330, 1 to 340, 1 to 350, 1 to 360, 1 to 370, 1 to 380, 1 to 390, 1 to 400, 1 to 410, 1 to 420, 1 to 430, 1 to 440, 1 to 440, 1 to 450, 1 to 460, 1 to 470, 1 to 480, 1 to 490, 1 to 500, 1 to 510, 1 to 520, 1 to 530, 1 to 540, 1 to 550, 1 to 560, 1 to 570, 1 to 580, 1 to 590, 1 to 600, 1 to 610, 1 to 620, 1 to 630, 1 to 640, 1 to 650, 1 to 660, 1 to 670, or 1 to 680 of SEQ ID NO: 1. For example, a FGFR3 polypeptide fragment may include any polypeptide having at least 80% sequence identity to a fragment of SEQ ID NO: 1, e.g., at least amino acids 1 to 300, 1 to 310, 1 to 320, 1 to 330, 1 to 340, 1 to 350, 1 to 360, 1 to 370, 1 to 380, 1 to 390, 1 to 400, 1 to 410, 1 to 420, 1 to 430, 1 to 440, 1 to 440, 1 to 450, 1 to 460, 1 to 470, 1 to 480, 1 to 490, 1 to 500, 1 to 510, 1 to 520, 1 to 530, 1 to 540, 1 to 550, 1 to 560, 1 to 570, 1 to 580, 1 to 590, 1 to 600, 1 to 610, 1 to 620, 1 to 630, 1 to 640, 1 to 650, 1 to 660, 1 to 670, or 1 to 680 of SEQ ID NO: 1. For example, a FGFR3 polypeptide fragment may include any polypeptide having at least 85% sequence identity to a fragment of SEQ ID NO: 1, e.g., at least amino acids 1 to 300, 1 to 310, 1 to 320, 1 to 330, 1 to 340, 1 to 350, 1 to 360, 1 to 370, 1 to 380, 1 to 390, 1 to 400, 1 to 410, 1 to 420, 1 to 430, 1 to 440, 1 to 440, 1 to 450, 1 to 460, 1 to 470, 1 to 480, 1 to 490, 1 to 500, 1 to 510, 1 to 520, 1 to 530, 1 to 540, 1 to 550, 1 to 560, 1 to 570, 1 to 580, 1 to 590, 1 to 600, 1 to 610, 1 to 620, 1 to 630, 1 to 640, 1 to 650, 1 to 660, 1 to 670, or 1 to 680 of SEQ ID NO: 1. For example, a FGFR3 polypeptide fragment may include any polypeptide having at least 90% sequence identity to a fragment of SEQ ID NO: 1, e.g., at least amino acids 1 to 300, 1 to 310, 1 to 320, 1 to 330, 1 to 340, 1 to 350, 1 to 360, 1 to 370, 1 to 380, 1 to 390, 1 to 400, 1 to 410, 1 to 420, 1 to 430, 1 to 440, 1 to 440, 1 to 450, 1 to 460, 1 to 470, 1 to 480, 1 to 490, 1 to 500, 1 to 510, 1 to 520, 1 to 530, 1 to 540, 1 to 550, 1 to 560, 1 to 570, 1 to 580, 1 to 590, 1 to 600, 1 to 610, 1 to 620, 1 to 630, 1 to 640, 1 to 650, 1 to 660, 1 to 670, or 1 to 680 of SEQ ID NO: 1. For example, a FGFR3 polypeptide fragment may include any polypeptide having at least 95% sequence identity to a fragment of SEQ ID NO: 1, e.g., at least amino acids 1 to 300, 1 to 310, 1 to 320, 1 to 330, 1 to 340, 1 to 350, 1 to 360, 1 to 370, 1 to 380, 1 to 390, 1 to 400, 1 to 410, 1 to 420, 1 to 430, 1 to 440, 1 to 440, 1 to 450, 1 to 460, 1 to 470, 1 to 480, 1 to 490, 1 to 500, 1 to 510, 1 to 520, 1 to 530, 1 to 540, 1 to 550, 1 to 560, 1 to 570, 1 to 580, 1 to 590, 1 to 600, 1 to 610, 1 to 620, 1 to 630, 1 to 640, 1 to 650, 1 to 660, 1 to 670, or 1 to 680 of SEQ ID NO: 1. For example, a FGFR3 polypeptide fragment can include, e.g., amino acids 1 to 323 of SEQ ID NO: 1 (sFGFR3_Del4), amino acids 1 to 440 of SEQ ID NO: 1 (sFGFR3_Del3), or amino acids 1 to 548 of SEQ ID NO: 1 (sFGFR3_Del2). In an embodiment, a sFGFR3 polypeptide fragment consisting of amino acids 1 to 694 (sFGFR3_Del1) of SEQ ID NO: 1 is excluded as a soluble FGFR3 polypeptide within the scope of the invention.

The term "fusion protein" or "fusion polypeptide" relates to a protein comprising two or more polypeptides, preferably functional domains, derived from different proteins. The two or more polypeptides are linked directly or indirectly by peptide bonds. A fusion protein is generated by joining two or more nucleic acid sequences. This can be done recombinantly and also via nucleic acid synthesis. Translation of this fusion construct results in a single protein with the functional properties derived from the two or more polypeptides.

As used herein, the term "idiopathic skeletal growth retardation disorder" refers to a skeletal disease the cause of which is unknown and for which treatment with exogenous growth hormone (GH), e.g. recombinant human GH (rhGH), is ineffective.

The term "monitoring" as used herein refers to the accompaniment of a diagnosed skeletal growth retardation disorder during a treatment procedure or during a certain period of time, typically during at least 1 week, 2 weeks, 3 weeks, 4 weeks, 1 month, 2 months, 3 months, 4 months, 5 months, 6 months, 1 year, 2 years, 3 years, 5 years, 10 years 15 years, or any other period of time. The term "accompaniment" means that states of and, in particular, changes of the state of the skeletal growth retardation disorder may be detected based on the body weight and/or skull length and/or width of the subject, particular based on changes therein in any type of periodical time segment, determined e.g., 1, 2, 3, 4 or more times per month or per year, or approximately every 1, 2, 3, 4, 5, 6, 7, 8, 12 or 16 weeks, over the course of the treatment. Body weight and/or skull size of the subject or changes thereof can also be determined at treatment specific events, e.g. before and/or after every treatment or drug/therapy administration.

The term "nucleic acid" or "nucleic acid molecule" is to be understood as a single or doublestranded oligo- or polymer of deoxyribonucleotide or ribonucleotide bases or both. Typically, a nucleic acid is formed through phosphodiester bonds between the individual nucleotide monomers. In the context of the present invention, the term nucleic acid includes but is not limited to ribonucleic acid (RNA) and deoxyribonucleic acid (DNA) molecules. The depiction of a single strand of a nucleic acid also defines (at least partially) the sequence of the complementary strand. The nucleic acid may be single or double stranded, or may contain portions of both double and single stranded sequences. The nucleic acid may be obtained by biological, biochemical or chemical synthesis methods or any of the methods known in the art. As used herein, the term "nucleic acid" comprises the terms "polynucleotide" and "oligonucleotide". In the context of the present invention, the term nucleic acid comprises in particular cDNA, recombinant DNA, and mRNA. The nucleic acid can also be an artificial nucleic acid. Artificial nucleic acids include polyamide or peptide nucleic acid (PNA), morpholino and locked nucleic acid (LNA), as well as glycol nucleic acid (GNA) and threose nucleic acid (TNA). Each of these is distinguished from naturally-occurring DNA or RNA by changes to the backbone of the molecule.

A "peptide linker", "linker" or "splicer" in the context of the present invention refers to a peptide chain of between 1 and 100 amino acids. In preferred embodiments, a peptide linker according to the present invention has a minimum length of at least 1, 2, 3, 4, 5, 6, 7, 8, 9, or 10 amino acids. In further preferred embodiments, a peptide linker according to the present invention has a maximum length of 100, 95, 90, 85, 80, 75, 70, 65, 60, 55, 50, 45, 40, 35, 34, 33, 32, 31, 30, 29, 28, 27, 26, 25, 24, 23, 22, 21, or 20 amino acids. Preferred ranges are 5 to 25 and 10 to 20 amino acids in length. It is preferred that the peptide linker is a flexible peptide linker, i.e. provides flexibility among the two fusion protein members that are linked together. Such flexibility is generally increased if the amino acids are small and do not have bulky side chains that impede rotation or bending of the amino acid chain. Thus, preferably the peptide linker of the present invention has an increased content of small amino acids, in particular of glycins, alanines, serines, threonines, leucines and isoleucines. Preferably, at least 20%, 30%, 40%, 50%, 60% 70%, 80, 90% or more of the amino acids of the peptide linker are small amino acids.

For example, the amino acids of the linker are selected from glycines and serines, i.e., said linker is a poly-glycine or a poly-glycine/serine linker, wherein "poly" means a proportion of at least 50%, 60% 70%, 80, 90% or even 100% glycine and/or serine residues in the linker. In especially preferred embodiments, the above-indicated preferred minimum and maximum lengths of the peptide linker according to the present invention may be combined. In further preferred embodiments, the peptide linker of the present invention is non-immunogenic; in particularly preferred embodiments, the peptide linker is non-immunogenic in humans. For example, the sFGFR3 fusion polypeptide described herein (e.g., SEQ ID NO: 4 with or without amino acid residues 1 to 8 of SEQ ID NO: 4 or SEQ ID NO: 33) can include a glycine and serine linker, such as the amino acid sequence SGSGSGSGSGSGSGS.

"Pharmaceutically" or "pharmaceutically acceptable" refers to molecular entities and compositions that do not produce an adverse, allergic or other untoward reaction when administered to a mammal, especially a human, as appropriate. A pharmaceutically acceptable carrier or excipient refers to a non-toxic solid, semi-solid or liquid filler, diluent, encapsulating material or formulation auxiliary of any type. The form of the pharmaceutical compositions, the route of administration, the dosage and the regimen naturally depend upon the condition to be treated, the severity of the illness, the age, weight, and sex of the subject, etc. The pharmaceutical compositions of the invention can be formulated for a topical, oral, intranasal, intraocular, intravenous, intramuscular or subcutaneous administration and the like.

The term "polypeptide" as used herein refers to any peptide-linked chain of amino acids, regardless of length or post-translational modification. A polypeptide usable in the present invention can be further modified by chemical modification. This means that a chemically modified polypeptide comprises other chemical groups than the naturally occurring amino acids. Examples of such other chemical groups include, without limitation, glycosylated amino acids and phosphorylated amino acids. Chemical modifications of a polypeptide may provide advantageous properties as compared to the parent polypeptide, e.g., one or more of enhanced stability, increased biological half-life, or increased water solubility. Chemical modifications applicable to a polypeptide of the invention include without limitation: PEGylation, glycosylation of non-glycosylated polypeptides, or the modification of the glycosylation pattern present in the polypeptide. Further exemplary modifications include: replacement(s) of an amino acid with a modified and/or unusual amino acid, e.g. a replacement of an amino acid with an unusual amino acid like Nle, Nva or Orn; modifications to the N-terminal and/or C-terminal ends of the peptides, such as, e.g., N-terminal acylation (preferably acetylation) or desamination, or modification of the C-terminal carboxyl group into an amide or an alcohol group; modifications at the amide bond between two amino acids: acylation (preferably acetylation) or alkylation (preferably methylation) at the nitrogen atom or the alpha carbon of the amide bond linking two amino acids; modifications at the alpha carbon of the amide bond linking two amino acids ,such as, e.g., acylation (preferably acetylation) or alkylation (preferably methylation) at the alpha carbon of the amide bond linking two amino acids; chirality changes, such as, e.g., replacement of one or more naturally occurring amino acids (L enantiomer) with the corresponding D-enantiomers; retro-inversions in which one or more naturally-occurring amino acids (L-enantiomer) are replaced with the corresponding D-enantiomers, together with an inversion of the amino acid chain (from the C-terminal end to the N-terminal end); azapeptides, in which one or more alpha carbons are replaced with nitrogen atoms; and/or betapeptides, in which the amino group of one or more amino acid is bonded to the β carbon rather than the α carbon.

As used herein, "prevent", "preventing" or "prevention" of a disease or disorder means preventing that such disease or disorder occurs in subject.

The term "prokaryotic cell" as used herein refers to any kind of bacterial organism suitable for application in recombinant DNA technology such as cloning or protein expression includes both Gram-negative and Gram-positive microorganisms. Suitable bacteria may be selected from e.g. *Escherichia* (in particular *E. coli*), *Anabaena, Caulobacter, Gluconobacter, Rhodobacter, Pseudomonas, Paracoccus, Bacillus, Brevibacterium, Corynebacterium, Rhizobium (Sinorhizobium), Flavobacterium, Klebsiella, Enterobacter, Lactobacillus, Lactococcus, Methylobacterium, Propionibacterium, Staphylococcus* or *Streptomyces.*

The term "skeletal growth retardation disorder", as used herein, refers to a skeletal disease characterize by deformities and/or malformations of the bones. These disorders include, but are not limiting to, skeletal growth retardation disorders caused by growth plate (physeal) fractures, idiopathic skeletal growth retardation disorders and FGFR3-related skeletal diseases. For example, the skeletal growth retardation disorder may include a skeletal dysplasia, e.g., achondroplasia, homozygous achondroplasia, heterozygous achondroplasia, achondrogenesis, acrodysostosis, acromesomelic dysplasia, atelosteogenesis, camptomelic dysplasia, chondrodysplasia punctata, rhizomelic type of chondrodysplasia punctata, cleidocranial dysostosis, congenital short femur, craniosynostosis (e.g., Muenke syndrome, Crouzon syndrome, Apert syndrome, Jackson-Weiss syndrome, Pfeiffer syndrome, or Crouzonodermoskeletal syndrome), dactyly, brachydactyly, camptodactyly, polydactyly, syndactyly, diastrophic dysplasia, dwarfism, dyssegmental dysplasia, enchondromatosis, fibrochondrogenesis, fibrous dysplasia, hereditary multiple exostoses, hypochondroplasia, hypophosphatasia, hypophosphatemic rickets, Jaffe-Lichtenstein syndrome, Kniest dysplasia, Kniest syndrome, Langer-type mesomelic dysplasia, Marfan syndrome, McCune-Albright syndrome, micromelia, metaphyseal dysplasia, Jansen-type metaphyseal dysplasia, metatrophic dysplasia, Morquio syndrome, Nievergelt-type mesomelic dysplasia, neurofibromatosis (e.g., type 1, e.g., with bone manifestations or without bone manifestations; type 2; schwannomatosis; or any described herein), osteoarthritis, osteochondrodysplasia, osteogenesis imperfecta, perinatal lethal type of osteogenesis imperfecta, osteopetrosis, osteopoikilosis, peripheral dysostosis, Reinhardt syndrome, Roberts syndrome, Robinow syndrome, short-rib polydactyly syndromes, short stature, spondyloepiphyseal dysplasia congenita, spondyloepimetaphyseal dysplasia, or thanatophoric dysplasia. Accordingly, the invention provides for the treatment or prevention of symptoms of a skeletal growth retardation disorder, e.g., achondroplasia.

The term "soluble" as used herein indicates that the protein or polypeptide, in particular a receptor, is not directly bound to a cellular membrane, and is, accordingly, characterized by the absence or functional disruption of all or a substantial part of the transmembrane (i.e., lipophilic) domain, so that the soluble protein or polypeptide is devoid of any membrane anchoring function. The cytoplasmic domains may also be absent. In this respect, the transmembrane domain of FGFR3 extends from amino acid 367-399 of the wild-type sequence according to SEQ ID NO: 6 and the cytoplasmic domain of FGFR3 extends from amino acid 400-806 of the wild-type sequence according to SEQ ID NO: 6. The extracellular domain (ECD) of FGFR3 extends from amino acids 1 to 366 of the wild-type FGFR3 sequence according to SEQ ID NO: 6 or fragments thereof.

Exemplary sFGFR3 polypeptides can include, but are not limited to, at least amino acids 1 to 300, 1 to 310, 1 to 320, 1 to 330, 1 to 340, 1 to 350, 1 to 360, 1 to 370, 1 to 380, 1 to 390, 1 to 400, 1 to 410, 1 to 420, 1 to 430, 1 to 440, 1 to 440, 1 to 450, 1 to 460, 1 to 470, 1 to 480, 1 to 490, 1 to 500, 1 to 510, 1 to 520, 1 to 530, 1 to 540, 1 to 550, 1 to 560, 1 to 570, 1 to 580, 1 to 590, 1 to 600, 1 to 610, 1 to 620, 1 to 630, 1 to 640, 1 to 650, 1 to 660, 1 to 670, 1 to 680, or 1 to 690 of SEQ ID NO: 1. Additionally, sFGFR3 polypeptides can include any polypeptide having at least 50% (e.g., 55%, 60%, 65%, 70%, 75%, 80%, 85%, 86%, 87%, 88%, 89%, 90%, 91%, 92%, 93%, 94%, 95%, 96%, 97%, 98%, 99%, or more) sequence identity to any of these sFGFR3 polypeptide fragments. For example, sFGFR3 polypeptides can include fragments of sFGFR3_Del1 (SEQ ID NO: 1). sFGFR3_Del1 (SEQ ID NO: 1) features a deletion of amino acids 311 to 422 of SEQ ID NO: 6. In particular, sFGFR3 polypeptides may include, e.g., amino acids 1 to 323 of SEQ ID NO: 1 (sFGFR3_Del4), amino acids 1 to 440 of SEQ ID NO: 1 (sFGFR3_Del3), or amino acids 1 to 548 of SEQ ID NO: 1 (sFGFR3_Del2). sFGFR3 polypeptides may also include a deletion of the N-terminus of the sFGFR3 polypeptide (e.g., amino acid residue 1 to 56 of SEQ ID NO: 1).

The term "soluble FGFR3 polypeptide" or "sFGFR3 polypeptide" as used herein refers to a FGFR3 polypeptide that is characterized by the absence or functional disruption of all or a substantial part of the transmembrane (i.e., lipophilic) domain and also does not contain any polypeptide portion that would anchor the FGFR3 polypeptide to a cell membrane. A sFGFR3 polypeptide is thus a non-membrane bound form of a FGFR3 polypeptide. In particular, the transmembrane domain of FGFR3 extends from amino acid residues 367 to 399 of the wild-type FGFR3 sequence (SEQ ID NO: 6). Thus the sFGFR3 polypeptide includes a deletion of amino acid residues 367 to 399 of the wild-type FGFR3 polypeptide sequence (SEQ ID NO: 6). The sFGFR3 polypeptide can further include deletions of the cytoplasmic domain of the wild-type FGFR3 polypeptide sequence (amino acid residues 400-806 of SEQ ID NO: 6). Additionally, the sFGFR3 can include deletions of the extracellular domain of the wild-type FGFR3 polypeptide sequence (amino acid residues 1 to 366 of SEQ ID NO: 6).

The term "specifically binds" as used herein refers to a binding reaction which is determinative of the presence of the herein defined binding partner (e.g., FGF or aggrecan) in a heterogeneous population of proteins and, in particular, cells, such as in an organism, preferably a human body. As such, the specified ligand binds to its defined binding partner and does not bind in a substantial amount to other molecules present in an organism, preferably human organism, and more preferably in extracellular fluids (e.g. interstitial fluid and plasma) or in/on the extracellular matrix, in particular the cartilage. Generally, a ligand that "specifically binds" a target molecule has an equilibrium dissociation constant K_{D} is less than about 10⁻⁵ (e.g., 10⁻⁶, 10⁻⁷, 10⁻⁸, 10⁻⁹, 10⁻¹⁰, 10⁻¹¹, and 10⁻¹² or less) for that target molecule, preferably at room temperature (e.g., 20°C to 25°C). Methods of how to measure equilibrium dissociation constants are well known in the art. A preferred method uses a BiaCore® system. In particular with respect to a fragment and/or variant of a protein (such as an aggrecan-binding protein or FGFR3) that specifically binds another protein, specifically binds means that it has at least 10, 20, 30, 40, 50, 60, 70, 80, or 90% of the binding activity of the protein it is derived from (i.e., of the aggrecan-binding protein or FGFR3, respectively). For a suitable binding assay, see Example 1 (FGF/Aggrecan binding) and Example 4. Other proteins or fragments or variants thereof can be tested accordingly.

As used herein, the term "subject" or "patient" denotes a human or non-human mammal, such as a rodent, a feline, a canine, an equine, or a primate. Preferably, the subject is a human being, more preferably a child (i.e. in terms of the present invention a human that is still growing, particularly in height). It is noted that the cartilaginous matrix of the growth plate is less dense in a newborn or in a child than in an adult. Therefore, without wishing to be bound by theory, one can expect that the polypeptides of the invention will better penetrate said cartilaginous matrix in a newborn or a child. In one embodiment, the subject has been diagnosed as suffering from a skeletal growth retardation disorder as described herein, in particular an FGFR3-related skeletal disease.

As used herein, "treat", "treating", or "treatment" of a disease or disorder means accomplishing one or more of the following: (a) reducing the severity of the disorder; (b) limiting or preventing development of symptoms characteristic of the disorder(s) being treated; (c) inhibiting worsening of symptoms characteristic of the disorder(s) being treated; (d) limiting or preventing recurrence of the disorder(s) in an individual that have previously had the disorder(s); and (e) limiting or preventing recurrence of symptoms in individuals that were previously symptomatic for the disorder(s).

The above preferably applies to one or more symptoms of achondroplasia, which include: short stature, a long trunk, and shortened limbs, which are noticeable at birth; an adult height of between 42-56 inches; a head that is large and a forehead that is prominent; portions of the face can be underdeveloped; at birth, the legs appear straight, but as a child begins to walk, he or she develops a knock-knee or bowed-leg deformity; the hands and the feet appear large, but the fingers and toes are short and stubby; straightening of the arm at the elbow may be limited, but usually does not keep a patient from doing any specific activities; children may develop an excessive curve of the lower back and a waddling walking pattern; dental problems, e.g. from overcrowding of teeth; weight control problems; neurologic and respiratory problems; and/or fatigue, pain, and numbness in the: Lower back and/or spine.

The term "variant" is, with respect to polypeptides, to be understood as a polypeptide which differs in comparison to the polypeptide from which it is derived by one or more changes in the amino acid sequence. The polypeptide from which a protein variant is derived is also known as the parent polypeptide. Typically, a variant is constructed artificially, preferably by gene-technological means. Typically, the parent polypeptide is a wild-type protein or wild-type protein domain. The variants usable in the present invention may also be derived from homologs, orthologs, or paralogs of the parent polypeptide. The changes in the amino acid sequence may be amino acid exchanges, insertions, deletions, N-terminal truncations, or C-terminal truncations, or any combination of these changes, which may occur at one or several sites. In preferred embodiments, a variant usable in the invention exhibits a total number of up to 200 (up to 1, 2, 3, 4, 5, 6, 7, 8, 9, 10, 15, 20, 25, 30, 35, 40, 45, 50, 55, 60, 65, 70, 75, 80, 85, 90, 95, 100, 110, 120, 130, 140, 150, 160, 170, 180, 190, or 200) changes in the amino acid sequence (i.e. exchanges, insertions, deletions, N-terminal truncations, and/or C-terminal truncations). The amino acid exchanges may be conservative and/or non-conservative. In preferred embodiments, a variant usable in the present invention differs from the protein or domain from which it is derived by up to 1, 2, 3, 4, 5, 6, 7, 8, 9, 10, 15, 20, 25, 30, 35, 40, 45, 50, 55, 60, 65, 70, 75, 80, 85, 90, 95, or 100 amino acid exchanges, preferably conservative amino acid changes. Alternatively or additionally, a "variant" as used herein can be characterized by a certain degree of sequence identity to the parent polypeptide from which it is derived. More precisely, a protein variant in the context of the invention exhibits at least 80% sequence identity to its parent polypeptide. Preferably, the sequence identity of protein variants is over a continuous stretch of 20, 30, 40, 45, 50, 60, 70, 80, 90, 100, 150, 200, 250, 300, 400, 500, 600 or more amino acids, more preferably over the entire length of the reference polypeptide (the parent polypeptide). The term "at least 80% sequence identity" is used throughout the specification with regard to polypeptide sequence comparisons. This expression preferably refers to a sequence identity of at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% to the respective reference polypeptide. Preferably, the polypeptide in question and the reference polypeptide exhibit the indicated sequence identity over a continuous stretch as specified above.

The sequence identity of a "variant" polypeptide of the invention can be determined over an identified range of an amino acid sequence of a sFGF decoy polypeptide of the invention (e.g., amino acids 1 to 310 of SEQ ID NO: 1) or with reference to the entire amino acid sequence of an identified sFGF decoy polypeptide (e.g., all of the amino acids of SEQ ID NO: 1). When determining the percent sequence identity for a variant polypeptide of the invention, the sequence alignment may omit any specifically excluded amino acid residues (e.g., amino acids 367 to 399 of SEQ ID NO: 6 or amino acids 324 to 694 of SEQ ID NO: 1). For example, for a variant polypeptide of a sFGF decoy polypeptide of the invention that exhibits at least 80% sequence identity to amino acids 1 to 310 of SEQ ID NO: 1, the sequence alignment for comparison may occur across only amino acids 1 to 310 or may take into account two or more identified ranges of amino acid sequences within the full length sFGF decoy polypeptide.

The term "variant" is, with respect to nucleic acids, to be understood as a polynucleotide which differs in comparison to the nucleic acid from which it is derived by one or more changes in the nucleotide sequence. The nucleic acid from which variant is derived is also known as the parent nucleic acid. Typically, a variant is constructed artificially, preferably by gene-technological means. Typically, the parent nucleic acid is a wild-type nucleic acid or part thereof. The variants usable in the present invention may also be derived from homologs, orthologs, or paralogs of the parent nucleic acid. The changes in the nucleotide sequence may be exchanges, insertions, deletions, 5' truncations, or 3' truncations, or any combination of these changes, which may occur at one or several sites. In preferred embodiments, a variant usable in the present invention exhibits a total number of up to 600 (up to 1, 2, 3, 4, 5, 6, 7, 8, 9, 10, 15, 20, 25, 30, 35, 40, 45, 50, 55, 60, 65, 70, 75, 80, 85, 90, 95, 100, 110, 120, 130, 140, 150, 160, 170, 180, 190, 200, 300, 400, 500 or 600) changes in the nucleotide sequence. The nucleotide exchanges may be lead to non-conservative and/or preferably conservative amino acid exchanges as set out above with respect to polypeptide variants. Alternatively or additionally, a "variant" as used herein can be characterized by a certain degree of sequence identity to the parent nucleic acid from which it is derived. More precisely, a nucleic acid variant in the context of the present invention exhibits at least 80% sequence identity to its parent nucleic acid. Preferably, the sequence identity of nucleic acid variants is over a continuous stretch of 20, 30, 40, 45, 50, 60, 70, 80, 90, 100, 150, 200, 250, 300, 400, 500, 600 or more amino acids, more preferably over the entire length of the reference nucleic acid (the parent nucleic acid). The term "at least 80% sequence identity" is used throughout the specification also with regard to nucleic acid sequence comparisons. This term preferably refers to a sequence identity of at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% to the respective reference nucleic acid or to the respective reference nucleic acid. Preferably, the nucleic acid in question and the reference nucleic acid or exhibit the indicated sequence identity over a continuous stretch as specified above.

As used herein, the term "vector" refers to a protein or a polynucleotide or a mixture thereof which is capable of being introduced or of introducing the nucleic acid comprised therein into a cell. In the context of the present invention it is preferred that the nucleic acid of the third aspect is expressed within the cell upon introduction of the vector. Suitable vectors are known in the art and include, for example, plasmids, cosmids, artificial chromosomes (e.g. bacterial, yeast or human), bacteriophages, viral vectors (e.g. retroviruses, lentiviruses, adenoviruses, adeno-associated viruses or baculoviruses), or nano-engineered substances (e.g. ormosils). Required vector technologies are well known in the art (see e.g. Lodish et al., Molecular Cell Biology, W. H. Freeman; 6th edition, June 15, 2007; or Green and Sambrook, Molecular Cloning - A Laboratory Manual, 2012 Cold Spring Harbor Laboratory Press).

### BRIEF DESCRIPTION OF THE SEVERAL VIEWS OF THE DRAWINGS

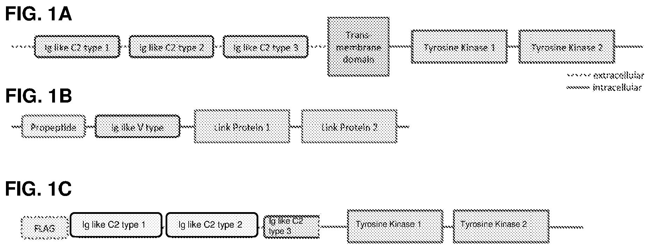

**Figures 1A-1C** are a series of diagrams showing the protein structure and domains of (A) wild-type human fibroblast growth factor receptor 3 (FGFR3) (∼87 kDa), (B) wild-type human hyaluronan and proteoglycan link protein 1 (HPLN1) (∼40 kDa), and (C) a soluble FGFR3 (sFGFR3, ∼72 kDa) developed previously by the inventor (see PCT/EP2014/050800; SEQ ID NO: 1). The sFGFR3 is shown with a FLAG tag at the N-terminus of the sFGFR3 polypeptide.

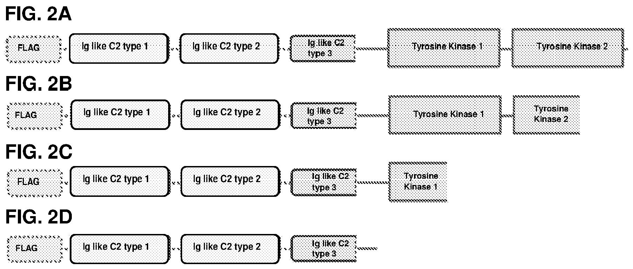

**Figures 2A-2D** are a series of diagrams showing the structure of exemplary FGF decoy polypeptide fragments (sFGFR3 deletion variants) (A) sFGFR3_Del1 (∼70 kDa), (B) sFGFR3_Del2 (-62 kDa), (C) sFGFR3_Del3 (-49 kDa), and (D) sFGFR3_Del4 (-37 kDa). Each exemplary sFGFR3 fragment (sFGFR3 deletion variants) is shown with an optional FLAG tag at the N-terminus of the sFGFR3 fragment.

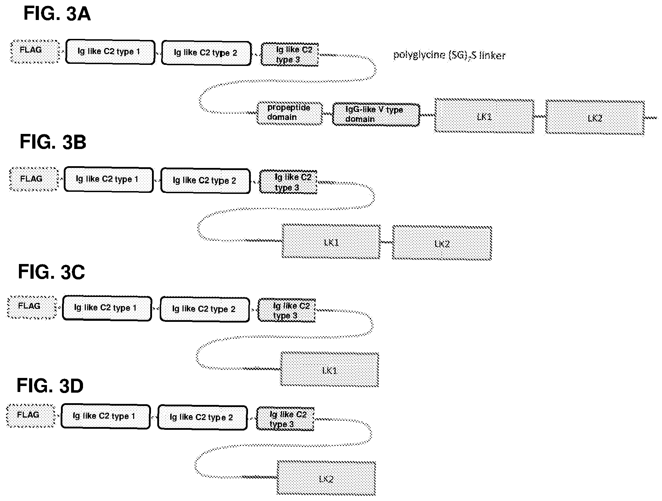

**Figures 3A-3D** are a series of diagrams showing the structure of exemplary fusion proteins containing a sFGFR3_Del4 and HPLN1 or fragments thereof: (A) FLAG-sFGFR3_Del4-HPLN1 (∼79 kDa), (B) FLAG-sFGFR3_Del4-LK1-LK2 (∼62 kDa; SEQ ID NO: 4 with or without amino acid residues 1 to 8 of SEQ ID NO: 4 or SEQ ID NO: 33), (C) FLAG-sFGFR3_Del4-LK1 (-50 kDa), and (D) FLAG-sFGFR3_Del4-LK2 (∼50 kDa). Each exemplary sFGFR3 fusion protein is shown with an optional FLAG tag at the N-terminus of the sFGFR3 fusion protein.

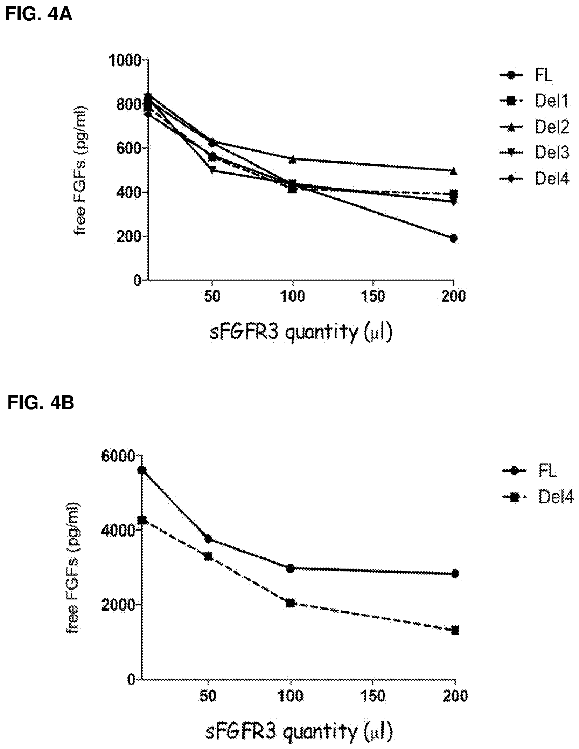

**Figures 4A-4B** are graphs showing FGF binding using the deletion (Del) variants and a comparison with a FLAG-sFGFR3 full-length protein. (A) The different recombinant proteins were incubated with a fixed amount of human FGF2. (B) Fixation of human FGF9 was verified using the Del4 variant.

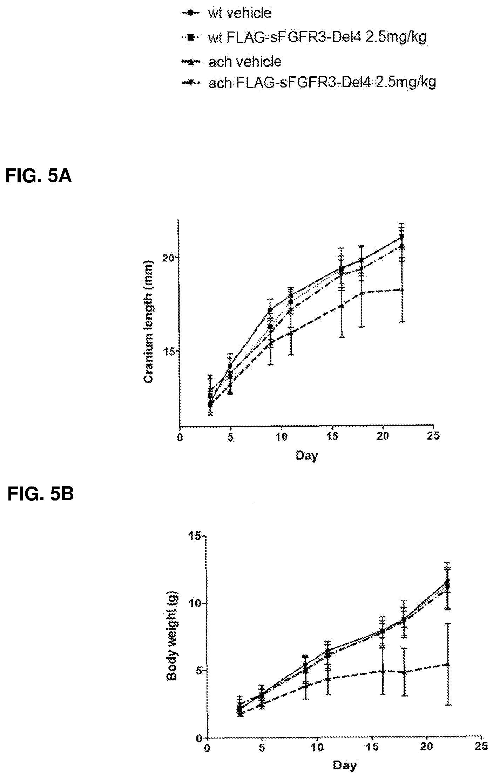

**Figures 5A-5B** are graphs showing an evaluation of the velocity of growth using cranium length (A) and body weight (B) following FLAG-sFGFR3_Del4 treatment.

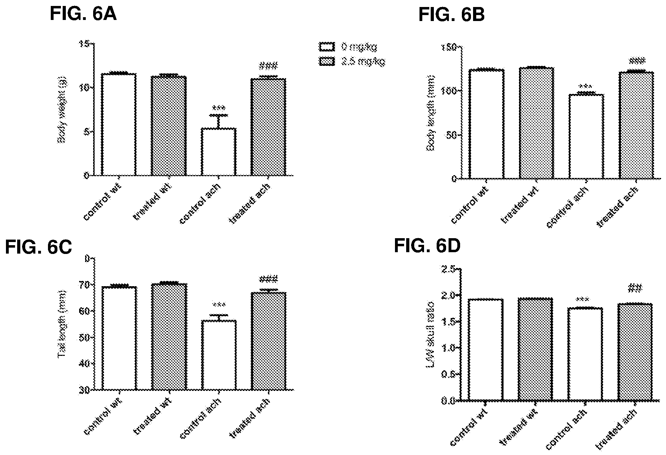

**Figures 6A-6D** are graphs showing an *in vivo* evaluation of FLAG-sFGFR3_Del4 treatment administered at the dose of 2.5 mg/kg (e.g., 2.5 mg/kg twice per week for 3 weeks). Treatment effect was evaluated by the measurement of body weight (A), body length (B), tail length (C), and the ratio skull length (L)/skull width (W) (D).

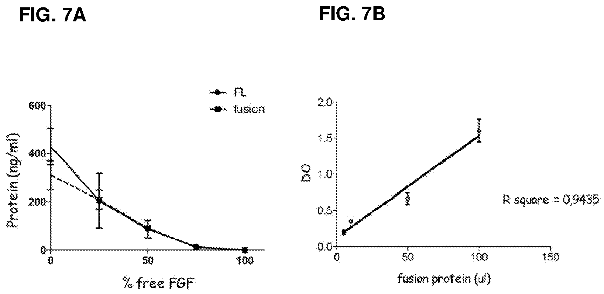

**Figures 7A-7B** are graphs showing FGF (A) and aggrecan (B) binding using FLAG-sFGFR3_Del4-LK1-LK2.

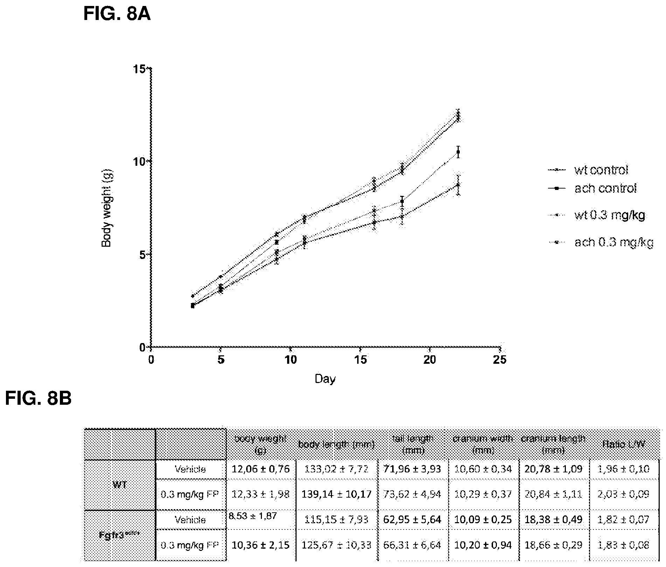

**Figures 8A-8B** are graphs showing an *in vivo* evaluation of the therapeutic efficacy of FLAG-sFGFR3_Del4-LK1-LK2. A. Velocity of growth was evaluated by body weight measurement during the treatment period. B. Skeletal parameters of WT and transgenic *Fgfr3*^{*achl*+} mice in the treatment or control groups.

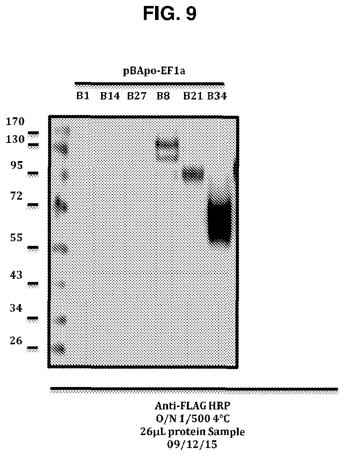

**Figure 9** is an image of a Western blot showing the role of the signal peptide (e.g., MGAPACALALCVAVAIVAGASS) in sFGFR3 polypeptides and sFGFR3 fusion polypeptides. Anti-FLAG monoclonal M2 antibody was used for detection of sFGFR3_Del1 (SEQ ID NO: 1) with the FLAG tag in the N-terminal position followed by the signal peptide sequence (lane B1), sFGFR3_Del4-LK1-LK2 (SEQ ID NO: 4) with the FLAG tag in the N-terminal position followed by the signal peptide sequence (lane B14), sFGFR3_Del4 with the FLAG tag in the N-terminal position followed by the signal peptide sequence (lane B27), sFGFR3_Del1 (SEQ ID NO: 1) with the FLAG tag in the C-terminal position (lane B8), sFGFR3_Del4-LK1-LK2 (SEQ ID NO: 4) with the FLAG tag in the C-terminal position (lane B21), and sFGFR3_Del4 with the FLAG tag in the C-terminal position (lane B34).

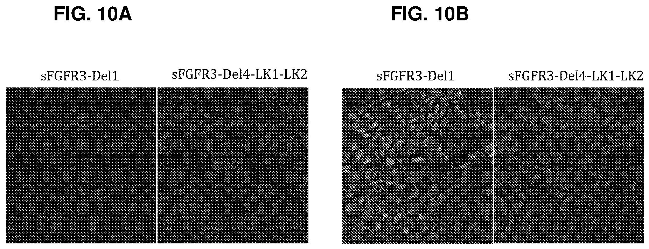

**Figures 10A-10B** are photomicrographs showing immunohistochemical staining of FLAG-sFGFR3 in the femoral growth plates of *Fgfr3*^{*ach*/*+*} mice treated with sFGFR3_Del1 (SEQ ID NO: 1) and sFGFR3_Del4-LK1-LK2 (SEQ ID NO: 4) at day 5 (Figure 10A) and at day 22 (Figure 10B) after birth.

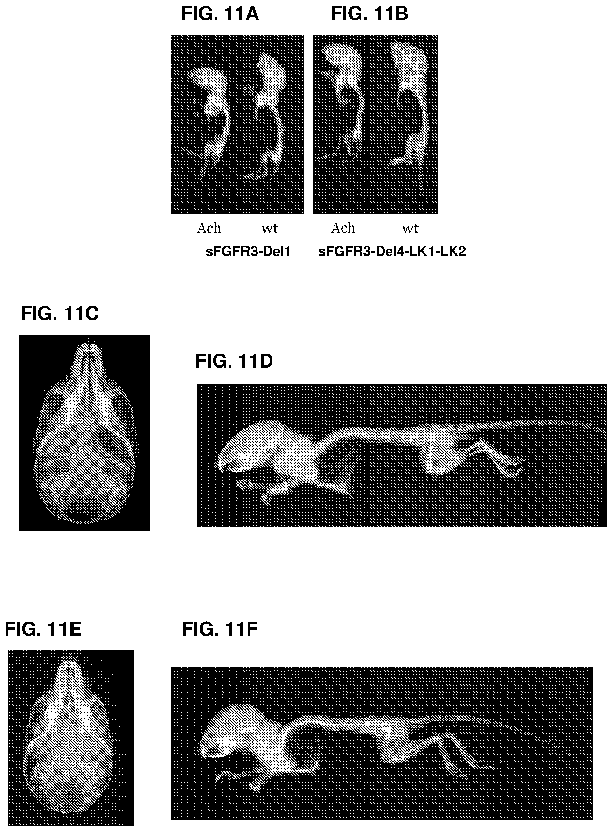

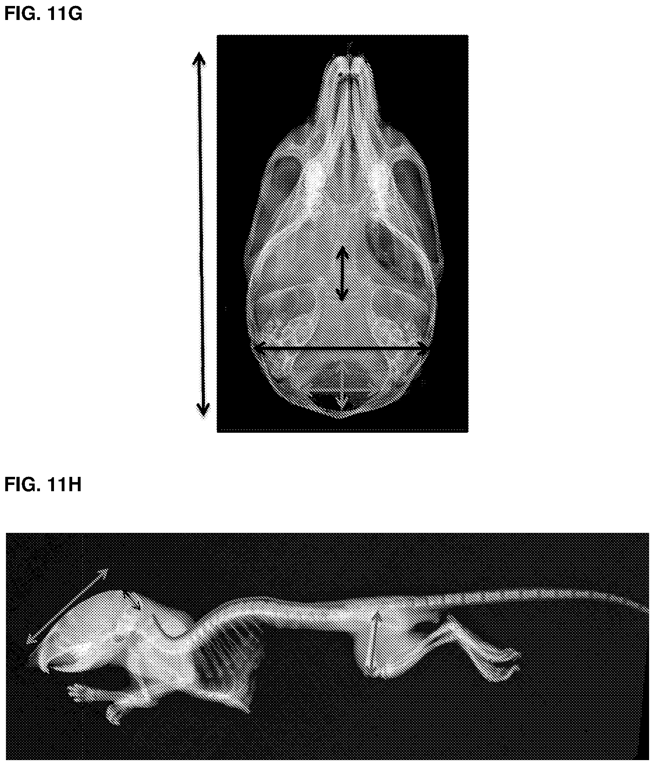

**Figures 11A-11H** are x-ray radiographs of wild-type mice and *Fgfr3*^{*ach*/+} mice after 5 days of treatment with sFGFR3_Del1 and sFGFR3_Del4-LK1-LK2 (Figures 11A-11B) or with no treatment (Figures 11C-11H).

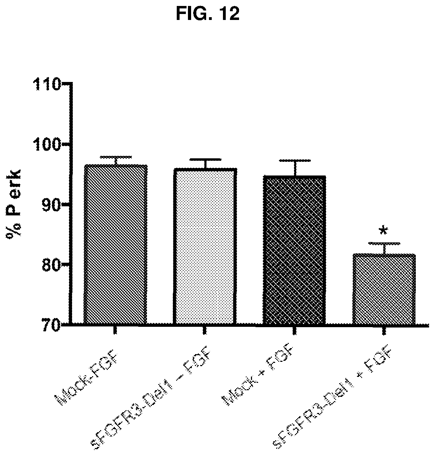

**Figure 12** is a graph showing phosphorylated ERK in ATDC5 chondrocyte cells after a 24 hour challenge with sFGFR3_Del1 (SEQ ID NO: 1) in the presence or absence of human FGF2 (100 pg/ml). Results are expressed as percentage of the condition with only hFGF2. A Mock supernatant was used as a control.

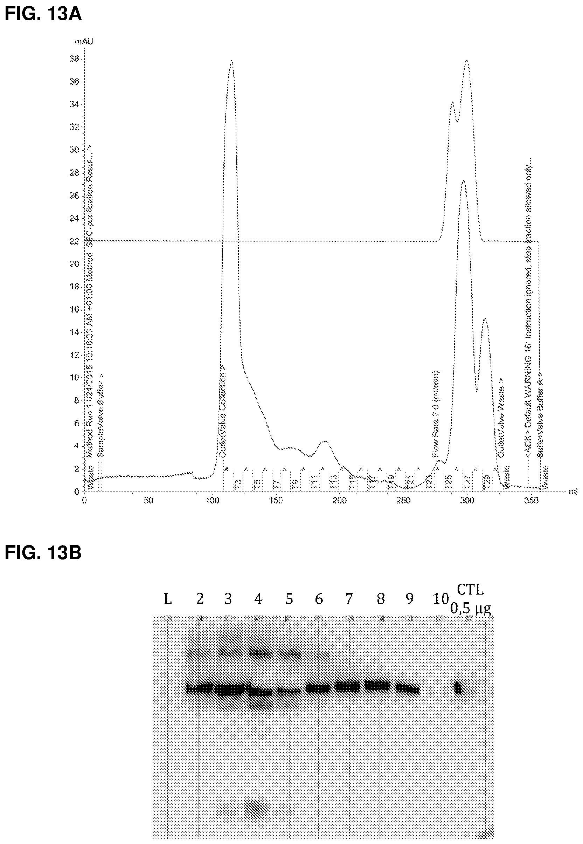

**Figures 13A-13C** are images and graphs showing the effect of different sFGFR3_Del1 fractions on cellular proliferation. Shown are a size exclusion chromatogram of sFGFR3_Del1 (Figure 12A); a western blot using anti-FLAG monoclonal M2 antibody of sFGFR3_Del1 (Figure 12B); and proliferation of ATDC5 chondrocyte cells in the presence of FGF and different fractions of sFGFR3_Del1 (Figure 12C).

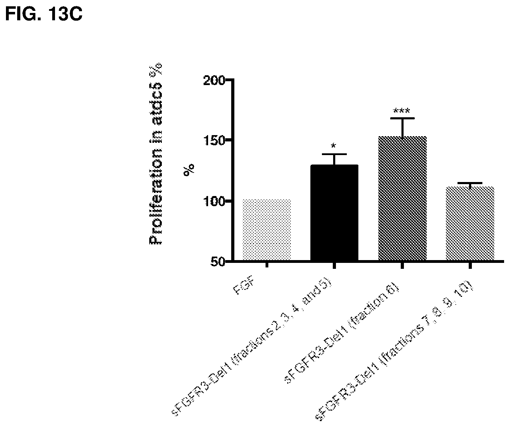

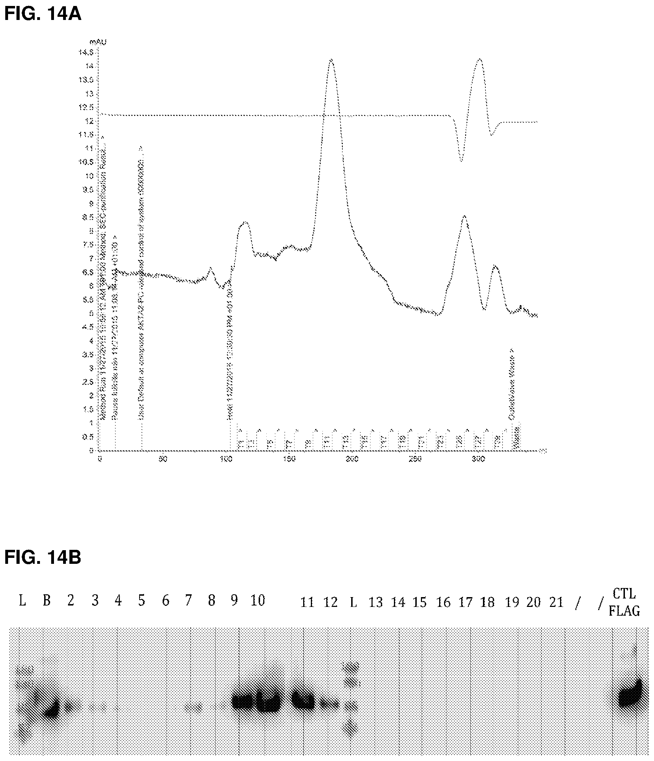

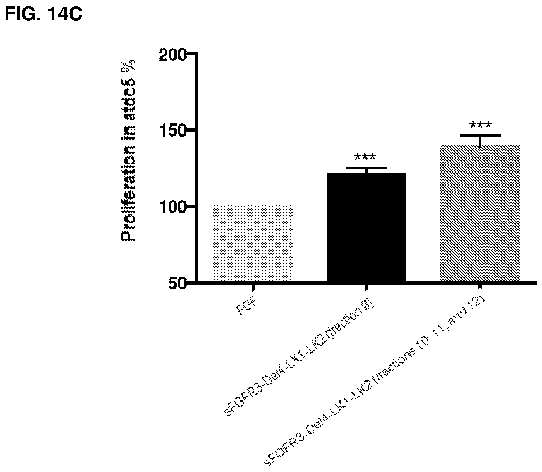

**Figures 14A-14C** are images and graphs showing the effect of different sFGFR3_Del4-LK1-LK2 fractions on chondrocyte proliferation. Shown are a size exclusion chromatogram of sFGFR3_Del4-LK1-LK2 (Figure 13A); a western blot using anti-FLAG monoclonal M2 antibody of sFGFR3_Del1 (Figure 13B); and proliferation of ATDC5 chondrocyte cells in the presence of FGF and different fractions of sFGFR3_Del1 (Figure 13C).

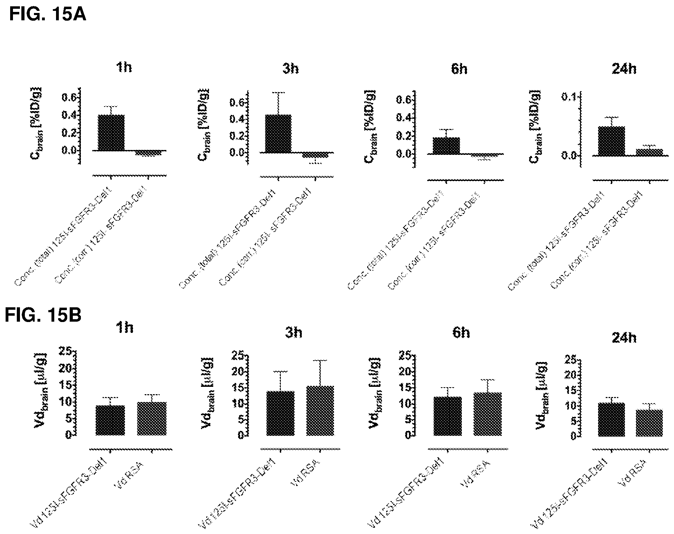

**Figures 15A-15E** are graphs showing brain tissue and plasma concentrations and stability of ¹²⁵I- FGFR3_Del1 in C57BL/6 mice after 1 h, 3 h, 6 h, or 24 h of administration.

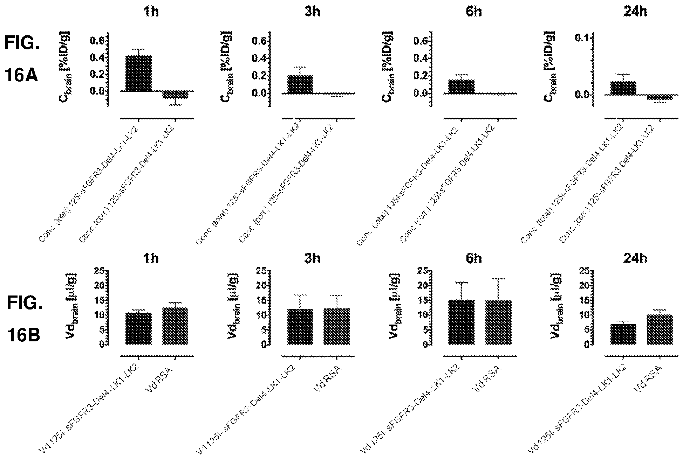

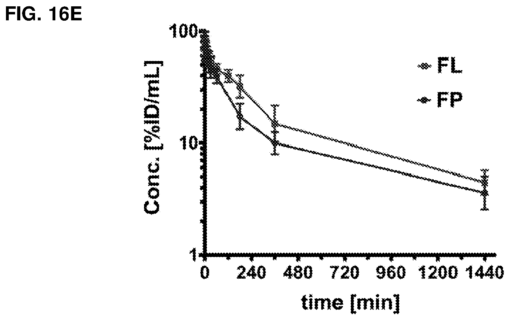

**Figures 16A-16E** are graphs showing brain tissue and plasma concentrations and stability of ¹²⁵I- sFGFR3_Del4-LK1-LK2 in C57BL/6 mice after 1 h, 3 h, 6 h, or 24 h of administration. A comparison of plasma concentrations of ¹²⁵I- FGFR3_Del1 and ¹²⁵I- sFGFR3_Del4-LK1-LK2 is also shown (Figure 16E).

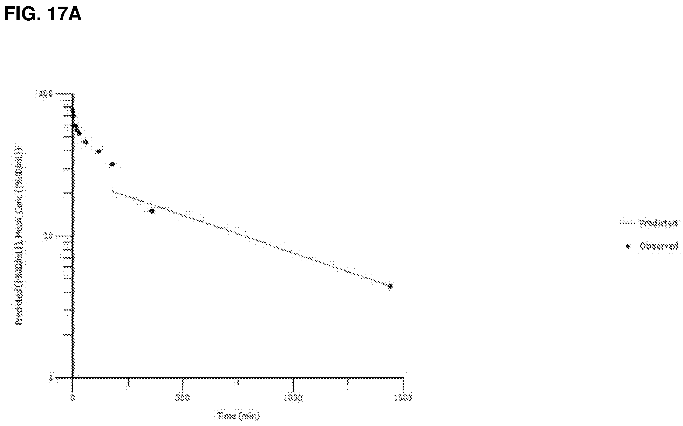

**Figures 17A-17B** are graphs showing a non-compartmental analysis (NCA) of ¹²⁵I-FGFR3_Del1 and ¹²⁵I- sFGFR3_Del4-LK1-LK2 plasma concentrations.

### DETAILED DESCRIPTION OF THE INVENTION

We have discovered that polypeptide variants of soluble FGFR3 fibroblast growth factor receptor 3 (sFGFR3) may be used to treat or prevent skeletal growth retardation disorders, such as achondroplasia, in a subject in need thereof (e.g., a human, particularly an infant or a child). The invention provides sFGFR3 polypeptide variants including a fragment of the extracellular domain (ECD). In particular, sFGFR3 polypeptides of the invention include sFGFR3 deletion (Del) variants featuring a deletion of, e.g., amino acids 311 to 422 of SEQ ID NO: 6, to provide the following exemplary Del variants: sFGFR3_Del2 (amino acids 1 to 548 of SEQ ID NO: 1), sFGFR3_Del3 (amino acids 1 to 440 of SEQ ID NO: 1), and sFGFR3_Del4 (amino acids 1 to 323 of SEQ ID NO: 1). The invention also features fusion polypeptides including a sFGFR3 polypeptide fragment, such as a sFGFR3 polypeptide fragment including, e.g., amino acids 1 to 323 of SEQ ID NO: 1, fused to a heterologous polypeptide, such as an aggrecan-binding protein including human hyaluronan and proteoglycan link protein 1 (HPLN1) or fragments thereof (e.g., sFGFR3_Del4-LK1-LK2 (SEQ ID NO: 4 with or without amino acid residues 1 to 8 of SEQ ID NO: 4 or SEQ ID NO: 4 with or without amino acid residues 1 to 8 of SEQ ID NO: 4 or SEQ ID NO: 33)). Methods for administering sFGFR3 polypeptides (e.g., Del variants as described herein) and fusion polypeptides (e.g., sFGFR3_Del4-LK1-LK2 (SEQ ID NO: 4 with or without amino acid residues 1 to 8 of SEQ ID NO: 4)) to treat or prevent skeletal growth retardation disorders (e.g., achondroplasia) in a subject in need thereof (e.g., a human, particularly an infant or a child) are also described.

We have determined that smaller functional portions of sFGFR3 exhibit a similar mechanism of action relative to larger soluble FGFR3 fragments and full-length FGFR3 polypeptides while maintaining the capability of dimerization following fixation of free FGFs. Several FGFR3 polypeptide variants with shorter intra-cellular domains were designed. All variants bound human FGF2 with similar affinity relative to sFGFR3_Del1 (SEQ ID NO: 1). Therapeutic benefit was shown using even the shortest FGFR3 variant, sFGFR3_Del4 (amino acids 1 to 323 of SEQ ID NO: 1), which restored bone growth in transgenic *Fgfr3*^{*ach*/+} mice. Therapeutic efficacy in the treatment of achondroplasia was also observed when sFGFR3_Del4 was administered in a mouse model of this disease, thereby demonstrating the use of this FGFR3 fragment in treatment of growth disorders and for designing additional constructions, such as fusion proteins. The sFGFR3_Del4 construct was also used to validate the use of body weight and skull length monitoring as indexes of velocity of growth. Furthermore, the invention provides fusion proteins comprising the smaller functional portions of sFGFR3 and human hyaluronan and proteoglycan link protein 1 (HPLN1). These fusion proteins bind FGF, as well as aggrecan, and exhibit improved therapeutic benefit relative to previous sFGFR3 decoys.

### Fibroblast Growth Factor Receptor 3

The present disclosure is not limited to a particular Fibroblast Growth Factor Receptor 3 (FGFR3) polypeptide or nucleic acid encoding a FGFR3. FGFR3 polypeptides encompass members of the fibroblast growth factor receptor (FGFR) family that mediate binding to fibroblast growth factors (FGFs) (e.g., FGF1, FGF2, FGF3, FGF4, FGF5, FGF6, FGF7, FGF8, FGF9, FGF10, or FGF18) and play a role in bone development and maintenance. In particular, a FGFR3 polypeptide can bind to FGF1, FGF2, FGF9 and/or FGF 18.

An FGFR3 polypeptide can include a polypeptide having the amino acid sequence of any one of the known FGFR3 polypeptides or a fragment thereof. FGFR3 polypeptides can include naturally occuring isoforms, such as FGFR3 produced by alternative splicing of the Ig3 domain of FGFR3, in which the C-terminal half of Ig3 is encoded by two separate exons, exon 8 (isoform 1; FGFR3 IIIb) and exon 9 (isoform 3; FGFR3 IIIc). In particular, a FGFR3 polypeptide can include the FGFR3 IIIc-type ECD with the C-terminal Ig3 half encoded by exon 9 (Accession No. NP_000133), which corresponds to FGFR3 transcript variant 1(Accession No. NM_000142.4). A FGFR3 polypeptide can also include the FGFR3 IIIb-type ECD with the C-terminal half encoded by exon 8 (Accession No. NP_001156685), which corresponds to FGFR3 transcript variant 3 (Accession No. NM_001163213).

FGFR3 polypeptides may include not only the FGFR3 amino acid sequences described above, but any polypeptide having at least 50% (e.g., 55%, 60%, 65%, 70%, 75%, 80%, 85%, 86%, 87%, 88%, 89%, 90%, 91%, 92%, 93%, 94%, 95%, 96%, 97%, 98%, 99%, or more) amino acid sequence identity to these FGFR3 polypeptides (e.g., SEQ ID NO: 6) or an amino acid fragment of these FGFR3 amino acid sequences (e.g., at least 50, 100, 150, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 550, 600, 650, 700, 750, or more amino acid residues of an FGFR3 amino acid sequence (e.g., SEQ ID NO: 6)). In addition to the exemplary FGFR3 polypeptides discussed above, this disclosure also provides any FGFR3 polypeptide comprising the identical or similar FGF binding affinity for treating skeletal growth retardation disorders (e.g., achondroplasia) in a patient, e.g., a human.

### Soluble FGFR3

sFGFR3 polypeptides of the invention include soluble (e.g., non-membrane-bound) forms of any of the FGFR3s described herein. The present disclosure is not limited to a particular sFGFR3 and may include any sFGFR3 polypeptide that binds one or more FGFs (e.g., FGF1 (SEQ ID NO: 7), FGF2 (SEQ ID NO: 8), FGF9 (SEQ ID NO: 9), and/or FGF 18 (SEQ ID NO: 10)), and accordingly, may be used as a decoy receptor for one or more FGFs to treat skeletal growth retardation disorders, e.g., achondroplasia. The invention further includes nucleic acids encoding the sFGFR3 polypeptides described herein that may be used to treat the conditions described herein, e.g., achondroplasia, in a subject in need thereof, such as SEQ ID NO: 5. The sFGFR3 polypeptide can be, for example, fragments of FGFR3 isoform 2 lacking exons 8 and 9 encoding the C-terminal half of the IgG3 domain and exon 10 including the transmembrane domain (i.e., sFGFR3_Del1; SEQ ID NO: 1 and Accession No. NP_075254), corresponding to FGFR3 transcript variant 2 (Accession No. NM_022965). Compositions including sFGFR3 are further described in PCT publication Nos: WO 2014/111744 and WO 2014/111467, which are each incorporated herein by reference in their entirety.

For example, sFGFR3 polypeptides can include fragments of the amino acid sequence of FGFR3 isoform 2 (e.g., at least amino acids 1 to 300, 1 to 310, 1 to 320, 1 to 330, 1 to 340, 1 to 350, 1 to 360, 1 to 370, 1 to 380, 1 to 390, 1 to 400, 1 to 410, 1 to 420, 1 to 430, 1 to 440, 1 to 440, 1 to 450, 1 to 460, 1 to 470, 1 to 480, 1 to 490, 1 to 500, 1 to 510, 1 to 520, 1 to 530, 1 to 540, 1 to 550, 1 to 560, 1 to 570, 1 to 580, 1 to 590, 1 to 600, 1 to 610, 1 to 620, 1 to 630, 1 to 640, 1 to 650, 1 to 660, 1 to 670, 1 to 680, or 1 to 690 of SEQ ID NO: 1). In particular, sFGFR3 polypeptides may include, but are not limited to, amino acids 1 to 323 of SEQ ID NO: 1 (sFGFR3_Del4), amino acids 1 to 440 of SEQ ID NO: 1 (sFGFR3_Del3), or amino acids 1 to 548 of SEQ ID NO: 1 (sFGFR3_Del2).

sFGFR3 polypeptides and fragments thereof can also include an N-terminal signal peptide sequence. The N-terminal signal peptide is present on the synthesized protein when it is synthesized, but is typically cleaved from the sFGFR3 polypeptide upon export of the polypeptide from the cell. The sFGFR3 polypeptides and sFGFR3 fusion polypeptides of the invention include both secreted (i.e., lacking the N-terminal signal) and non-secreted (i.e., having the N-terminal signal) forms thereof. One skilled in the art will appreciate that the position of the N-terminal signal peptide will vary in different sFGFR3 polypeptides and may include, for example, the first 5, 8, 10, 11, 12, 13, 14, 15, 16, 17, 18, 19, 20, 21, 22, 23, 24, 25, 27, 30, or more amino acid residues on the N-terminus of the polypeptide. For example, an exemplary signal peptide can include, but is not limited to, amino acids 1 to 22 of SEQ ID NO: 1. Additionally, sFGFR3 polypeptides and fusion polypeptides, such as sFGFR3_Del2, sFGFR3_Del3, and sFGFR3_Del4 (corresponding to amino acids 1 to 548 of SEQ ID NO: 1, amino acids 1 to 440 of SEQ ID NO: 1, and amino acids 1 to 323 of SEQ ID NO: 1, respectively), or a FGFR3 fusion polypeptide (e.g., SEQ ID NO: 4 with or without amino acids 1 to 8 of SEQ ID NO: 4 or SEQ ID NO: 33), can include deletions of the N-terminal amino acids, e.g., at least the amino acids 1 to 5, 10, 15, 20, 25, 30, 35, 40, 45, 50, 55, or 56 of SEQ ID NO: 1. One of skill in the art can predict the position of a signal sequence cleavage site, e.g., by an appropriate computer algorithm such as that described in Bendtsen et al. (J. Mol. Biol. 340(4):783-795, 2004) and available on the Web at www.cbs.dtu.dk/services/SignalP/.

### sFGFR3 Fusion Polypeptides

sFGFR3 polypeptides of the invention can optionally be fused to a functional domain from a heterologous polypeptide (e.g., an aggrecan-binding protein) to provide a sFGFR3 fusion polypeptide, as described herein. In some sFGFR3 polypeptides, a flexible linker, may be included between the sFGFR3 polypeptide and the fusion polypeptide (e.g., an aggrecan-binding protein), such as a serine or glycine-rich sequence (e.g., a poly-glycine or a poly-glycine/serine linker). Further exemplary fusion proteins and linkers are described below.

For example, the sFGFR3 polypeptides described above, such as fragments of sFGFR3_Del1 (e.g., amino acids 1 to 323 of SEQ ID NO: 1 (sFGFR3_Del4), amino acids 1 to 440 of SEQ ID NO: 1 (sFGFR3_Del3), or amino acids 1 to 548 of SEQ ID NO: 1 (sFGFR3_Del2)), can be a fusion polypeptide including, e.g., an aggrecan-binding protein or any polypeptide that targets cartilage when administered to a subject (e.g., a human). In particular, any functional portion of a protein that binds to aggregan, e.g., any protein that interacts with the globular domain (G1, G2, or G3) of aggrecan, can be included in a sFGFR3 fusion polypeptide

Exemplary aggrecan-binding proteins that can be used to produce the SFGFR3 fusion polypeptides described herein include antibodies, fibulin-1, borrelial aggrecan-binding proteins (e.g., Borrelia glycosaminoglycan-binding protein (Bgp) and Borrelia burgdorferi high temperature requirement A (BbHtrA)), and cartilage oligomeric matrix protein/thrombospondin 5 (COMP/TSP5). For example, an aggrecan-binding protein may include human hyaluronan and proteoglycan link protein 1 (HPLN1) or fragments thereof. In particular, the HPLN1 fragment can include a cartilage link domain 1 (LK1; amino acids 158 to 252 of SEQ ID NO: 3), a cartilage link domain 2 (LK2; amino acids 259 to 349 of SEQ ID NO: 3), or both LK1 and LK2 domains (158 to 349 amino acids of SEQ ID NO: 3). Additional sFGFR3 fusion polypeptides may include any polypeptide that has at least 50% (e.g., 55%, 60%, 65%, 70%, 75%, 80%, 85%, 86%, 87%, 88%, 89%, 90%, 91%, 92%, 93%, 94%, 95%, 96%, 97%, 98%, 99%, or more) amino acid sequence identity to the HPLN1 polypeptide (e.g., SEQ ID NO: 3) or an amino acid fragment thereof (e.g., at least 50, 100, 110, 120, 130, 140, 150, 150, 170, 180, 190, 200, 210, 220, 230, 240, 250, 260, 270, 280, 290, 300, 310, 320, 330, 340, 350, or more amino acid residues of the entire length of the HPLN1 amino acid sequence (i.e., SEQ ID NO: 3)). For example, an aggrecan-binding protein or fragment thereof of a FGFR3 fusion polypeptide can include, e.g., both LK1 and LK2 domains (amino acids 158 to 349 of SEQ ID NO: 3), in combination with a sFGFR3 polypeptide or fragment thereof, such as amino acids 1 to 323 of SEQ ID NO: 1 (sFGFR3_Del4).

Additionally, the sFGFR3 polypeptides (e.g., sFGFR3_Del2, sFGFR3_Del3, and sFGFR3_Del4) can be a fusion polypeptide including, e.g., an Fc region of an immunoglobulin at the N-terminal or C-terminal domain. An immunoglobulin molecule has a structure that is well known in the art. It includes two light chains (∼23 kD each) and two heavy chains (∼50-70 kD each) joined by inter-chain disulfide bonds. Immunoglobulins are readily cleaved proteolytically (e.g., by papain cleavage) into Fab (containing the light chain and the VH and CH1 domains of the heavy chain) and Fc (containing the CH2 and CH3 domains of the heavy chain, along with adjoining sequences). Useful Fc fragments as described herein include the Fc fragment of any immunoglobulin molecule, including IgG, IgM, IgA, IgD, or IgE, and their various subclasses (e.g., IgG-1, IgG-2, IgG-3, IgG-4, IgA-1, IgA-2), from any mammal (e.g., human). For instance, the Fc fragment is human IgG-1. The Fc fragments of the invention may include, for example, the CH2 and CH3 domains of the heavy chain and any portion of the hinge region. The Fc region may optionally be glycosylated at any appropriate one or more amino acid residues known to those skilled in the art. An Fc fragment as described herein may have 1, 2, 3, 4, 5, 6, 7, 8, 9, 10, 11, 12, 13, 14, 15, 16, 17, 18, 19, 20, 25, 30, 35, 40, 50, or more additions, deletions, or substitutions relative to any of the Fc fragments described herein.

The sFGFR3 fusion polypeptides described herein may include a peptide linker region between the SFGFR3 polypeptide or fragment thereof and the heterologous polypeptide or fragment thereof (e.g., an aggrecan-binding protein or fragment thereof or an Fc region). The linker region may be of any sequence and length that allows the sALP to remain biologically active, e.g., not sterically hindered. Exemplary linker lengths are between 1 and 200 amino acid residues, e.g., 1-5, 6-10, 11-15, 16-20, 21-25, 26-30, 31-35, 36-40, 41-45, 46-50, 51-55, 56-60, 61-65, 66-70, 71-75, 76-80, 81-85, 86-90, 91-95, 96-100, 101-110, 111-120, 121-130, 131-140, 141-150, 151-160, 161-170, 171-180, 181-190, or 191-200 amino acid residues. For instance, linkers include or consist of flexible portions, e.g., regions without significant fixed secondary or tertiary structure. Preferred ranges are 5 to 25 and 10 to 20 amino acids in length. Such flexibility is generally increased if the amino acids are small and do not have bulky side chains that impede rotation or bending of the amino acid chain. Thus, preferably the peptide linker of the present invention has an increased content of small amino acids, in particular of glycines, alanines, serines, threonines, leucines and isoleucines.

Exemplary flexible linkers are glycine-rich linkers, e.g., containing at least 50%, 60%, 65%, 70%, 75%, 80%, 85%, 90%, 95%, or even 100% glycine residues. Linkers may also contain, e.g., serine-rich linkers, e.g., containing at least 50%, 60%, 65%, 70%, 75%, 80%, 85%, 90%, 95%, or even 100% serine residues. In some cases, the amino acid sequence of a linker consists only of glycine and serine residues. For example, the sFGFR3 fusion polypeptide (e.g., SEQ ID NO: 4 with or without amino acid residues 1 to 8 of SEQ ID NO: 4 or SEQ ID NO: 33) can include a glycine and serine linker, such as the amino acid sequence SGSGSGSGSGSGSGS. A linker may optionally be glycosylated at any appropriate one or more amino acid residues. Additionally, a linker as described herein may include any other sequence or moiety, attached covalently or non-covalently. The linker may also be absent, in which the FGFR3 polypeptide and heterologous polypeptide (e.g., an aggrecan-binding protein or fragment thereof or Fc region) are fused together directly, with no intervening residues.

Additional amino acid residues can be introduced into the FGFR3 fusion polypeptide according to the cloning strategy used to produce the FGFR3 fusion polypeptides. For instance, the additional amino acid residues do not provide a portion of the FGFR3 transmembrane domain in order to maintain the polypeptide in a soluble form. Furthermore, any such additional amino acid residues, when incorporated into the FGFR3 polypeptide or fusion polypeptide of the invention, do not provide a cleavage site for endoproteases of the host cell. The likelihood that a designed sequence would be cleaved by the endoproteases of the host cell can be predicted as described, e.g., by Ikezawa (Biol. Pharm. Bull. 25:409-417, 2002).

The FGFR3 polypeptides and fusion polypeptides of the invention may be associated into dimers or tetramers. Additionally, the polypeptide or fusion polypeptide of the invention (e.g., a sFGFR3 polypeptide or fusion polypeptide) may be glycosylated or PEGylated.

### Production of sFGFR3 Nucleic Acids and Polypeptides

Nucleic acids encoding sFGFR3 and sFGFR3 fusion polypeptides of the invention can be produced by any method known in the art. Typically, a nucleic acid encoding the desired fusion polypeptide is generated using molecular cloning methods, and is generally placed within a vector, such as a plasmid or virus. The vector is used to transform the nucleic acid into a host cell appropriate for the expression of the fusion polypeptide. Representative methods are disclosed, for example, in Maniatis et al. (Cold Springs Harbor Laboratory, 1989). Many cell types can be used as appropriate host cells, although mammalian cells are preferable because they are able to confer appropriate post-translational modifications. Host cells of the present invention may include, e.g., Human Embryonic Kidney 293 (HEK 293) cells, Chinese Hamster Ovary (CHO) cell, L cell, C127 cell, 3T3 cell, BHK cell, COS-7 cell or any other suitable host cell known in the art. For example, the host cell is a HEK 293 cells. Alternatively, the host cell can be a CHO cell.

### Methods of Treatment

Provided herein are methods for treating a skeletal growth retardation disorder in a patient, such as a patient having achondroplasia (e.g., a human having achondroplasia). In particular, the patient may exhibit or may be likely to have one or more symptoms of a skeletal growth retardation disorder (e.g., achondroplasia). The method involves administering a sFGFR3 polypeptide, such as sFGFR3_Del2, sFGFR3_Del3, and sFGFR3_Del4 (corresponding to amino acids 1 to 548 of SEQ ID NO: 1, amino acids 1 to 440 of SEQ ID NO: 1, and amino acids 1 to 323 of SEQ ID NO: 1, respectively), or a FGFR3 fusion polypeptide (e.g., SEQ ID NO: 4 with or without amino acid residues 1 to 8 of SEQ ID NO: 4 or SEQ ID NO: 33) to the patient (e.g., a human). For example, the patient exhibits signs or symptoms of a skeletal growth retardation disorder, such as those described herein (e.g., achondroplasia), e.g., prior to administration of the sFGFR3 polypeptide or FGFR3 fusion polypeptide. Treatment with a sFGFR3 polypeptide of the invention, such as sFGFR3_Del2, sFGFR3_Del3, and sFGFR3_Del4 (e.g., amino acids 1 to 548 of SEQ ID NO: 1, amino acids 1 to 440 of SEQ ID NO: 1, and amino acids 1 to 323 of SEQ ID NO: 1, respectively), or a sFGFR3 fusion polypeptide of the invention (e.g., SEQ ID NO: 4 with or without amino acid residues 1 to 8 of SEQ ID NO: 4 or SEQ ID NO: 33) can also occur after a patient (e.g., a human) has been diagnosed with a skeletal growth retardation disorder, such as those described herein (e.g., achondroplasia), or after a patient exhibits signs or symptoms of a skeletal growth retardation disorder, such as those described herein (e.g., achondroplasia). In particular, the patient is treated with sFGFR3_Del4-LK1-LK2.

Treatment with a sFGFR3 polypeptide, such as sFGFR3_Del2, sFGFR3_Del3, and sFGFR3_Del4 (e.g., amino acids 1 to 548 of SEQ ID NO: 1, amino acids 1 to 440 of SEQ ID NO: 1, and amino acids 1 to 323 of SEQ ID NO: 1, respectively), or a sFGFR3 fusion polypeptide (e.g., SEQ ID NO: 4 with or without amino acid residues 1 to 8 of SEQ ID NO: 4 or SEQ ID NO: 33) can result in an improvement in a symptom of a skeletal growth retardation disorder, e.g., achondroplasia. The methods can be used to treat symptoms associated with a skeletal growth retardation disorder, e.g., achondroplasia, such that there is reversal or a reduction in the severity of symptoms of the skeletal growth retardation disorder, e.g., achondroplasia.

### Skeletal Growth Retardation Disorder

Skeletal growth retardation disorders can be treated or prevented by administering a sFGFR3 polypeptide or a sFGFR3 fusion polypeptide as described herein. In particular, the method involves administering a sFGFR3 polypeptide, such as sFGFR3_Del2, sFGFR3_Del3, and sFGFR3_Del4 (e.g., amino acids 1 to 548 of SEQ ID NO: 1, amino acids 1 to 440 of SEQ ID NO: 1, and amino acids 1 to 323 of SEQ ID NO: 1, respectively), or a sFGFR3 fusion polypeptide (e.g., sFGFR3_Del4-LK1-LK2 (SEQ ID NO: 4 with or without amino acid residues 1 to 8 of SEQ ID NO: 4 or SEQ ID NO: 33)) to a patient (e.g., a human). Skeletal growth retardation disorders that can be treated with the sFGFR3 polypeptides and sFGFR3 fusion polypeptides described herein are characterized by deformities and/or malformations of the bones and can include, but are not limited to, FGFR3-related skeletal diseases. In particular, the patient is treated with sFGFR3_Del4-LK1-LK2.

Administration of a sFGFR3 polypeptide or a sFGFR3 fusion polypeptide as described herein (such as, e.g., sFGFR3_Del4-LK1-LK2) can treat a skeletal growth retardation disorder including, but not limited to, achondroplasia, achondrogenesis, acrodysostosis, acromesomelic dysplasia, atelosteogenesis, camptomelic dysplasia, chondrodysplasia punctata, rhizomelic type of chondrodysplasia punctata, cleidocranial dysostosis, congenital short femur, Crouzon syndrome, Apert syndrome, Jackson-Weiss syndrome, Pfeiffer syndrome, Crouzonodermoskeletal syndrome, dactyly, brachydactyly, camptodactyly, polydactyly, syndactyly, diastrophic dysplasia, dwarfism, dyssegmental dysplasia, enchondromatosis, fibrochondrogenesis, fibrous dysplasia, hereditary multiple exostoses, hypophosphatasia, hypophosphatemic rickets, Jaffe-Lichtenstein syndrome, Kniest dysplasia, Kniest syndrome, Langer-type mesomelic dysplasia, Marfan syndrome, McCune-Albright syndrome, micromelia, metaphyseal dysplasia, Jansen-type metaphyseal dysplasia, metatrophic dysplasia, Morquio syndrome, Nievergelt-type mesomelic dysplasia, neurofibromatosis (such as type 1 (e.g., with bone manifestations or without bone manifestations), type 2, or schwannomatosis), osteoarthritis, osteochondrodysplasia, osteogenesis imperfecta, perinatal lethal type of osteogenesis imperfecta, osteopetrosis, osteopoikilosis, peripheral dysostosis, Reinhardt syndrome, Roberts syndrome, Robinow syndrome, short-rib polydactyly syndromes, short stature, spondyloepiphyseal dysplasia congenita, or spondyloepimetaphyseal dysplasia. For instance, administration of the sFGFR3 polypeptides or sFGFR3 fusion polypeptide described herein may resolve and/or prevent symptoms associated with any of the aforementioned disorders.

The sFGFR3 polypeptides and sFGFR3 fusion polypeptides of the present invention can be used to treat symptoms associated with a skeletal growth retardation disorder, such as a FGFR3-related skeletal disease (e.g., achondroplasia). Non-limiting examples of symptoms of skeletal growth retardation disorders that may be treated, e.g., with a sFGFR3 polypeptide or a sFGFR3 fusion polypeptide, include the following: short limbs and trunk, bowlegs, a waddling gait, skull malformations (e.g., a large head), cloverleaf skull, craniosynostosis (e.g., premature fusion of the bones in the skull), wormian bones (e.g., abnormal thread-like connections between the bones in the skull), anomalies of the hands and feet (e.g., polydactyly or extra fingers), "hitchhiker" thumbs and abnormal fingernails and toenails, and chest anomalies (e.g., pear-shaped chest or narrow thorax). Additional symptoms that can treated by administering sFGFR3 polypeptides and sFGFR3 fusion polypeptides can also include non-skeletal abnormalities in patients having skeletal growth retardation disorders, e.g., anomalies of the eyes, mouth, and ears, such as congenital cataracts, myopia, cleft palate, or deafness; brain malformations, such as hydrocephaly, porencephaly, hydranencephaly, or agenesis of the corpus callosum; heart defects, such as atrial septal defect, patent ductus arteriosus, or transposition of the great vessels; developmental delays; or mental retardation. Accordingly, adinistration of a sFGFR3 polypeptide or a sFGFR3 fusion polypeptide, as described herein, may result in an improvement in one or more symptoms of a skeletal growth retardation disorder.

Any skeletal growth retardation disorder that is a FGFR3-related skeletal disease (e.g., caused by or associated with overactivation of FGFR3 as result of a gain-of-function FGFR3 mutation) can be treated by administering a sFGFR3 polypeptide or a sFGFR3 fusion polypeptide as described herein to a patient (e.g., a human). For example, a sFGFR3 polypeptide or a sFGFR3 fusion polypeptide can be administered to treat FGFR3-related diseases, such as skeletal dysplasias and FGFR3-related craniosynostosis. FGFR3-related skeletal diseases can include, but are not limited to, achondroplasia, thanatophoric dysplasia type I (TDI), thanatophoric dysplasia type II (TDII), severe achondroplasia with developmental delay and acanthosis nigricans (SADDAN), hypochondroplasia, and craniosynostosis (e.g., Muenke syndrome and Crouzon syndrome with acanthosis nigricans).

Patients (e.g., human patients) with mutations in the *FGFR3* gene associated with different FGFR3-related skeletal disorders, such as achondroplasia, hypochondroplasia, SADDAN, TDI, and TDII, can also be treated with a sFGFR3 polypeptide, such as sFGFR3_Del2, sFGFR3_Del3, and sFGFR3_Del4, or a sFGFR3 fusion polypeptide, such as a fusion polypeptide including sFGFR3_Del4 and a fragment of an aggrecan-binding protein (e.g., sFGFR3_Del4-LK1-LK2 (SEQ ID NO: 4 with or without amino acid residues 1 to 8 of SEQ ID NO: 4 or SEQ ID NO: 33)).

For example, the sFGFR3 polypeptide or sFGFR3 fusion polypeptide can be administered to treat achondroplasia resulting from the G380R, G375C, G346E or S279C mutations of the *FGFR3* gene. Administration of the sFGFR3 polypeptides and sFGFR3 fusion polypeptides may be used to treat the following exemplary FGFR3-related skeletal disorders: hypochondroplasia resulting from the G375C, G346E or S279C mutations of the *FGFR3* gene; TDI resulting from the R248C, S248C, G370C, S371C, Y373C, X807R, X807C, X807G, X807S, X807W and K650M mutations of the *FGFR3* gene; TDII resulting from the K650E mutation of the *FGFR3* gene; and SADDAN resulting from the K650M mutation of the *FGFR3* gene. Thus, a patient treated with the sFGFR3 polypeptides or sFGFR3 fusion polypeptides disclosed herein may have, e.g., a mutation in the *FGFR3* gene.

Any of the aforementioned mutations in the *FGFR3* gene (e.g., the G380R mutation of the *FGFR3* gene) can be detected in a sample from the patient (e.g., a human with achondroplasia, hypochondroplasia, SADDAN, TDI, and TDII) prior to or after treatment (e.g., treatment with a sFGFR3 polypeptide, such as sFGFR3_Del2, sFGFR3_Del3, and sFGFR3_Del4, or a sFGFR3 fusion polypeptide (e.g., sFGFR3_Del4-LK1-LK2). Additionally, the parents of the patient and/or fetal samples (e.g., fetal nucleic acid obtained from maternal blood, placental, or fetal samples) may be tested by methods known in the art for the mutation.

### Achondroplasia

Achondroplasia is the most common cause of dwarfism in humans and can be treated or prevented by administering a sFGFR3 polypeptide or a sFGFR3 fusion polypeptide as described herein. In particular, achondroplasia may be treated by administering a FGFR3 polypeptide, such as sFGFR3_Del2, sFGFR3_Del3, and sFGFR3_Del4 (e.g., amino acids 1 to 548 of SEQ ID NO: 1, amino acids 1 to 440 of SEQ ID NO: 1, and amino acids 1 to 323 of SEQ ID NO: 1, respectively) or a sFGFR3 fusion polypeptide (e.g., sFGFR3_Del4-LK1-LK2 (SEQ ID NO: 4 with or without amino acid residues 1 to 8 of SEQ ID NO: 4 or SEQ ID NO: 33)) to a patient (e.g., a human). Administration of a sFGFR3 polypeptide or a sFGFR3 fusion polypeptide, as described herein, may result in an improvement in symptoms including, but not limited to, growth retardation, skull deformities, orthodontic defects, cervical cord compression (with risk of death, e.g., from central apnea or seizures), spinal stenosis (e.g., leg and lower back pain), hydrocephalus (e.g., requiring cerebral shunt surgery), hearing loss due to chronic otitis, cardiovascular disease, neurological disease, respiratory problems, fatigue, pain, numbness in the lower back and/or spin, and obesity.

Patients treated using the sFGFR3 polypeptides or the sFGFR3 fusion polypeptides described herein may include, e.g., infants, children, and adults with achondroplasia. Infants are often diagnosed with achondroplasia at birth, and thus, treatment with a sFGFR3 polypeptide or sFGFR3 fusion protein, as described herein, may begin as early as possible in the patient's life, e.g., shortly after birth, or prior to birth (*in utero*)*.*

Symptoms of achondroplasia in patients (e.g., humans) may also be monitored prior to or after a patient is treated with a sFGFR3 polypeptide, such as sFGFR3_Del2, sFGFR3_Del3, and sFGFR3_Del4 (e.g., amino acids 1 to 548 of SEQ ID NO: 1, amino acids 1 to 440 of SEQ ID NO: 1, and amino acids 1 to 323 of SEQ ID NO: 1, respectively), or a sFGFR3 fusion polypeptide (e.g., sFGFR3_Del4-LK1-LK2 (SEQ ID NO: 4 with or without amino acid residues 1 to 8 of SEQ ID NO: 4 or SEQ ID NO: 33)). For instance, symptoms of achondroplasia may be monitored prior to treatment to assess the severity of achondroplasia and condition of the patient prior to performing the methods. The methods of the invention may include diagnosis of achondroplasia in a patient and monitoring the patient for changes in the symptoms of achondroplasia, such as changes in body weight, skull length and/or skull width of the patient based on changes monitored over a period of time, e.g., 1, 2, 3, 4 or more times per month or per year or approximately every 1, 2, 3, 4, 5, 6, 7, 8, 12 or 16 weeks over the course of treatment with the sFGFR3 polypeptide or the sFGFR3 fusion polypeptide of the present invention. Body weight and/or skull size of the patient or changes thereof can also be determined at treatment specific events, e.g. before and/or after administration of the sFGFR3 polypeptide or sFGFR3 fusion polypeptide as described herein. For example, body weight and/or skull size are measured in response to administration of the sFGFR3 polypeptide or sFGFR3 fusion polypeptide of the present invention.
Body weight can be measured simply be weighing the subject on a scale, preferably in a standardized manner, e.g. with the same (in particular for humans) or no clothes or at a certain time of the day, preferably in a fasting state (for example in the morning before breakfast is taken, or after at least 1, 2, 3, 4, 5 or more hours of fasting).

Skull size is preferably represented by length, height, width and/or circumference. Measurements can be taken by any known or self-devised standardized method. For a human subject, the measurement of skull circumference is preferred. It is usually taken with a flexible and non-stretchable material such as a tape, which is wrapped around the widest possible circumference of the head (though not around the ears or the facial area below and including the eyebrows), e.g. from the most prominent part of the forehead around to the widest part of the back of the head. Another preferred measurement for a human subject can determine the height of the skull, for example from the underside of the chin to the uppermost point of the head. For a rodent subject, the measurement of the length of the skull (e.g. tip of the nasal bone to back of the occipital bone) is preferred. Alternatively, also the width of the skull (e.g. widest points of the parietal bone) or the height of the skull (e.g. lowest point of the angular process of lower jaw to frontal bone) are preferred. Preferably, any measurement is taken more than once, e.g. at least 3 times, and the largest number us taken as the length, height, width and/or circumference.

### Pharmaceutical compositions and formulations

A composition of the present invention (e.g., including a sFGFR3 polypeptide, such as sFGFR3_Del2, sFGFR3_Del3, and sFGFR3_Del4 (e.g., amino acids 1 to 548 of SEQ ID NO: 1, amino acids 1 to 440 of SEQ ID NO: 1, and amino acids 1 to 323 of SEQ ID NO: 1, respectively), or a sFGFR3 fusion polypeptide (e.g., sFGFR3_Del4-LK1-LK2 (SEQ ID NO: 4 with or without amino acid residues 1 to 8 of SEQ ID NO: 4 or SEQ ID NO: 33)) can be administered by a variety of methods known in the art. As will be appreciated by the skilled artisan, the route and/or mode of administration will vary depending upon the desired results. The route of administration can depend on a variety of factors, such as the environment and therapeutic goals. In particular, the sFGFR3 polypeptides and sFGFR3 fusion polypeptides described herein (e.g., sFGFR3_Del4-LK1-LK2) can be administered by any route known in the art, e.g., subcutaneous (e.g., by subcutaneous injection), intravenously, orally, nasally, intramuscularly, sublingually, intrathecally, or intradermally. By way of example, pharmaceutical compositions of the invention can be in the form of a liquid, solution, suspension, pill, capsule, tablet, gelcap, powder, gel, ointment, cream, nebulae, mist, atomized vapor, aerosol, or phytosome.

### Dosage

Any amount of a pharmaceutical composition (e.g., including a sFGFR3 polypeptide, such as sFGFR3_Del2, sFGFR3_Del3, and sFGFR3_Del4 (e.g., amino acids 1 to 548 of SEQ ID NO: 1, amino acids 1 to 440 of SEQ ID NO: 1, and amino acids 1 to 323 of SEQ ID NO: 1, respectively), or a sFGFR3 fusion polypeptide (e.g., sFGFR3_Del4-LK1-LK2 (SEQ ID NO: 4 with or without amino acid residues 1 to 8 of SEQ ID NO: 4 or SEQ ID NO: 33)) can be administered to a patient, such as a patient with a skeletal growth retardation disorder (e.g., a patient with achondroplasia). The dosages will depend on many factors including the mode of administration and the age of the patient. Typically, the amount of the composition (e.g., including a sFGFR3 polypeptide or a sFGFR3 fusion polypeptide) contained within a single dose will be an amount that is effective to treat a condition (e.g., achondroplasia) as described herein without inducing significant toxicity.

For example, the sFGFR3 polypeptides, such as sFGFR3_Del2, sFGFR3_Del3, and sFGFR3_Del4 (e.g., amino acids 1 to 548 of SEQ ID NO: 1, amino acids 1 to 440 of SEQ ID NO: 1, and amino acids 1 to 323 of SEQ ID NO: 1, respectively), or sFGFR3 fusion polypeptides (e.g., sFGFR3_Del4-LK1-LK2 (SEQ ID NO: 4 with or without amino acids 1 to 8 of SEQ ID NO: 4 or SEQ ID NO: 33)) described herein can be administered to patients (e.g., patients with achondroplasia) in individual doses ranging, e.g., from 0.0002 mg/kg to about 20 mg/kg (e.g., from 0.002 mg/kg to 20 mg/kg, from 0.01 mg/kg to 2 mg/kg, from .2 mg/kg to 20 mg/kg, from 0.01 mg/kg to 10 mg/kg, from 10 mg/kg to 100 mg/kg, from 0.1 mg/kg to 50 mg/kg, 0.5 mg/kg to 20 mg/kg, 1.0 mg/kg to 10 mg/kg, 1.5 mg/kg to 5 mg/kg, or 0.2 mg/kg to 3 mg/kg). In particular, the sFGFR3 polypeptide or sFGFR3 fusion polypeptides as described herein can be administered in individual doses of, e.g., 0.001 mg/kg to 7 mg/kg. These doses can be administered one or more times (e.g., 2, 3, 4, 5, 6, 7, 8, 9, 10, 11, or 12 or more times) per day, week, month, or year.

Exemplary doses of the sFGFR3 polypeptides of fragments thereof, such as sFGFR3_Del2, sFGFR3_Del3, and sFGFR3_Del4 (e.g., amino acids 1 to 548 of SEQ ID NO: 1, amino acids 1 to 440 of SEQ ID NO: 1, and amino acids 1 to 323 of SEQ ID NO: 1, respectively), or sFGFR3 fusion polypeptides (e.g., sFGFR3_Del4-LK1-LK2 (SEQ ID NO: 4 with or without amino acids 1 to 8 of SEQ ID NO: 4 or SEQ ID NO: 33)) include, e.g., 0.0002, 0.0005, 0.0010, 0.002, 0.005, 0.01, 0.02, 0.05, 0.1, 0.2, 0.5, 1, 2, 3, 2.5, 4, 5, 6, 7, 8, 9, 10, 15, or 20 mg/kg. For all dosages or ranges recited herein, the term "about" may be used to modify these dosages by ±10% of the recited values or range endpoints. In particular, sFGFR3 compositions in accordance with the present disclosure can be administered to patients in doses ranging from about 0.0002 mg/kg/day to about 20 mg/kg/day, about 0.02 mg/kg/day to about 15 mg/kg/day, or about 0.2 mg/kg/day to about 10 mg/kg/day (e.g., 0.75 mg/kg/day). For example, the sFGFR3 compositions can be administered to patients in a weekly dosage ranging, e.g., from about 0.0014 mg/kg/week to about 140 mg/kg/week, e.g., about 0.14 mg/kg/week to about 105 mg/kg/week, or, e.g., about 1.4 mg/kg/week to about 70 mg/kg/week (e.g., 5 mg/kg/week). The dosage will be adapted by the clinician in accordance with conventional factors such as the extent of the disease (e.g., achondroplasia) and different parameters from the patient (e.g., a patient with achondroplasia).

Dosages of compositions including sFGFR3 polypeptides, such as sFGFR3_Del2, sFGFR3_Del3, and sFGFR3_Del4 (e.g., amino acids 1 to 548 of SEQ ID NO: 1, amino acids 1 to 440 of SEQ ID NO: 1, and amino acids 1 to 323 of SEQ ID NO: 1, respectively), or sFGFR3 fusion polypeptides (e.g., sFGFR3_Del4-LK1-LK2 (SEQ ID NO: 4 with or without amino acids 1 to 8 of SEQ ID NO: 4 or SEQ ID NO: 33)) may be provided in either a single or multiple dosage regimens. Doses can be administered, e.g., hourly, bihourly, daily, bidaily, twice a week, three times a week, four times a week, five times a week, six times a week, weekly, biweekly, monthly, bimonthly, or yearly. Alternatively, doses can be administered, e.g., twice, three times, four times, five times, six times, seven times, eight times, nine times, 10 times, 11 times, or 12 times per day. In particular, the dosing regimen is twice weekly. For example, the sFGFR3 polypeptides or sFGFR3 fusion polypeptides described herein can be administered at a dosage of 2.5 mg/kg twice weekly. The duration of the dosing regimen can be, e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 day(s), week(s), or month(s), or even for the remaining lifespan of the patient. The amount, frequency, and duration of dosage will be adapted by the clinician in accordance with conventional factors such as the extent of the disease and different parameters from the patient.

### Carriers/vehicles

Preparations containing a sFGFR3 polypeptide (e.g., amino acids 1 to 548 of SEQ ID NO: 1, amino acids 1 to 440 of SEQ ID NO: 1, or amino acids 1 to 323 of SEQ ID NO: 1, respectively), or sFGFR3 fusion polypeptides (e.g., sFGFR3_Del4-LK1-LK2 (SEQ ID NO: 4 with or without amino acids 1 to 8 of SEQ ID NO: 4 or SEQ ID NO: 33)) may be provided to patients in combination with pharmaceutically acceptable sterile aqueous or non-aqueous solvents, suspensions or emulsions. Examples of non-aqueous solvents are propylene glycol, polyethylene glycol, vegetable oil, fish oil, and injectable organic esters. Aqueous carriers include water, water-alcohol solutions, emulsions or suspensions, including saline and buffered medical parenteral vehicles including sodium chloride solution, Ringer's dextrose solution, dextrose plus sodium chloride solution, Ringer's solution containing lactose, or fixed oils.

Intravenous vehicles may include fluid and nutrient replenishers, electrolyte replenishers, such as those based upon Ringer's dextrose, and the like. Pharmaceutically acceptable salts can be included therein, for example, mineral acid salts such as hydrochlorides, hydrobromides, phosphates, sulfates, and the like; and the salts of organic acids such as acetates, propionates, malonates, benzoates, and the like. Additionally, auxiliary substances, such as wetting or emulsifying agents, pH buffering substances, and the like, may be present in such vehicles. A thorough discussion of pharmaceutically acceptable carriers is available in Remington's Pharmaceutical Sciences (Mack Pub. Co., N.J. 1991).

Preferably, the pharmaceutical compositions contain vehicles which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions. The doses used for the administration can be adapted as a function of various parameters, and in particular as a function of the mode of administration used, of the relevant pathology, or alternatively of the desired duration of treatment. For example, it is well within the skill of the art to start doses of the compound at levels lower than those required to achieve the desired therapeutic effect and to gradually increase the dosage until the desired effect is achieved. However, the daily dosage of the products may be varied over a wide range from 0.01 to 1.000 mg per adult per day. Preferably, the compositions contain 0.01, 0.05, 0.1, 0.5, 1.0, 2.5, 5.0, 10.0, 15.0, 25.0, 50.0, 100, 250 and 500 mg of the active ingredient for the symptomatic adjustment of the dosage to the subject to be treated. A medicament typically contains from about 0.01 mg to about 500 mg of the active ingredient, preferably from 1 mg to about 100 mg of the active ingredient. An effective amount of the drug is ordinarily supplied at a dosage level from 0.0002 mg/kg to about 20 mg/kg of body weight per day, especially from about 0.001 mg/kg to 7 mg/kg of body weight per day. To prepare pharmaceutical compositions, an effective amount of a polypeptide according to the invention may be dissolved or dispersed in a pharmaceutically acceptable carrier or aqueous medium.

The pharmaceutical forms suitable for injectable use include sterile aqueous solutions or dispersions; formulations including sesame oil, peanut oil or aqueous propylene glycol; and sterile powders for the extemporaneous preparation of sterile injectable solutions or 20 dispersions. In all cases, the form must be sterile and must be fluid to the extent that easy syringability exists. It must be stable under the conditions of manufacture and storage and must be preserved against the contaminating action of microorganisms, such as bacteria and fungi. Solutions of the active compounds as free base or pharmacologically acceptable salts can be prepared in water suitably mixed with a surfactant, such as hydroxypropylcellulose. Dispersions can also be prepared in glycerol, liquid polyethylene glycols, mixtures thereof and in oils. Under ordinary conditions of storage and use, these preparations contain a preservative to prevent the growth of microorganisms.

The polypeptides and nucleic acids according to the invention can be formulated into a composition in a neutral or salt form. Pharmaceutically acceptable salts include the acid addition salts (formed with the free amino groups of the protein) and which are formed with inorganic acids such as,for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like. The carrier can also be a solvent or dispersion medium containing, for example, water, ethanol, polyol (for example, glycerol, propylene glycol, and liquid polyethylene glycol, and 5 the like), suitable mixtures thereof, and vegetables oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminium monostearate and gelatin.

Sterile injectable solutions are prepared by incorporating the active compounds in the required amount in the appropriate solvent with several of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof. The preparation of more, or highly concentrated solutions for direct injection is also contemplated, where the use of DMSO as solvent is envisioned to result in extremely rapid penetration, delivering high concentrations of the active agents to a small tumor area. Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed. For parenteral administration in an aqueous solution, for example, the solution may be suitably buffered and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. For example, one dosage could be dissolved in 1 ml of isotonic NaCl solution and either added to 1000 ml of hypodermoclysis fluid or injected at the proposed site of infusion. Some variation in dosage will necessarily occur depending on the condition of the subject being treated. The person responsible for administration will, in any event, determine the appropriate dose for the subject.

### Gene Therapy

The sFGFR3 polypeptides, such as sFGFR3_Del2, sFGFR3_Del3, and sFGFR3_Del4 (e.g., amino acids 1 to 548 of SEQ ID NO: 1, amino acids 1 to 440 of SEQ ID NO: 1, and amino acids 1 to 323 of SEQ ID NO: 1, respectively), or sFGFR3 fusion polypeptides such as sFGFR3_Del4-LK1-LK2 (e.g., SEQ ID NO: 4 with or without amino acid residues 1 to 8 of SEQ ID NO: 4 or SEQ ID NO: 33), could also be delivered through gene therapy, where an exogenous nucleic acid encoding the proteins is delivered to tissues of interest and expressed *in vivo.* Gene therapy methods are discussed, e.g., in Verme et al. (Nature 389:239-242, 1997), Yamamoto et al. (Molecular Therapy 17:S67-S68, 2009), and Yamamoto et al., (J. Bone Miner. Res. 26:135-142, 2011), each of which is hereby incorporated by reference. Both viral and non-viral vector systems can be used. The vectors may be, for example, plasmids, artificial chromosomes (e.g., bacterial, mammalian, or yeast artificial chromosomes), virus or phage vectors provided with an origin of replication, and optionally, a promoter for the expression of the nucleic acid encoding the viral polypeptide and optionally, a regulator of the promoter. The vectors may contain one or more selectable marker genes, for example, an ampicillin or kanamycin resistance gene in the case of a bacterial plasmid or a resistance gene for a fungal vector. Vectors may be used in *in vitro,* for example, for the production of DNA, RNA, or the viral polypeptide, or may be used to transfect or transform a host cell, for example, a mammalian host cell, e.g., for the production of the viral polypeptide encoded by the vector. The vectors may also be adapted to be used *in vivo,* for example, in a method of vaccination or gene therapy.

Examples of suitable viral vectors include, retroviral, lentiviral, adenoviral, adeno-associated viral, herpes viral, including herpes simplex viral, alpha-viral, pox viral, such as Canarypox and vaccinia-viral based systems. Gene transfer techniques using these viruses are known in the art. Retrovirus vectors, for example, may be used to stably integrate the nucleic acids of the invention into the host genome. Replication-defective adenovirus vectors by contrast remain episomal and therefore allow transient expression. Vectors capable of driving expression in insect cells (e.g., baculovirus vectors), in human cells, yeast, or in bacteria may be employed in order to produce quantities of the viral polypeptide(s) encoded by the nucleic acids of the invention, for example, for use in subunit vaccines or in immunoassays. In an additional example, a replication-deficient simian adenovirus vector may be used as a live vector. These viruses contain an E1 deletion and can be grown on cell lines that are transformed with an E1 gene. These vectors can be manipulated to insert a nucleic acid of the invention, such that the encoded viral polypeptide(s) may be expressed.

Promoters and other expression regulatory signals may be selected to be compatible with the host cell for which expression is designed. For example, mammalian promoters include the metallothionein promoter, which can be induced in response to heavy metals such as cadmium, and the β-actin promoter. Viral promoters, such as the SV40 large T antigen promoter, human cytomegalovirus (CMV) immediate early (1E) promoter, rous sarcoma virus LTR promoter, adenovirus promoter, or a HPV promoter, particularly the HPV upstream regulatory region (URR) may also be used. All these promoters, as well as additional promoters, are well-described in the art.

The nucleic acid molecules described herein may also be administered using non-viral based systems. For example, these administration systems include microsphere encapsulation, poly(lactide-co-glycolide), nanoparticle, and liposome-based systems. Non-viral based systems also include techniques facilitating the delivery of "naked" polynucleotides (such as electroporation, "gene gun" delivery and various other techniques used for the introduction of polynucleotides).

The introduced polynucleotide can be stably or transiently maintained in the host cell. Stable maintenance typically requires that the introduced polynucleotide either contains an origin of replication compatible with the host cell or integrates into a replicon of the host cell such as an extrachromosomal replicon (e.g., a plasmid) or a nuclear or mitochondrial chromosome.

The invention is described by way of the following examples which are to be construed as merely illustrative and not limitative of the scope of the invention.

### EXAMPLES

### Example 1: Decoy design and testing procedures

### Structures and sequences of the different protein variants.

A diagram of the different domains of FGFR3, HPLN1 and a soluble FGFR3 (sFGFR3) is shown in Figure 1 (see PCT/EP2014/050800). The FGFR3 deletion variants of the examples are shown in Figure 2 and the fusion proteins of the examples are shown in Figure 3.

SEQ ID NO: 1 provides the amino acid sequence of sFGFR3 of PCT/EP2014/050800, SEQ ID NO: 2 the amino acid sequence of the same sFGFR3 but with the full Ig like C2 type domain 3, SEQ ID NO: 3 the amino acid sequence of HPLN1, SEQ ID NO: 4 the amino acid sequence of FLAG-sFGFR3_Del4-LK1-LK2 (see Figure 3B), SEQ ID NO: 5 the nucleic acid sequence of FLAG-sFGFR3_Del4-LK1-LK2 (see Figure 3B), and SEQ ID NO: 6 the wild-type human FGFR3.

### Cloning and protein production system.

The Del plasmids were obtained by site directed mutagenesis of the sFGFR3-pFLAG-CMV3 plasmid. The cDNA sequence for LK1-LK2 was optimized for Homo Sapiens while encoding for the original protein sequence (GeneOptimizer process, GeneArt). The synthesized fragment was subcloned into sFGFR3-pFLAG-CMV3 (Garcia, S. *et al..* 5, 203ra124 (2013)) using Pmll and Kpnl cloning sites. Plasmid DNA was purified from transformed bacteria and concentration determined by UV spectroscopy. Final constructs were verified by sequencing.

Recombinant proteins were produced by transient transfection using CaCl₂ transfection reagent in HEK 293 cells. Each transfection was performed in a cell factory (High flask T600, Merck Millipore) with 80% confluent HEK 293 in 100 ml DMEM without phenol red (Life Technologies) supplemented with 2 mM glutamine (Life Technologies) and antibiotics (Life Technologies). CaCl₂ (690 µl) and a pFLAG-sFGFR3 variant (240 µg) were resuspended with 6.27 ml H₂O in 7,2 ml HBS, incubated 30 min at room temperature, and then incubated for 16 h onto the cells at 37°C in 5% CO2. Medium was then replaced by 120 ml supplemented DMEM without phenol red. After 72 h, production medium was filtrated using 0.22 µm filters and concentrated on Amicon Ultra-15 60kDa (Merck Millipore). Recombinant protein was then purified using an affinity column (ANTI-FLAG M2 Affinity Gel, Sigma Aldrich) according to the manufacturer's instructions. Protein amounts were measured by specific ELISA (R&D Systems) according to the manufacturer's instructions.

### FGF binding.

Fixed amounts of human FGF2, or human FGF9 (R&D Systems) were incubated for 2 h at 37°C with increasing doses of each recombinant protein (0 to 250 ng/ml) in PBS 1% BSA. Specific commercial ELISA kits (R&D Systems; Clinisciences) were used to quantify remaining unbound FGFs. 1000 pg/ml or 1200 pg/ml were used for FGF2 and FGF9 respectively based on the sensitivity of the corresponding ELISA kits.

### Aggrecan binding.

ELISA plate were coated with 10 µg/mL of Aggrecan (R&D systems #1220-PG-025) in a final volume of 50µl per well and incubated overnight at room temperature. Plate were blocked with 300µl of PBS 1% BSA per well during 1h at room temperature. Different amount of protein (0 to 100□l) were incubated for 1h at room temperature. Specific detection antibody against FGFR3 from an R&D system ELISA kit (#DYC766E) was used to detect recombinant protein fixed to aggrecan as recommended by the manufacturer.

### Efficacy study in mice and evaluation of the velocity of growth.

The Principles of Laboratory Animal Care (NIH publication no. 85-23, revised 1985; http://grants1.nih.gov/grants/olaw/references/phspol.htm) and the European commission guidelines for the protection of animals used for scientific purposes (http://ec.europa.eu/environment/chemicals/lab_animals/legislation_en.htm) were followed at all times. All procedures were approved by the Institutional Ethic Committee for the use of Laboratory Animals (CIEPAL Azur) (approval # NCE-2012-52).

Experiments were performed on transgenic *Fgfr3*^{*ach*/*+*} animals in which expression of the mutant FGFR3 is driven by the Col2a1 promoter/enhancer (Naski et al., Development 125, 4977-4988, 1998). Mice were exposed to a 12 h light/dark cycle and had free access to standard laboratory food and water. Genotypes were verified by PCR of genomic DNA using the primers 5'-AGGTGGCCTTTGACACCTACCAGG-3' (SEQ ID NO: 31) and 5'-TCTGTTGTGTTTCCTCCCTGTTGG-3' (SEQ ID NO: 32), which amplify 360 bp of the FGFR3 transgene (Naski et al., *supra*)*.*

Several doses of each recombinant protein were tested. At day 3, all newborn mice from a single litter received the same dose. Control litters received 10 µl of PBS containing 50% glycerol (vehicle). Thereafter, subcutaneous injections were done twice a week for three weeks, alternatively on the left and right sides of the back. Mice were observed daily with particular attention to locomotion and urination alterations. Breeding was set up to theoretically generate litters with half wild type and half heterozygous *Fgfr3*^{*ach*/*+*} mice. To avoid bias due to variations of phenotype penetrance, experiments were performed on at least 2 litters (one treated and one control) arising from the same breeders. No statistical difference between males and females has been observed; they were thus considered as one group for all analyses (Garcia, S. et al. Sci. Transl. Med. 5, 203ra124, 2013).

At day 22, all animals were sacrificed by lethal injection of pentobarbital. Gender was determined. All subsequent measurements and analyses were performed without knowing mice genotype to avoid any experimental bias. Genotyping were performed at the end of the study to reveal the correspondence of data with a specific genotype. Because achondroplasia is a disease with an important phenotypic variability, all animals were included in the study, to improve the power of the study. Animals dead before day 22 were used for the study of the impact of treatment on premature death and animals reaching day 22 were used for all the analyses. All experiments and data measurements were performed in a blinded manner at all times.

Following sacrifice at day 22, body weights were measured. Blood (500 µl) was harvested by cardiac puncture and 25 µl were mixed with 25 µl 0.5 M EDTA. Samples were analyzed without centrifugation for blood numeration (Hemavet 950FS, Mascot Hematology). Cadavers were carefully skinned and eviscerated and skeletal measurements (body and tail lengths) were obtained using an electronic digital calliper (Fisher Scientific). Total body length was measured from the nose to the end of the last caudal vertebra; tail was measured starting at the first caudal vertebra. Organs were harvested, weighed and stored in 10% formalin for further histological analysis using standard paraffin-embedded techniques. Organs were observed for macroscopic abnormalities such as modification of color or texture, presence of nodules.

### PK/PD analysis.

To determine the *PK*/*PD* parameters of FLAG-sFGFR3 and FLAG-sFGFR3_Del4-LK1-LK2, 8 week-old WT mice received an intravenous or subcutaneous bolus of 50 mg/kg and 100 mg/kg of protein, respectively. At 15 min, 1 h, 3 h, 8 h, 24 h, and 48 h blood was harvested by retro-orbital puncture using heparin catheter (n = 4). Concentration of the FLAG tagged protein was measured by anti FLAG ELISA (Sigma).

### Statistical analysis.

Statistical analyses were performed with GraphPad Prism 6.0 software. To determine the statistical tests to be used, necessary assumptions were verified. To verify normality and equal variance, an Agostino and Pearson omnibus normality test (alpha=0.05) and a Brown-Forsythe test (p<0.05) were performed, respectively. Because all skeletal measurements data sets (body weight, body length, tail length, cranium length, width, length/width) fulfilled normality and equal variance requirements, two-tailed Student's t test for comparisons of two independent groups were used in the different statistical analyses. Comparison of mortality data between treated and control groups was done using a Kruskal-Wallis test (p<0.05) with a Dunn's test. Comparison of blood numeration was done using a one-way ANOVA with a Dunnett's multiple comparison test (95% CI). For organ weight correlation analyses, Pearson or Spearman tests were used when data sets followed or not normal distribution, respectively multiple comparison test (alpha 0.05). To compare correlations, a Fisher r-to-z transformation was performed. Comparison of decoys binding to human and murine FGFs was done by linear regression.

### Example 2: In vitro testing of the deletion variants

Summary: All four sFGFR3_Del1, sFGFR3_Del2, sFGFR3_Del3 and sFGFR3_Del4 variants bind human FGF2 with similar affinity than the sFGFR3 full-length construct. sFGFR3_Del4 binds FGF9 with the same affinity as FLAG-sFGFR3.

All four variants were tested *in vitro* for their ability to bind human FGF2. Similar to the protocol used to validate the mechanism of action of the FLAG-sFGFR3 molecule; different amounts of FLAG-sFGFR3_Del were incubated with constant quantities of FGF2. All variants bind human FGF2 in a receptor-dose-dependent manner with a similar affinity than the initial FLAG-sFGFR3 protein (Figure 4A). Linear regression analysis showed no statistical differences between the five slopes (*P*= 0,5478). sFGFR3_Del4 was also able to bind human FGF9 in a dose-dependent manner (Figure 4B).

### Example 3: In vitro testing of the Del4 deletion variant

Summary: Del4 is effective at restoring bone growth in transgenic *Fgfr3*^{*ach*/+} mice.

To evaluate FLAG-sFGFR3_Del4 for its therapeutic efficacy, 3 day-old animals received 2.5 mg/kg of protein twice per week for 3 weeks. Control groups received vehicle. Experiments were performed blinded. A total of 108 animals were included.

The biological effects of FLAG-sFGFR3_Del4 were evaluated following a 3-week-long injection regimen to 3 day-old neonate mice. All newborn male and female mice from one litter received the treatment twice per week over the course of 3 weeks: 2.5 mg/kg FLAG-sFGFR3_Del4, (n = 74) or vehicle for control groups (n = 52). The first observation was the significant reduction in mortality with treatment: mortality for vehicle-treated *Fgfr3*^{*ach*/*+*} mice was 63% compared with 40% in the treated group.

The velocity of growth was evaluated during the three-week treatment by monitoring cranium length and body weight on growing animals. Results show an improvement in growth velocity with the FLAG-sFGFR3_Del4 treatment (Figures 5A and 5B). Both type of measurement (body weight and skull length) also proved to be useful to evaluate the velocity of growth and monitor treatment response.

At day 22, all surviving animals were sacrificed and their growth was evaluated as measurements of body weight, body length, and tail length. FLAG-sFGFR3_Del4 treatment had a positive effect on overall skeletal growth in similar range than FLAG-sFGFR3 treatment (Figures 6A-6D). *Fgfr3*^{*ach*/*+*} mice were on average 20% lighter than their WT littermates, but gained weight when treated with FLAG-sFGFR3_Del4 (Figure 6A). Transgenic mice treated with FLAG-sFGFR3_Del4 had a stature (body and tail lengths) that was not significantly different from that of FLAG-sFGFR3 treated *Fgfr3*^{*ach*/*+*} mice and from that of vehicle-treated WT controls (*P* = 0.1743 for the tail length analysis, P = 0,3377 for the body length analysis, Student's t test), indicating that the extracellular domain of sFGFR3 was sufficient to restore bone growth in transgenic mice with achondroplasia.

### Example 4: In vitro testing of a targeted decoy

Summary: FLAG-sFGFR3_Del4-LK1-LK2 effectively binds FGF2 and aggrecan.

Prior to initiating *in vivo* studies, FLAG-sFGFR3_Del4-LK1-LK2 was evaluated for its ability to bind FGF2 and aggrecan. For this, different amounts of recombinant protein were incubated with constant quantities of FGF2. Our results show that FLAG-sFGFR3_Del4-LK1-LK2 binds human FGF2 with the same affinity as FLAG-sFGFR3 (Figure 7A).

To verify aggrecan binding, different amounts of recombinant protein were incubated with a fixed amount of aggrecan. As seen in Figure 7B, FLAG-sFGFR3_Del4-LK1-LK2 binds aggrecan in a dose dependent manner. FLAG-sFGFR3 and FLAG-sFGFR3_Del4 were used as negative controls and were not able to bind aggrecan (data not shown). These results confirm that a fusion protein containing the extracellular portion of sFGFR3 and portion of the HPLN1 protein can bind both FGF and aggrecan, a cartilage specific component.

### Example 5: In vivo testing of a targeted decoy

Summary: FLAG-sFGFR3_Del4-LK1-LK2 is effective at restoring bone growth in *Fgfr3*^{*ach*/*+*} mice.

Comparison of the pharmacokinetic parameters between the initial full-length protein and the fusion protein was performed by the injection of a bolus of protein either by the intravenous or subcutaneous route. Results show that the FLAG-sFGFR3_Del4-LK1-LK2 protein had similar PK parameters with a blood half-life within the same range than that of FLAG-sFGFR3 (see Table 1). However, it has to be kept in mind that the fusion protein targets the cartilage and is therefore retained in this tissue, such that the half-life measured using blood would be expected to be much lower in comparison to FLAG-sFGFR3 which keeps circulating. In fact, in view of the computed instability index (II) (37.41 for FLAG-sFGFR3_Del4-LK1-LK2 classifying it as stable vs. 44.04 for FLAG-sFGFR3, classifying it as unstable), surprisingly indicating that FLAG-sFGFR3_Del4-LK1-LK2 is more stable than FLAG-sFGFR3, and in view of the similar half-life determined in blood, it must be assumed that the actual time for elimination of FLAG-sFGFR3_Del4-LK1-LK2 from the body is significantly longer than for FLAG-sFGFR3, which is highly advantageous since it may allow reducing the effective dosage and/or the frequency of administration.

The therapeutic potential of FLAG-sFGFR3_Del4-LK1-LK2 was evaluated by injecting subcutaneously 0.3 mg/kg of protein twice per week for 3 weeks starting at age day 3. Control groups received vehicle and experiments were performed blinded. A total of 102 animals were included.

FLAG-sFGFR3_Del4-LK1-LK2 treatment resulted in a significant reduction in mortality of transgenic animals, from 63% in the control group to 37.5% in the treated group. Velocity of growth was significantly improved by the FLAG-sFGFR3_Del4-LK1-LK2 treatment as seen in Figure 8A. Following sacrifice at day 22, growth was evaluated as measurements of body weight, body length, and tail length. At the dose of 0.3 mg/kg, FLAG-sFGFR3_Del4-LK1-LK2 treatment had a positive effect on overall skeletal growth of transgenic *Fgfr3*^{*ach*/+} mice and their WT littermates (Figure 8B).

Potential side effects were evaluated in liver, lung, heart, spleen and kidneys at the time of sacrifice. None of the 102 animals that received chronic subcutaneous injections of FLAG-sFGFR3_Del4-LK1-LK2 or vehicle presented macroscopic abnormalities. In all groups, organ weight changes correlated with changes in total body weight (Table 2), suggesting no direct effect of FLAG-sFGFR3_Del4-LK1-LK2 treatment on organ growth. Blood counts were normal for all animals in this study (Table 3).

**Table 1: Evaluation of the PK/PD parameters of FLAG-sFGFR3_Del4-LK1-LK2. Comparison with the a full-length FLAG-sFGFR3 protein.**

| | Distribution half-life (αt_{1/2}) | Elimination half-life (βt_{1/2}) | Ka |
|---|---|---|---|
| FLAG-sFGFR3 | 0.3h | 4.5h | 3,05 |
| FLAG-sFGFR3_Del4-LK1-LK2 | 0.4h | 3.7h | 2,59 |

**Table 2: Coefficient correlation (r) between organ and body weight in the different treatment groups (FLAG-sFGFR3_Del4-KL1-KL2 (FP) or vehicle). Pearson or Spearman tests were used for statistical analysis of organ/body weights correlations in each treatment group. No statistical difference was found (Fisher r-to-z transformation) (n = 19-38).**

| **Correlation coefficient** | **Treatment** | **pancreas** | **spleen** | **kidney L** | **kidney R** | **liver** | **heart** | **lung** |
|---|---|---|---|---|---|---|---|---|
| **WT** | Vehicle | 0,5612 | 0,8016 | 0,7636 | 0,7651 | 0,821 | 0,7771 | 0,7442 |
| | 0.3 mg/kg FP | 0.749 | 0,6826 | 0,7411 | 0,749 | 0,9136 | 0,747 | 0,7168 |
| **Fgfr3^{ach/+}** | Vehicle | 0,8103 | 0,7109 | 0,7641 | 0,7467 | 0,7715 | 0,6757 | 0,6174 |
| | 0.3 mg/kg FP | 0,7001 I | 0,9038 | 0,824 | 0,8569 | 0,9276 | 0,8179 | 0,8351 |
| **Fisher r to z transformation** | | | | | | | | |
| **WT** | control vs treated | -1,11 | 0,892 | 0,172 | 0,184 | -1,28 | 0,188 | 0,195 |
| **Fgfr3^{ach/+}** | control vs treated | 0,644 | -1,493 | -0,404 | -0,782 | -1,526 | -0,814 | -1,19 |
| **p value** | | | | | | | | |
| **WT** | control vs treated | 0,132 | 0,186 | 0,431 | 0,427 | 0,197 | 0,425 | 0,422 |
| **Fgfr3^{ach/+}** | control vs treated | 0,259 | 0,067 | 0,343 | 0,217 | 0,063 | 0,207 | 0,115 |

**Table 3: Blood counts were not modified by sFGFR3 treatment. Plasma from the vehicle and 0.3 mg/kg FLAG-sFGFR3_Del4-LK1-LK2 (FP) groups were analyzed at time of sacrifice (n = 19-38). Data are means ± SD. Statistical comparisons with vehicle-treated WT mice were performed using a one way ANOVA.**

| | | WBCs (K/µl) | Neutrophils (%) | Lymphocytes (%) | Monocytes (%) | Eosinophils (%) | Basophils (%) | |
|---|---|---|---|---|---|---|---|---|
| **WT** | Vehicle | 1,82 ± 0,59 | 0,17 ± 0,17 | 1,49 ± 0,41 | 0,08 ± 0,04 | 0,05 ± 0,06 | 0,02 ± 0,03 | |
| | 0.3 mg/kg FP | 2,7 ± 1,09 | 0,25 ± 0,31 | 2,2 ± 0,75 | 0,17 ± 0,09 | 0,06 ± 0,07 | 0,02 ± 0,03 | |
| **Fgfr3^{ach/+}** | Vehicle | 1,79 ± 0,9 | 0,2 ± 0,19 | 1,41 ± 0,64 | 0,1 ± 0,1 | 0,05 ± 0,05 | 0,02 ± 0,02 | |
| | 0.3 mg/kg FP | 2,36 ± 0,81 | 0,18 ± 0,12 | 1,97 ± 0,65 | 0,14 ± 0,07 | 0,05 ± 0,05 | 0,02 ± 0,02 | |
| | | | | | | | | |

| | | RBCs (K/µl) | Hemoglobin (g/dl) | Hematocrit (%) | MCV | MCH | MCHC | Platelets (K/µl) |
|---|---|---|---|---|---|---|---|---|
| **WT** | Vehicle | 3,25 ± 0,39 | 4,72 ± 0,57 | 17,59 ± 2,23 | 54,17 ± 1,76 | 14,53 ± 0,31 | 26,87 ± 0,98 | 307,56 ± 84,71 |
| | 0.3 mg/kg FP | 3,49 ± 0,82 | 5,12 ± 1,29 | 19,31 ± 4,82 | 55,26 ± 1,27 | 14,65 ± 0,54 | 26,51 ± 0,69 | 312,11 ± 80,99 |
| **Fgfr3^{ach/+}** | Vehicle | 3,13 ± 0,32 | 4,54 ± 0,42 | 17,19 ± 2,12 | 54,83 ± 1,65 | 14,55 ± 0,74 | 26,57 ± 1,88 | 466,17 ± 45,69 |
| | 0.3 mg/kg FP | 3,43 ± 0,45 | 5 ± 2,54 | 18,68 ± 2,54 | 54,51 ± 1,93 | 14,6 ± 0,68 | 26,78 ± 0,88 | 456,9 ± 117,23 |

### Conclusion.

This study shows that a fusion protein, containing portions of sFGFR3 and of HPLN1, can be used to restore endochondral bone growth in a murine model of achondroplasia.

We first validated that a decoy variant containing the extracellular portion and a very short portion of the intracellular domain of sFGFR3 was sufficient to cause effective FGF binding and engender *in vivo* efficacy restoring bone growth in mice carrying the G380R mutation.

The fusion protein has been designed to specifically target cartilage, thus increasing exposure of the target tissue, allowing decreasing a potential effective dose. Biodistribution studies suggest cartilage trapping with similar diffusion through body (PK/PD analysis). FLAG-sFGFR3_Del4-LK1-LK2 treatment is effective at restoring bone growth in the transgenic murine model. Similar to FLAG-sFGFR3, no apparent toxicity was associated with FLAG-sFGFR3_Del4-LK1-LK2 treatment, suggesting that this protein can be used as a therapeutic for achondroplasia and related disorders.

We have also validated the use of body weight and skull length and/or width monitoring to evaluate velocity of growth. While both approaches give similar results, it is technically easier to use body weight as an index of velocity as skull measurement on newborn animals may created unwanted side effects if not done properly.

In conclusion, it has been demonstrated that FLAG-sFGFR3_Del4-LK1-LK2 is a viable treatment option for achondroplasia and related disorders.

### Example 6: Decoy design and purification of sFGFR3 polypeptides and fusion polypeptides

Further experiments were performed to generate sFGFR3 polypeptides (e.g., sFGFR3_Del4) and a sFGFR3 fusion polypeptide (e.g., sFGFR3_Del4-LK1-LK2) with a FLAG-tag in the C-terminal position. Recombinant proteins were produced by transient transfection using CaCl₂ transfection reagent in Freestyle™ 293FT cells (TheroFisher Scientific). The plasmid used was pBApo-EF1α-sFGFR3-FLAG plasmid for all variants (sFGFR3_Del1, sFGFR3_Del4, and sFGFR3_Del4-LK1-LK2). An Erlenmeyer flask (2000ml) was seeded with 293FT cells at a concentration of 1.8 X 10⁶ cells/ml. Cells were centrifuged for 5 minutes at room temperature prior to transfection, and then resuspended in 300 ml final volume of medium (Freestyle™ 293 Expression Medium Life technologies). A solution including sFGFR3-FLAG (600 µg plasmid DNA) and 1.73 m of 2M CaCl₂ was resuspended in 18 ml of 2 X Hepes Buffered Saline (HBS) and then incubated for 30 min at room temperature. The mixture was then added to an erlenmeyer flask and 600 ml of fresh media was added after 4 hours. After 72 hours, the supernatant was purified using the Akta™ Flux 6 cross-flow filtration system with a combination of two different capsules: 1) the ULTA Prime GF capsule featuring a pleated glass microfibre depth filter with a 5 micron pore size rating and a filter surface area of 0.095 m², and 2) the ULTA Pure HC capsule featuring a pleated polyethersulfone sterilizing grade membrane layer with a polyethersulfone prefilter with 0.6/0.2 micron pore size rating and a filter surface area of 0.1 m² (GE Healthercare Life Sciences).

Stable cells lines were then generated by stable transfection of the GP2-293 packaging cell line using CaCl₂ transfection (Clontech). The Del vectors were designed based on the transient transfection results obtained in 293FT cells to create stable cell lines that produce sFGFR3_Del1, sFGFR3_Del4, and sFGFR3_Del4-LK1-LK2. In particular, the plasmids included the signal peptide (MGAPACALALCVAVAIVAGASS) in the same as the previously designed N-terminal FLAG-sFGFR3 variants, the FLAG tag in the C terminal position, an EF1α promoter instead of a CMV promoter, and a pBAbe backbone. Co-transfection of pVSVg and pBABE-EF1α Puro sFGFR3_Del1 Cter FLAG, pBABE-EF1α Puro sFGFR3_Del4-LK1-LK2 Cter FLAG, or pBABE-EF1α Puro sFGFR3_Del4 Cter FLAG were performed according to the manufacturer's instructions.

The recombinant sFGFR3 polypeptides and fusion polypeptides were then purified using cross flow filtration system. The advantages of this technique are the elimination of contaminants without aggregation and the purified recombinant sFGFR3 polypeptide are produced under optimized conditions for IEX chromatography. The cross-flow filtration was performed using an Ultrafiltration Hollow Fiber Cartridge (Fiber: UFP-750C from GE Healthcare) at 10X concentration, followed by 3 times volume exchange in Tris 20mM pH 7 for sFGFR3_Del1 and Tris 20mM pH 8.5 for sFGFR3_Del4 and sFGFR3_Del4-LK1-LK2. Ion Exchange Chromatography (IEX) was then performed at pH 7 for sFGFR3_Del1 and at pH 8.5 for sFGFR3_Del4 and sFGFR3_Del4-LK1-LK2 using a Hi Prep Q FF 20 ml column (GE Healthcare Life Sciences). Aspiration and elution was performed at a rate of 5 ml/min. A volume fraction of 8 ml was added to the column, then the unbound sFGFR3 polypeptide was washed with a buffer including Tris 20 mM followed by a wash with Tris 20 mM, 1 M NaCl. Finally, size exclusion chromatography was performed using a HiLoad Superdex 200 prep grad (GE Healthcare), with 13 ml of the supernatant at a flow rate of 1 ml/min and an elution buffer of 20 mM Tris, 150 mM NaCl pH 7.4 for all recombinant sFGFR3 polypeptides and fusion polypeptides.

### Example 7: Role of signal sequence in sFGFR3 polypeptides and fusion polypeptides

Western blots of the sFGFR3_Del1, sFGFR3_Del4, and sFGFR3_Del4-LK1-LK2 were performed using TBOLT™ 4-12% Bis-Tris Plus Gels (Life technologies) and an IBLOT® 2 Gel Transfer Device (Life Technologies) with a PVDF membrane stack. Sample preparation and migration were performed using the manufacturer's instructions. The transfer procedure was the P0 program of the iBlot® 2 Gel Transfer Device (i.e., 20V for 1min, 23V for 4min, and 25V for 2min). After 30 minutes of blocking, incubation with anti-FLAG monoclonal M2 antibody (Sigma Aldrich) was performed for 1 hour. A C-DiGit Chemiluminescence Western Blot Scanner was then used for analysis with a standard ECL substrate (Licor).

When the FLAG tag of sFGFR3_Del1, sFGFR3_Del4, and sFGFR3_Del4-LK1-LK2 is in the N-terminal position followed by the signal peptide sequence, the sFGFR3 polypeptides and fusion polypeptide were not detectable via Western blot using the anti-FLAG monoclonal M2 antibody (Figure 9; lanes B1, B14, and B27). In contrast, sFGFR3_Del1, sFGFR3_Del4, and sFGFR3_Del4-LK1-LK2 with the FLAG tag in the C-terminal position were detectable via Western blot using the anti-FLAG monoclonal M2 antibody (see Figure 9; lanes B8, B21, and B34). These results demonstrate that the signal sequence (MGAPACALALCVAVAIVAGASS) is cleaved from sFGFR3_Del1, sFGFR3_Del4, and sFGFR3_Del4-LK1-LK2 when the FLAG tag is present at the C-terminal position, but not at the N-terminal position, which appears to disrupt secretion.

### Example 8: Biodistribution

Treatment with sFGFR3_Del1 or sFGFR3_Del4-LK1-LK2 was performed in *Fgfr3*^{*ach*/+} mice as described in Garcia et al. (*supra*), which is incorporated herein by reference in its entirety. Mice were sacrificed at day 5 and day at day 22 after birth. The biobiodistribution of sFGFR3_Del1 and sFGFR3_Del4-LK1-LK2 in the growth plate of the mice and the effect of sFGFR3_Del1 and sFGFR3_Del4-LK1-LK2 on all bones, growth plate thickness, and the hypertrophic chondrocyte zone were determined over a three week period. The presence of the FLAG tagged polypeptides was determined in the growth plate. These results demonstrate that sFGFR3_Del4-LK1-LK2 is trapped faster within the growth plate (e.g., at day 5), which may be due to an aggrecan defect in *Fgfr3*^{*ach*/+} mice (Figure 10A). Additionally, there was a greater accumulation of sFGFR3_Del1 at day 22 (Figure 10B). There was also a greater accumulation of sFGFR3_Del1 at day 22 in *Fgfr3*^{*ach*/+} mice (Figure 10B). Results further indicated that sFGFR3_Del4-LK1-LK2 is trapped faster within the growth plate in wild-type mice at day 5 relative to sFGFR3_Del1 and also that there is greater accumulation of sFGFR3_Del4-LK1-LK2 at day 22 in wild-type mice relative to sFGFR3_Del1.

**Table 4: Measurement of body lengthy, tail lenght, skull height, and skull length of wild-type mice and Fgfr3^{ach/+} mice after 5 days of treatment with sFGFR3_Del1 and sFGFR3_Del4-LK1-LK2.**

| Time | Polypeptide | GENOTYPE | Body length | Tail Length | Skull height 1 | Skull height 2 | Skull length |
|---|---|---|---|---|---|---|---|
| D3+2 | sFGFR3_Del1 | wt | 57.42 | 27.42 | 4.42 | 2.99 | 13.02 |
| | | ach | 53.45 | 25.66 | 4.61 | 3.01 | 12.53 |
| | sFGFR3_Del4-LK1-LK2 | wt | 62.77 | 30.71 | 4.89 | 2.96 | 14.10 |
| | | ach | 60.06 | 29.07 | 4.22 | 2.51 | 13.28 |

| | | | | | | | |
|---|---|---|---|---|---|---|---|
| Skull measurements and x-ray radiography were also performed on wild-type mice and *Fgfr3*^{*ach*/*+*} mice after 5 days of treatment with sFGFR3_Del1 and sFGFR3_Del4-LK1-LK2 (Table 4; Figures 11A and B). Skull measurements and x-ray radiography was also performed on wild-type mice and *Fgfr3*^{*ach*/*+*} mice without treatment (Tables 5 and 6; Figures 11C-11H). | | | | | | | |

**Table 5: Measurements of skull parameters in wild-type mice and Fgfr3^{ach/+} mice (n = 12). Dorsal measurements include skull length, skull width, palatine length, foramen magnum height, and foramen magnum weidth. Agostino and Pearson omnibus normality test (a = 0.05) and Brown-Forsythe test (P < 0.05) followed by two-tailed Student's t test were performed to determine statistical significance.**

| | wt | ach | p value |
|---|---|---|---|
| Skull length | 20.75 ± 0.19 | 18.61 ± 0.40 | <0.0001 *** |
| Skull width | 10.55 ± 0.06 | 10.61 ± 0.17 | 0.7568 ns |
| Skull L/W | 1.96 ± 0.02 | 1.75 ± 0.03 | <0.0001 *** |
| palatine | 3.163 ± 0.09 | 2.722 ± 0.11 | 0.0097 ** |
| foramen height | 3.499 ± 0.08 | 3.396 ± 0.07 | 0.3871 ns |
| foramen width | 4.762 ± 0.02 | 4.599 ± 0.05 | 0.0122 * |

**Table 6: Measurements of axial/appendicular and lateral skeletal parameters in wild-type mice and Fgfr3^{ach/+} mice (n = 12). The axial/appendicular measurements include CTL (cervico-thoraco-lumbar length) and femur length (lateral). The lateral measurements include skull length and height. Agostino and Pearson omnibus normality test (a = 0.05) and Brown-Forsythe test (P < 0.05) followed by two-tailed Student's t test were performed to determine statistical significance.**

| | wt | ach | p value |
|---|---|---|---|
| CTL | 35.11 ± 0.58 | 31.17 ± 1.05 | 0.0030 ** |
| Femur length | 10.23 ± 0.30 | 8.83 ± 0.32 | 0.0053 ** |
| Skull length | 20.75 ± 0.19 | 18.61 ± 0.40 | <0.0001 *** |
| Skull height | 4.76 ± 0.17 | 5.107 ± 0.28 | 0.3076 ns |

### Example 9: Potency Assays

Potency assays were performed to determine the effect of sFGFR3_Del1 (SEQ ID NO: 1) on Erk/P-Erk Intracellular signaling in ATDC5 chondrocyte cells. ATDC5 cells were plated at a density of 7.5 × 10³ in 96-well plates and cultured for 24 hours in DMEM-F12/0.5% BSA (Life Technologies). Cells were then challenged for 24 hours with hFGF2 (100 pg/ml) in the presence of sFGFR3_Del1 (0 or 20 ng/ml). Intracellular signaling was evaluated with the ICW kit PhosphoPlus® p44/42 MAPK (Erk1/2)(Thr202/Tyr204) In-Cell Duet (ICW Compatible)-(cell signaling). After a 24 hour incubation, there was a decrease of Erk phosphorylation in ATDC5 cells incubated with sFGFR3_Del1 (SEQ ID NO: 1) and FGF decreased relative to ATDC5 cells incubated with sFGFR3_Del1 (SEQ ID NO: 1) or FGF alone (Figure 12). These results demonstrate that sFGFR3_Del1 (SEQ ID NO: 1) is a functional decoy for FGF. Thus, other sFGFR3 polypeptide variants, in particular, those including a deletion of amino acids 311 to 422 of SEQ ID NO: 6, such as sFGFR3_Del2, sFGFR3_Del3, or sFGFR3_Del4, would be expected to exhibit similar properties as a functional decoy for FGF.

### Example 10: Cellular proliferation

The effect of different fractions of purified sFGFR3 polypeptide or sFGFR3 fusion polypeptide on cellular proliferation was determined in ATDC5 chondrocyte cells. Size-exclusion chromatography followed by western blot analysis was performed as described above to purify and identify fractions of sFGFR3_Del1 (Figures 13A-13C) and sFGFR3_Det4-LK1-LK2 (Figures 14A-14C). ATDC5 cells were plated at a density of 5 × 10³ in 96-well plates and cultured for 48 hours in DMEM-F12/0.5% BSA (Life Technologies). Cells were then challenged for 72 hours with FGF2 (100 pg/ml) in the presence of fractions of either sFGFR3_Del1 or sFGFR3_Del4-LK1-LK2 (0 or 20 ng/ml). Proliferation was evaluated with the Cy quant proliferation assay (Life technologies) to measure fluorescence. These results demonstrate that different fractions of sFGFR3_Del1 (fractions 2 to 5 and 6; Figure 12C) and sFGFR3_Del4-LK1-LK2 (fractions 9-12) significantly increased cellular proliferation in ATDC5 chondrocyte cells.

### Example 11: Behavioral studies

Behavioral studies were performed to characterize the long-term effects of sFGFR3_Del1 and sFGFR3_Del4-LK1-LK2 in the C57BL6/J mouse. sFGFR3_Del1 and sFGFR3_Del4-LK1-LK2 were each administered subcutaneously twice per week during the first 8 postnatal weeks, starting from postnatal day 3. There was three different groups (16 male mice per group) treated with sFGFR3_Del1, sFGFR3_Del4-LK1-LK2, or a vehicle at 2.5mg/kg. Behavioral evaluation was conducted between postnatal weeks 10 and 14. The effects of sFGFR3_Del1 and sFGFR3_Del4-LK1-LK2 on behavior, sensory capacities, motor capacities, psychological characteristics, and cognitive function were investigated.

These studies showed no indications of long term toxicity of treatment with sFGFR3_Del1 and sFGFR3_Del4-LK1-LK2. For the Irwin test, activity meter test, elevated plus-maze test, and forced swimming test, no changes in any group were observed. For odor discrimination, I observed a minor decrease with sFGFR3_Del4-LK1-LK2. Administration of sFGFR3_Del4-LK1-LK2 also appeared to result in a minor decrease of motor coordination during the accelerating rotarod test and a minor improvement of motor coordination and spatial memory during the Morris water maze test, which were not statistically different.

### Example 12: FGFR3_Del1 and sFGFR3_Del4-LK1-LK2 do not cross the blood brain barrier

Pharmacokinetic studies were performed to determine the uptake of FGFR3_Del1 and sFGFR3_Del4-LK1-LK2 across the blood brain barrier in C57BL/6 mice. After intravenous (i.v.) bolus injection, brain tissue uptake of FGFR3_Del1 and sFGFR3_Del4-LK1-LK2 was measured at 4 time points (1 hour (h), 3 hours (h), 6 hours (h), and 24 hours (h)). FGFR3_Del1 and sFGFR3_Del4-LK1-LK2 were injected as radiolabeled tracer (¹²⁵I- FGFR3_Del1 or ¹²⁵I- sFGFR3_Del4-LK1-LK2) with 2.5 mg/kg unlabeled FGFR3_Del1 and sFGFR3_Del4-LK1-LK2. The injected dose of ¹²⁵I- FGFR3_Del1 or ¹²⁵I- sFGFR3_Del4-LK1-LK2 was about 10 µCi per animal, which corresponds to less than 0.1 mg/kg. After euthanizing the mice at 1h, 3h, 6h or 24h, the concentration of ¹²⁵I- FGFR3_Del1 or ¹²⁵I-sFGFR3_Del4-LK1-LK2 in organs and plasma was measured by liquid scintillation counting.

¹²⁵I- FGFR3_Del1 or ¹²⁵I- sFGFR3_Del4-LK1-LK2 concentrations were corrected for metabolism in plasma and in brain samples by measuring the fraction of trichloroacetic acid (TCA) precipitable material (e.g., intact tracer). The validity of the TCA correction was also confirmed by injecting samples on a size exclusion fast protein liquid chromatography (FPLC) column. The organ concentration of ¹²⁵I- FGFR3_Del1 or ¹²⁵I- sFGFR3_Del4-LK1-LK2 was corrected for intravascular content (V₀ by injecting radiolabeled albumin (³H-RSA) shortly before sacrificing the animal. The apparent organ volume of distribution of RSA represents V₀. The dose of albumin was negligible (on the order of 1% of the physiological concentration). For all organs other than brain, the concentrations were calculated by subtracting the vascular content and taking into account the TCA precipitable fraction in plasma. However, no correction was made for the uptake of degraded material into these organs other than the brain because no TCA precipitation was performed.

The brain concentrations were calculated by the following formula: C_{brain(Corr.)} = [V_{d}(FGFR3_Del1) - V₀] X Cₚₗₐₛₘₐ (terminal), in which V_{d}(FGFR3_Del1) is the volume of distribution of FGFR3_Del1 in brain (calculated as C_{brain} / Cₚₗₐₛₘₐ), V₀ is the volume of albumin distributed in the brain, and Cₚₗₐₛₘₐ₍ₜₑᵣₘᵢₙₐₗ₎ is the plasma concentration of FGFR3_Del1 at the terminal sampling time. Calculations for sFGFR3_Del4-LK1-LK2 were performed identically, and all concentrations were expressed as the percent of injected dose per gram or ml (%ID/g or %ID/mL), respectively, and the dose of the i.v. bolus equals 100%. If desired, these values may be converted to [mg/g] or [mg/mL] by multiplication with the injected dose: (body weight in g /1000 g) x 2.5 mg. All body weights were in the range of 25g-30g.

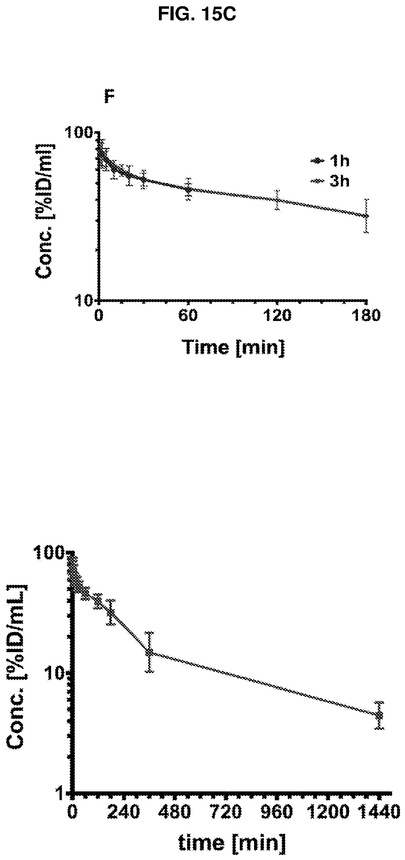

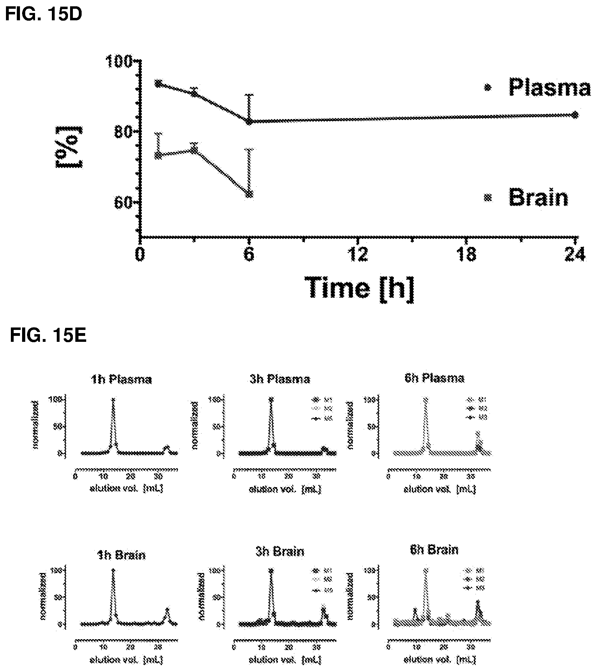

There was no detectable brain uptake of ¹²⁵I- FGFR3_Del1, as indicated by corrected brain concentrations (after correction for vascular content and degradation (TCA precipitability)), at any of the measured time points (1 h, 3 h, 6 h, or 24 h) (Figure 15A). Additionally, V_{d} of RSA (=V0) and V_{d} of ¹²⁵I- FGFR3_Del1 were not significantly different at any of the measured time points (1 h, 3 h, 6 h, or 24 h), as determined by a paired t-test (Figure 15B). Plasma concentration for¹²⁵I- FGFR3_Del1 were determined in order to compare the stability of ¹²⁵I- FGFR3_Del1 in plasma and brain tissue (Figure 15C). The TCA precipitable fraction of ¹²⁵I- FGFR3_Del1 was lower in brain tissue than plasma at all measured time points (concentrations in 24h brain samples were undetectable) (Figure 15D), which may indicate some uptake of low molecular weight degradation products. The FPLC elution profiles were consistent with TCA data (Figure 15E).

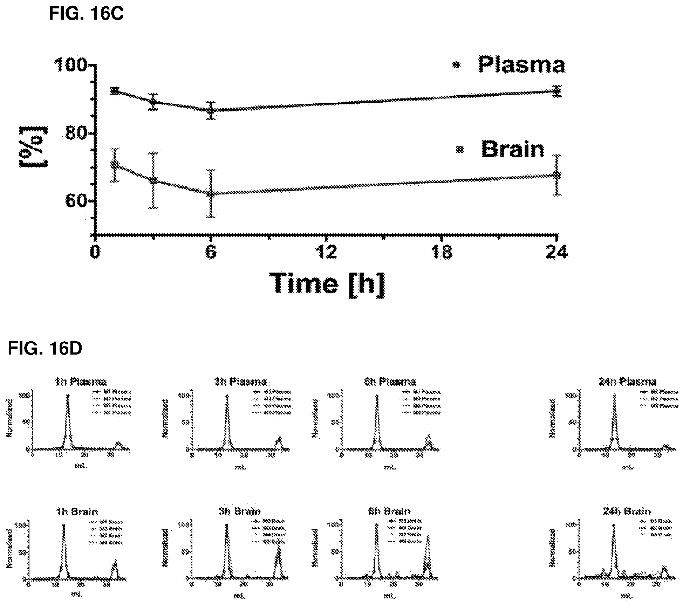

Similar results were obtained for brain tissue uptake of ¹²⁵I- sFGFR3_Del4-LK1-LK2. There was no detectable brain uptake of ¹²⁵I- sFGFR3_Del4-LK1-LK2, as indicated by corrected brain concentrations (after correction for vascular content and degradation (TCA precipitability)), at any of the measured time points (1 h, 3 h, 6 h, or 24 h) (Figure 16A). Additionally, V_{d} of RSA (=V0) and V_{d} of ¹²⁵I- sFGFR3_Del4-LK1-LK2 were not significantly different at any of the measured time points (1 h, 3 h, 6 h, or 24 h), as determined by a paired t-test (Figure 16B). The TCA precipitable fraction of ¹²⁵I-sFGFR3_Del4-LK1-LK2 was lower in brain than plasma at all measured time points (Figure 16C), which may indicate some uptake of low molecular weight degradation products. The FPLC elution profiles were consistent with TCA data (Figure 16D). Comparatively, plasma concentrations for ¹²⁵I-FGFR3_Del1 and ¹²⁵I- sFGFR3_Del4-LK1-LK2 were similar at all measured time points (Figure 16E).

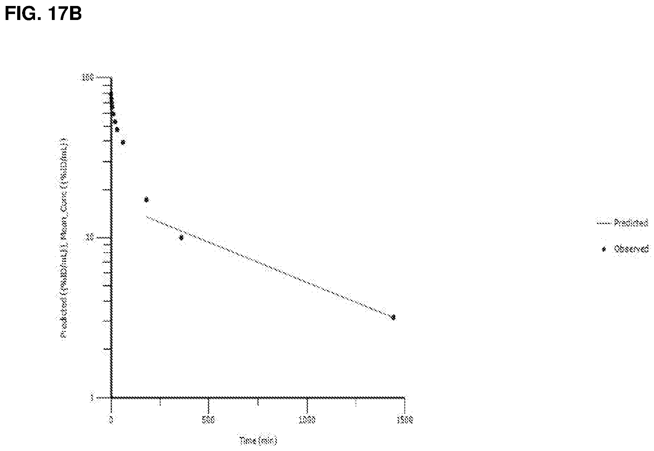

In a non-compartmental analysis (NCA) of ¹²⁵I- FGFR3_Del1 and ¹²⁵I- sFGFR3_Del4-LK1-LK2 plasma concentrations with Phoenix WinNonlin, the area under the curve (AUC) of ¹²⁵I- sFGFR3_Del4-LK1-LK2 was lower than the AUC of ¹²⁵I- FGFR3_Del1 by a factor of 0.71 (Figure 17A and 17B). Thus, the clearance of ¹²⁵I- sFGFR3_Del4-LK1-LK2 was 1.4-fold higher than the clearance of ¹²⁵I-FGFR3_Del1. The estimated terminal half lives were similar (i.e., 600 min for ¹²⁵I- sFGFR3_Del4-LK1-LK2 and 566 min for ¹²⁵I- FGFR3_Del1). Initial concentrations (C0) were almost identical for ¹²⁵I-FGFR3_Del1 or ¹²⁵I- sFGFR3_Det4-LK1-LK2. The volumes of distribution (Vz or Vss) of ¹²⁵I-sFGFR3_Del4-LK1-LK2 are higher than Vz or Vss for ¹²⁵I- FGFR3_Del1, consistent with the higher organ uptake of ¹²⁵I- sFGFR3_Del4-LK1-LK2 in kidney and liver seen in the early phase (e.g., the distribution phase).

In conclusion, there is no measurable uptake of either FGFR3_Del1 or sFGFR3_Del4-LK1-LK2 into brain tissue of mice at any of the time points analyzed in this study, at a dose of 2.5 mg/kg injected as an intravenous bolus. These results are based on the assumption that the ¹²⁵I-FGFR3_Del1 and ¹²⁵I-sFGFR3_Del4-LK1-LK2 (tracer-labeled proteins) behave similarly to unlabeled protein in brain tissue and plasma.

The invention also pertains to the following:
1. A soluble Fibroblast Growth Factor Receptor 3 (sFGFR3) polypeptide comprising an amino acid sequence:
   (i) comprising at least amino acid residues 1 to 310 or 1 to 323 of the amino acid sequence of SEQ ID NO: 1, but excluding at least amino acid residues 655 to 694 of the amino acid sequence of SEQ ID NO: 1, or a variant of said amino acid sequence, wherein said variant comprises an amino acid sequence comprising at least 80% sequence identity to at least amino acids 1 to 310 or 1 to 323 of SEQ ID NO: 1, wherein the sFGFR3 polypeptide has a length of at least 311 amino acids; or
   (ii) comprising at least amino acid residues 1 to 357 of the amino acid sequence of SEQ ID NO: 2, but excluding at least amino acid residues 703 to 741 of the amino acid sequence of SEQ ID NO: 2, or a variant of said amino acid sequence, wherein said variant comprises an amino acid sequence comprising at least 80% sequence identity to at least amino acids 1 to 357 of SEQ ID NO: 2; wherein said variant of (i) or (ii) specifically binds to an FGF.
2. The polypeptide of Item 1, wherein said polypeptide comprises at least amino acid residues 1 to 310 of the amino acid sequence of SEQ ID NO: 1 and further comprises a heterologous polypeptide.
3. The polypeptide of Item 2, wherein the polypeptide is a sFGFR3 fusion polypeptide comprising an aggrecan-binding protein or fragment thereof as the heterologous polypeptide.
4. The polypeptide of any one of Items 1 to 3, wherein the polypeptide comprises an amino acid sequence:
   (i) comprising at least amino acid residues 1 to 310 of the amino acid sequence of SEQ ID NO: 1, but excluding at least amino acid residues 655 to 694 of the amino acid sequence of SEQ ID NO: 1, or a variant of said amino acid sequence, wherein said variant comprises an amino acid sequence comprising at least 80% sequence identity to at least amino acids 1 to 310 of SEQ ID NO: 1; or
   (ii) comprising at least amino acid residues 1 to 357 of the amino acid sequence of SEQ ID NO: 2, but excluding at least amino acid residues 703 to 741 of the amino acid sequence of SEQ ID NO: 2, or a variant of said amino acid sequence, wherein said variant comprises an amino acid sequence comprising at least 80% sequence identity to at least amino acids 1 to 357 of SEQ ID NO: 2;
   wherein said variant of (i) or (ii) specifically binds to an FGF.
5. The polypeptide of Item 3, wherein the aggrecan-binding protein or fragment thereof is selected from the group consisting of:
   (a) a HPLN1 fragment comprising an amino acid sequence comprising at least amino acid residues 158 to 252 of the amino acid sequence of SEQ ID NO: 3, amino acid residues 259 to 349 of the amino acid sequence of SEQ ID NO: 3, or amino acid residues 158 to 349 of the amino acid sequence of SEQ ID NO: 3, or a variant of said amino acid sequence, wherein said variant comprises a sequence identity comprising at least 80% sequence identity to at least amino acid residues 158 to 252 or 259 to 249 of SEQ ID NO: 3, and wherein said variant specifically binds to aggrecan;
   (b) HPLN1 or a variant thereof, wherein said variant comprises an amino acid sequence comprising at least 80% sequence identity to the amino acid sequence of SEQ ID NO: 3, wherein said variant specifically binds to aggrecan;
   (c) an antibody, an antibody derivative, or an antibody mimetic specific for aggrecan; or
   (d) fibulin-1, borrelial aggrecan-binding protein, Cartilage oligomeric matrix protein/thrombospondin 5 (COMP/TSP5), or aggrecan-binding fragments thereof.
6. The polypeptide of any one of items 1 to 5, wherein the polypeptide comprises:
   (a) an amino acid sequence comprising amino acid residues 1 to 310 of the amino acid sequence of SEQ ID NO: 1, but excluding amino acid residues 324 to 694 of the amino acid sequence of SEQ ID NO: 1, or a variant of said amino acid sequence, wherein said variant comprises an amino acid sequence comprising at least 80% sequence identity to at least amino acids 1 to 310 of SEQ ID NO: 1;
   (b) an amino acid sequence comprising amino acid residues 1 to 357 of the amino acid sequence of SEQ ID NO: 2, but excluding amino acid residues 371 to 741 of the amino acid sequence of SEQ ID NO:2, or a variant of said amino acid sequence, wherein said variant comprises an amino acid sequence comprising at least 80% sequence identity to at least amino acids 1 to 357 of SEQ ID NO: 2;
   (c) an amino acid sequence comprising amino acid residues 1 to 310 of the amino acid sequence of SEQ ID NO: 1, but excluding amino acid residues 441 to 694 of the amino acid sequence of SEQ ID NO: 1, or a variant of said amino acid sequence, wherein said variant comprises an amino acid sequence comprising at least 80% sequence identity to at least amino acids 1 to 310 of SEQ ID NO: 1;
   (d) an amino acid sequence comprising amino acid residues 1 to 357 of the amino acid sequence of SEQ ID NO: 2, but excluding amino acid residues 488 to 741 of the amino acid sequence of SEQ ID NO:2, or a variant of said amino acid sequence, wherein said variant comprises an amino acid sequence comprising at least 80% sequence identity to at least amino acids 1 to 357 of SEQ ID NO: 2;
   (e) an amino acid sequence comprising amino acid residues 1 to 310 of the amino acid sequence of SEQ ID NO: 1, but excluding amino acid residues 549 to 694 of the amino acid sequence of SEQ ID NO: 1, or a variant of said amino acid sequence, wherein said variant comprises an amino acid sequence comprising at least 80% sequence identity to at least amino acids 1 to 310 of SEQ ID NO: 1; or
   (f) an amino acid sequence comprising amino acid residues 1 to 357 of the amino acid sequence of SEQ ID NO: 2, but excluding amino acid residues 596 to 741 of the amino acid sequence of SEQ ID NO:2, or a variant of said amino acid sequence, wherein said variant comprises an amino acid sequence comprising at least 80% sequence identity to at least amino acids 1 to 357 of SEQ ID NO: 2;
   wherein said variant of (a)-(f) specifically binds to an FGF.
7. The polypeptide of any one of items 1 to 6, wherein the FGF is Fibroblast Growth Factor 1 (FGF1), Fibroblast Growth Factor 2 (FGF2), Fibroblast Growth Factor 9 (FGF9), or Fibroblast Growth Factor (FGF 18).
8. The polypeptide of item 3, wherein said sFGFR3 fusion protein comprises an amino acid sequence comprising at least 80% sequence identity to the amino acid sequence of SEQ ID NO: 33.
9. The polypeptide of item 8, wherein the sFGFR3 polypeptide comprises (i) an amino acid sequence comprising amino acid residues 1 to 325 of the amino acid sequence of SEQ ID NO: 33, or (ii) an amino acid sequence comprising amino acid residues 1 to 338 of the amino acid sequence of SEQ ID NO: 33, or a variant of said amino acid sequence, wherein said variant of (i) or (ii) comprises an amino acid sequence comprising at least 80% sequence identity to at least amino acids 1 to 325 of SEQ ID NO: 33.
10. A nucleic acid encoding the polypeptide of any one of items 1 to 9.
11. A vector comprising the nucleic acid of item 10.
12. A cell comprising the nucleic acid of item 10 or the vector of item 11.
13. A composition comprising the polypeptide of any one of items 1 to 9, the nucleic acid of item 10, the vector of item 11, or the cell of item 12.
14. The composition of item 13, wherein the composition is formulated to provide about 0.0002 mg/kg/day to about 20 mg/kg/day of said polypeptide to a subject in need thereof.
15. The composition of item 14, wherein the composition is formulated to provide about 0.001 mg/kg/day to about 7 mg/kg/day of said polypeptide to said subject in need thereof.
16. The composition of any one of items 13 to 15, further comprising a pharmaceutically acceptable carrier.
17. The composition of item 16, wherein the composition is formulated for subcutaneous, topical, oral, intranasal, intraocular, intravenous, or intramuscular administration.
18. The composition of item 17, wherein the composition is formulated for subcutaneous administration.
19. The composition of any one of items 13-18 for use as a medicament.
20. The composition of any one of items 13 to 18 for use in the prevention or treatment of a skeletal growth retardation disorder in a subject in need thereof.
21. The composition of item 20, wherein the subject in need thereof is a human.
22. The composition of item 20 or 21, wherein the skeletal growth retardation disorder is a FGFR3-related skeletal disease.
23. The composition of item 22, wherein the skeletal growth retardation disorder is selected from the group consisting of achondroplasia, thanatophoric dysplasia type I (TDI), thanatophoric dysplasia type II (TDII), severe achondroplasia with developmental delay and acanthosis nigricans (SADDAN), hypochondroplasia, and a craniosynostosis syndrome.
24. The composition of item 23, wherein the skeletal growth retardation disorder is achondroplasia.
25. The composition of item 23, wherein the craniosynostosis syndrome is selected from the group consisting of Muenke syndrome, Crouzon syndrome, Apert syndrome, Jackson-Weiss syndrome, Pfeiffer syndrome, and Crouzonodermoskeletal syndrome.
26. A method for monitoring the treatment of a skeletal growth retardation disorder comprising measuring body weight and/or skull size of a subject in need thereof, wherein body weight and/or skull size of said subject in need thereof are measured in response to administration of the composition of any one of items 13 to 18.
27. The method of item 26, wherein said subject in need thereof is a human.
28. The polypeptide of item 3, wherein the aggrecan-binding protein is a Borrelia aggrecan-binding protein.
29. The polypeptide of item 28, wherein the Borrelia aggrecan-binding protein is selected from the group consisting of Borrelia glycosaminoglycan-binding protein (Bgp) and Borrelia burgdorferi high temperature requirement A (BbHtrA).
30. The polypeptide of item 2, wherein the heterologous polypeptide is or comprises an Fc region.
31. The polypeptide of any one of items 1 to 9, wherein the variant has a sequence identity of at least 85%, 90%, 95%, 97%, or 99% to said amino acid sequence of SEQ ID NO: 1 or SEQ ID NO: 2.
32. The polypeptide of item 5, wherein the variant has a sequence identity of at least 85%, 90%, 95%, 97%, or 99% to said amino acid sequence of SEQ ID NO: 3.
33. The polypeptide of item 8 or 9, wherein the variant has a sequence identity of at least 85%, 90%, 95%, 97%, or 99% to said amino acid sequence of SEQ ID NO: 33.
34. The polypeptide of any one of items 1 to 9, wherein the polypeptide is biologically active to improve bone growth.

## Claims

1. A secreted form of a soluble fibroblast growth factor receptor 3 (sFGFR3) polypeptide, wherein the amino acid sequence of the sFGFR3 polypeptide comprises amino acid residues 1-370 of SEQ ID NO: 2, or a variant with at least 99% sequence identity thereto.

2. The secreted form of the sFGFR3 polypeptide of claim 1, wherein the secreted form of the sFGFR3 polypeptide lacks the signal peptide, optionally wherein the amino acid sequence of the signal peptide consists of amino acid residues 1-22 of SEQ ID NO: 2.

3. The secreted form of the sFGFR3 polypeptide of claims 1 or 2, wherein the first amino acid of the secreted form of the sFGFR3 polypeptide corresponds to amino acid residue 23 of SEQ ID NO: 2 or the variant thereof.

4. The secreted form of the sFGFR3 polypeptide of any one of claims 1-3, wherein the amino acids of the secreted form of the sFGFR3 polypeptide corresponds to amino acid positions 23-370 of SEQ ID NO: 2 or the variant thereof.

5. The secreted form of the sFGFR3 polypeptide of any one of claim 1-4, wherein the sFGFR3 polypeptide does not comprise amino acid residues 371-741 of SEQ ID NO: 2.

6. The secreted form of the sFGFR3 polypeptide of any one of claims 1-5, wherein the secreted form of the sFGFR binds FGF, and wherein the FGF is FGF1, FGF2, FGF9, and/or FGF18.

7. A fusion protein comprising the secreted form of the sFGFR3 polypeptide of any one of claims 1-6 and an Fc region of an immunoglobulin, preferably wherein the Fc region is an immunoglobulin molecule selected from the group consisting of IgG-1, IgG-2, IgG-3, IgG-4, IgA-1, and IgA-2.

8. A composition comprising the secreted form of the sFGFR3 polypeptide of any one of claims 1-6, or the fusion protein of claim 7, preferably wherein the composition further comprises a pharmaceutically acceptable carrier.

9. The composition of claim 8, wherein the composition is formulated to provide 0.0002 mg/kg/day to 20 mg/kg/day of said polypeptide to a subject in need thereof.

10. The composition of claims 8 or 9, wherein the composition is formulated to provide 0.2 mg/kg/day to 3 mg/kg/day of said polypeptide to a subject in need thereof.

11. The composition of any one of claims 8-10, wherein the composition is formulated for subcutaneous, topical, oral, intranasal, intraocular, intravenous, or intramuscular administration.

12. The composition of claim 10, wherein the composition is formulated for subcutaneous administration or intravenous administration.

13. The composition of any one of claims 7-10 for use in treating a skeletal growth retardation disorder in a subject in need thereof.

14. The composition for use according to claim 13, wherein:
a) the subject is a human; and/or
b) the skeletal growth retardation disorder is a FGFR3-related skeletal disease selected from the group consisting of achondroplasia, thanatophoric dysplasia type I (TDI), thanatophoric dysplasia type II (TDII), severe achondroplasia with developmental delay and acanthosis nigricans (SADDAN), hypochondroplasia, and a craniosynostosis syndrome.

15. The composition for use according to claim 14, wherein the FGFR3-related skeletal disease is achondroplasia or hypochondroplasia.
